# EUROPÄISCHE PATENTSCHRIFT

(11) **EP 1 891 181 B1**
(45) Veröffentlichungstag und Bekanntmachung des Hinweises auf die Patenterteilung: **17.02.2010**
(21) Anmeldenummer: 06742977.9
(22) Anmeldetag: 18.05.2006
(51) Int. Cl.: C09K 19/04, C09K 19/16, C09K 19/30, C09K 19/34, C09K 19/20

(54) **FLÜSSIGKRISTALLINES MEDIUM UND FLÜSSIGKRISTALLANZEIGE MIT 1,2-DIFLUORETHENVERBINDUNGEN**
LIQUID-CRYSTALLINE MEDIUM AND LIQUID CRYSTAL DISPLAY WITH 1,2-DIFLUOROETHENE COMPOUNDS
MILIEU A CRISTAUX LIQUIDES ET AFFICHEUR A CRISTAUX LIQUIDES CONTENANT DES COMPOSES 1,2-DIFLUOROETHYLENE

(30) Priorität: 13.06.2005 DE 102005027171
(43) Veröffentlichungstag der Anmeldung: 27.02.2008
(73) Patentinhaber: Merck Patent GmbH, 64293 Darmstadt (DE)
(72) Erfinder: WITTEK, Michael, 64285 Darmstadt (DE); CZANTA, Markus, 64289 Darmstadt (DE); HIRSCHMANN, Harald, 64291 Darmstadt (DE); REIFFENRATH, Volker, 64380 Rossdorf (DE)
(86) Internationale Anmeldenummer: PCT/EP2006/004708
(87) Internationale Veröffentlichungsnummer: WO 2006/133783

(56) Entgegenhaltungen:
- EP-A- 0 560 382
- EP-A- 1 215 270
- EP-A- 1 416 030
- WO-A-2004/106460
- DE-A1- 10 124 480
- DE-A1- 10 151 491
- DE-A1- 10 155 073
- DE-A1-102004 008 638
- US-A- 5 380 461
- US-A1- 2001 004 108
- PATENT ABSTRACTS OF JAPAN Bd. 015, Nr. 177 (C-0829), 7. Mai 1991 (1991-05-07) -& JP 03 041037 A (SEIMI CHEM KK), 21. Februar 1991 (1991-02-21)
- PATENT ABSTRACTS OF JAPAN Bd. 016, Nr. 128 (C-0924), 2. April 1992 (1992-04-02) -& JP 03 294386 A (SEIMI CHEM KK), 25. Dezember 1991 (1991-12-25)
- PATENT ABSTRACTS OF JAPAN Bd. 1995, Nr. 02, 31. März 1995 (1995-03-31) -& JP 06 329566 A (ASAHI GLASS CO LTD; others: 01), 29. November 1994 (1994-11-29) in der Anmeldung erwähnt

## Beschreibung

Die vorliegende Erfindung betrifft ein flüssigkristallines Medium enthaltend 1,2-Difluorethenverbindungen, sowie dessen Verwendung für elektrooptische Zwecke und dieses Medium enthaltende Anzeigen. Neue erfindungsgemäße 1,2-Difluorethenverbindungen werden offenbart.

Flüssige Kristalle werden vor allem als Dielektrika in Anzeigevorrichtungen verwendet, da die optischen Eigenschaften solcher Substanzen durch eine angelegte Spannung beeinflusst werden können. Elektrooptische Vorrichtungen auf der Basis von Flüssigkristallen sind dem Fachmann bestens bekannt und können auf verschiedenen Effekten beruhen. Derartige Vorrichtungen sind beispielsweise Zellen mit dynamischer Streuung, DAP-Zellen (Deformation aufgerichteter Phasen), Gast/Wirt-Zellen, TN-Zellen mit verdrillt nematischer ("twisted nematic") Struktur, STN-Zellen ("super-twisted nematic"), SBE-Zellen ("superbirefringence effect") und OMI-Zellen ("optical mode interference"). Die gebräuchlichsten Anzeigevorrichtungen beruhen auf dem Schadt-Helfrich-Effekt und besitzen eine verdrillt nematische Struktur. Daneben gibt es auch Zellen, die mit einem elektrischen Feld parallel zur Substrat- und Flüssigkristallebene arbeiten, wie den IPS-Zellen ("in-plane switching"). Vor allem die TN-, STN- und IPS-Zellen sind derzeit kommerziell interessante Einsatzgebiete für die erfindungsgemäßen Medien.

Die Flüssigkristallmaterialien müssen eine gute chemische und thermische Stabilität und eine gute Stabilität gegenüber elektrischen Feldern und elektromagnetischer Strahlung besitzen. Ferner sollten die Flüssigkristallmaterialien eine niedrige Viskosität aufweisen und in den Zellen kurze Ansprechzeiten, tiefe Schwellenspannungen und einen hohen Kontrast ergeben.

Weiterhin sollten sie bei üblichen Betriebstemperaturen, d.h. in einem möglichst breiten Bereich unterhalb und oberhalb Raumtemperatur, eine geeignete Mesophase besitzen, beispielsweise für die oben genannten Zellen eine nematische oder cholesterische Mesophase. Da Flüssigkristalle in der Regel als Mischungen mehrerer Komponenten zur Anwendung gelangen, ist es wichtig, dass die Komponenten untereinander gut mischbar sind. Weitere Eigenschaften, wie die elektrische Leitfähigkeit, die dielektrische Anisotropie und die optische Anisotropie, müssen je nach Zellentyp und Anwendungsgebiet unterschiedlichen Anforderungen genügen. Beispielsweise sollten Materialien für Zellen mit verdrillt nematischer Struktur eine positive dielektrische Anisotropie und eine geringe elektrische Leitfähigkeit aufweisen.

Beispielsweise sind für Matrix-Flüssigkristallanzeigen mit integrierten nicht-linearen Elementen zur Schaltung einzelner Bildpunkte (MFK-Anzeigen) Medien mit großer positiver dielektrischer Anisotropie, breiten nematischen Phasen, relativ niedriger Doppelbrechung, sehr hohem spezifischen Widerstand, guter UV- und Temperaturstabilität und geringerem Dampfdruck erwünscht.

Derartige Matrix-Flüssigkristallanzeigen sind bekannt. Als nichtlineare Elemente zur individuellen Schaltung der einzelnen Bildpunkte können beispielsweise aktive Elemente (d.h. Transistoren) verwendet werden. Man spricht dann von einer "aktiven Matrix", wobei man zwei Typen unterscheiden kann:
1. MOS (Metal Oxide Semiconductor) oder andere Dioden auf Silizium-Wafer als Substrat.
2. Dünnfilm-Transistoren (TFT) auf einer Glasplatte als Substrat.

Die Verwendung von einkristallinem Silizium als Substratmaterial beschränkt die Displaygröße, da auch die modulartige Zusammensetzung verschiedener Teildisplays an den Stößen zu Problemen führt.

Bei dem aussichtsreicheren Typ 2, welcher bevorzugt ist, wird als elektrooptischer Effekt üblicherweise der TN-Effekt verwendet. Man unterscheidet zwei Technologien: TFTs aus Verbindungshalbleitern wie z.B. CdSe oder TFTs auf der Basis von polykristallinem oder amorphem Silizium. An letzterer Technologie wird weltweit mit großer Intensität gearbeitet.

Die TFT-Matrix ist auf der Innenseite der einen Glasplatte der Anzeige aufgebracht, während die andere Glasplatte auf der Innenseite die transparente Gegenelektrode trägt. Im Vergleich zu der Größe der Bildpunkt-Elektrode ist der TFT sehr klein und stört das Bild praktisch nicht. Diese Technologie kann auch für voll farbtaugliche Bilddarstellungen erweitert werden, wobei ein Mosaik von roten, grünen und blauen Filtern derart angeordnet ist, dass je ein Filterelement einem schaltbaren Bildelement gegenüber liegt.

Die TFT-Anzeigen arbeiten üblicherweise als TN-Zellen mit gekreuzten Polarisatoren in Transmission und sind von hinten beleuchtet.

Der Begriff MFK-Anzeigen umfasst hier jedes Matrix-Display mit integrierten nichtlinearen Elementen, d.h. neben der aktiven Matrix auch Anzeigen mit passiven Elementen wie Varistoren oder Dioden (MIM = Metall-Isolator-Metall).

Derartige MFK-Anzeigen eignen sich insbesondere für TV-Anwendungen (z.B. Taschenfernseher) oder für hochinformative Displays für Rechneranwendungen (Laptop) und im Automobil- oder Flugzeugbau. Neben Problemen hinsichtlich der Winkelabhängigkeit des Kontrastes und der Schaltzeiten resultieren bei MFK-Anzeigen Schwierigkeiten bedingt durch nicht ausreichend hohen spezifischen Widerstand der Flüssigkristallmischungen [TOGASHI, S., SEKIGUCHI, K., TANABE, H., YAMAMOTO, E., SORIMACHI, K., TAJIMA, E., WATANABE, H., SHIMIZU, H., Proc. Eurodisplay 84, Sept. 1984: A 210-288 Matrix LCD Controlled by Double Stage Diode Rings, p. 141 ff, Paris; STROMER, M., Proc. Eurodisplay 84, Sept. 1984: Design of Thin Film Transistors for Matrix Adressing of Television Liquid Crystal Displays, p. 145 ff, Paris]. Mit abnehmendem Widerstand verschlechtert sich der Kontrast einer MFK-Anzeige und es kann das Problem der "after image elimination" auftreten. Da der spezifische Widerstand der Flüssigkristallmischung durch Wechselwirkung mit den inneren Oberflächen der Anzeige im allgemeinen über die Lebenszeit einer MFK-Anzeige abnimmt, ist ein hoher (Anfangs)-Widerstand sehr wichtig, um akzeptable Standzeiten zu erhalten.

Insbesondere bei low-volt-Mischungen war es bisher nicht möglich, sehr hohe spezifische Widerstände zu realisieren. Weiterhin ist es wichtig, dass der spezifische Widerstand eine möglichst geringe Zunahme bei steigender Temperatur sowie nach Temperatur- und/oder UV-Belastung zeigt. Besonders nachteilig sind auch die Tieftemperatureigenschaften der Mischungen aus dem Stand der Technik. Gefordert wird, dass auch bei tiefen Temperaturen keine Kristallisation und/oder smektische Phasen auftreten und die Temperaturabhängigkeit der Viskosität möglichst gering ist. Die MFK-Anzeigen aus dem Stand der Technik genügen somit nicht den heutigen Anforderungen.

Neben Flüssigkristallanzeigen, die eine Hintergrundbeleuchtung verwenden, also transmissiv und gegebenenfalls transflektiv betrieben werden, sind besonders auch reflektive Flüssigkristallanzeigen interessant. Diese reflektiven Flüssigkristallanzeigen benutzen das Umgebungslicht zur Informationsdarstellung. Somit verbrauchen sie wesentlich weniger Energie als hintergrundbeleuchtete Flüssigkristallanzeigen mit entsprechender Größe und Auflösung. Da der TN-Effekt durch einen sehr guten Kontrast gekennzeichnet ist, sind derartige reflektive Anzeigen auch bei hellen Umgebungsverhältnissen noch gut abzulesen. Dies ist bereits von einfachen reflektiven TN-Anzeigen wie sie in z. B. Armbanduhren und Taschenrechnern verwendet werden, bekannt. Jedoch ist das Prinzip auch auf hochwertige, höher auflösende Aktiv-Matrix angesteuerte Anzeigen wie z. B. TFT-Displays anwendbar. Hier ist wie bereits bei den allgemeinen üblichen transmissiven TFT-TN-Anzeigen die Verwendung von Flüssigkristallen mit niedriger Doppelbrechung (Δn) nötig, um eine geringe optische Verzögerung (d · Δn) zu ereichen. Diese geringe optische Verzögerung führt zu einer meist akzeptablen geringen Blickwinkelabhängigkeit des Kontrastes (vgl. DE 30 22 818). Bei reflektiven Anzeigen ist die Verwendung von Flüssigkristallen mit kleiner Doppelbrechung noch wichtiger als bei transmissiven Anzeigen, da bei reflektiven Anzeigen die effektive Schichtdicke, die das Licht durchquert, ungefähr doppelt so groß ist wie bei transmissiven Anzeigen mit derselben Schichtdicke.

Es besteht somit immer noch ein großer Bedarf nach MFK-Anzeigen mit sehr hohem spezifischen Widerstand bei gleichzeitig großem Arbeitstemperaturbereich, kurzen Schaltzeiten auch bei tiefen Temperaturen und niedriger Schwellenspannung, die diese Nachteile nicht oder nur in geringerem Maße zeigen.

Bei TN-(Schadt-Helfrich)-Zellen sind Medien erwünscht, die folgende Vorteile in den Zellen ermöglichen:
- erweiterter nematischer Phasenbereich (insbesondere zu tiefen Temperaturen)
- lagerstabil, auch bei extrem tiefen Temperaturen
- Schaltbarkeit bei extrem tiefen Temperaturen (out-door-use, Automobil, Avionik)
- erhöhte Beständigkeit gegenüber UV-Strahlung (längere Lebensdauer)

Mit den aus dem Stand der Technik zur Verfügung stehenden Medien ist es nicht möglich, diese Vorteile unter gleichzeitigem Erhalt der übrigen Parameter zu realisieren.

Bei höher verdrillten Zellen (STN) sind Medien erwünscht, die eine höhere Multiplexierbarkeit und/oder kleinere Schwellenspannung und/oder breitere nematische Phasenbereiche (insbesondere bei tiefen Temperaturen) ermöglichen. Hierzu ist eine weitere Ausdehnung des zur Verfügung stehenden Parameterraumes (Klärpunkt, Übergang smektisch-nematisch bzw. Schmelzpunkt, Viskosität, dielektrische Größen, elastische Größen) dringend erwünscht.

Der Erfindung liegt die Aufgabe zugrunde, Medien insbesondere für derartige MFK-, TN- oder STN-Anzeigen bereitzustellen, welche die oben angegebenen Nachteile nicht oder nur in geringerem Maße, und vorzugsweise gleichzeitig sehr hohe spezifische Widerstände und niedrige Schwellenspannungen aufweisen.

Es wurde nun gefunden, dass diese Aufgabe gelöst werden kann, wenn man in Anzeigen erfindungsgemäße Medien verwendet. Die erfindungsgemäßen Medien zeichnen sich durch sehr kleine Rotationsviskositäten γ₁ in Kombination mit einem hohen Klärpunkt (T_{Cip}) und guten Tieftemperatureigenschaften aus.

In der JP 06329566 A und in der US 5380461 A werden fluorierte Stilbene beschrieben, die mit den Komponenten der Mischungen der vorliegenden Erfindung teilweise verwandt sind. Synthesemethoden zu dieser Verbindungsklasse werden dort offenbart.

Die folgenden Druckschriften beziehen sich alle auf die zitierten Derivate als Flüssigkristallkomponenten:

Die Druckschrift US 2001/0004108 A1 offenbart Difluorstilbenderivate mit 2 und 3 Ringgruppen als Bestandteil einer Flüssigkristallmischung aus Tolanen und Phenylcyclohexanen. Die Druckschrift JP 03-294386 A offenbart *trans*-Dihalogenstilbenderivate mit polaren Endgruppen. Die Druckschrift JP 06-329566 A offenbart Difluorstilbene mit zwei Ringgruppen mit weiteren Fluorsubstituenten. Die Druckschrift EP 0560382 A1 offenbart Difluorethylenderivate mit einem benachbarten Cyclohexanring. Die Druckschrift DE 10155073 A1 offenbart unpolare Difluorstilbene und weitere Mischungskomponenten, die aber keine Verbindungen mit einer Gruppe -CF₂O- umfassen. Die Druckschrift EP 1215270 A1 offenbart Flüssigkristallmischungen mit Difluorstilbenen für passive Displays. Die Druckschrift DE 10151491 A1 offenbart eine generische Gruppe von Verbindungen, die Difluorstilbene mit einer Endgruppe der Formel -SF₅ umfassen. Die Druckschrift DE 10124480 A1 offenbart eine generische Formel, die eine optionale Gruppe -CF=CF-zwischen Ringen und eine verbindliche Endgruppe der Formel -SF₅ umfasst. Die Druckschrift WO 2004/106460 A1 offenbart eine generische Formel mit 4 Ringen, die eine optionale Gruppe -CF=CF- zwischen Benzolringen und einen verbindlichen Tetrahydropyranring umfasst. Die Druckschrift EP 1416030 A1 offenbart ein optionale Cokomponente (IV) einer Flüssigkristallmischung, die optional ein Difluorstilben darstellt.

Die Druckschrift DE 102004008638 A1 offenbart flüssigkristalline Mischungen mit Verbindungen umfassend eine Gruppe -CF₂O-.

Gegenstand der Erfindung ist ein flüssigkristallines Medium mit positiver dielektrischer Anisotropie auf der Basis eines Gemisches von Verbindungen, **dadurch gekennzeichnet, dass** es eine oder mehrere Verbindungen der Formel I 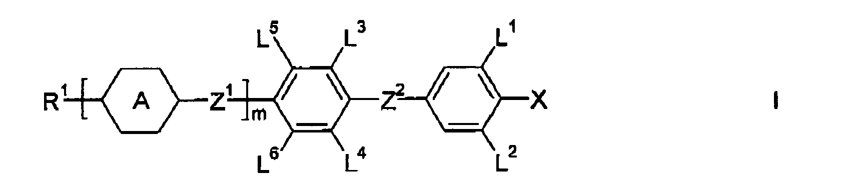 enthält,
worin
- R¹: einen halogenierten oder unsubstituierten Alkyl- oder Alkoxyrest mit 1 bis 15 C-Atomen, wobei in diesen Resten auch eine oder mehrere CH₂-Gruppen jeweils unabhängig voneinander durch -C≡C-, -CH=CH-, -O-, -CO-O- oder -O-CO- so ersetzt sein können, dass O-Atome nicht direkt miteinander verknüpft sind,
- Ring A: ein nach links oder rechts ausgerichtetes Ringsystem der Formeln 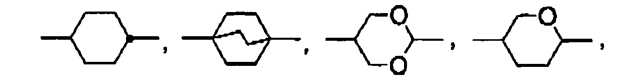 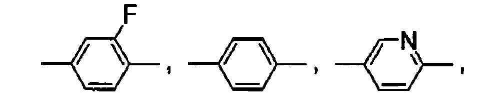 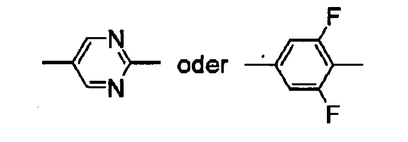
- Z¹, Z²: eine Einfachbindung, -C≡C-, -CF=CF-, -CH=CH-, -CF₂O-, oder -CH₂CH₂-, wobei mindestens eine Gruppe aus Z¹ und Z² die Gruppe -CF=CF- bedeutet,
- X: F, Cl, CN, SF₅ oder einen halogenierten oder unsubstituierten Alkyl- oder Alkoxyrest mit 1 bis 15 C-Atomen, wobei in diesen Resten auch eine oder mehrere CH₂-Gruppen jeweils unabhängig voneinander durch -C≡C-, -CH=CH-, -O-, -CO-O- oder -O-CO- so ersetzt sein können, dass O-Atome nicht direkt miteinander verknüpft sind, und
- L¹, L², L³, L⁴, L⁵ und L⁶: jeweils unabhängig voneinander H oder F, und
- m: 0 oder 1,
bedeuten,
und zusätzlich eine oder mehrere ausgewählte Verbindungen der allgemeinen Formeln K-1 bis K-12: 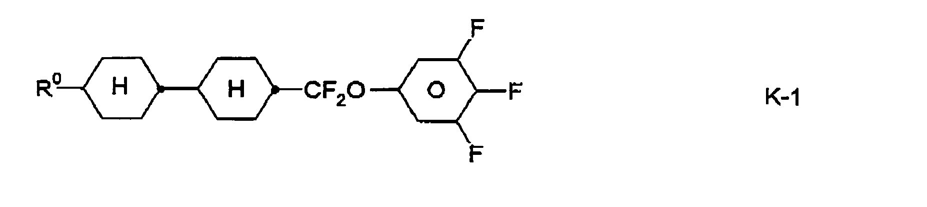 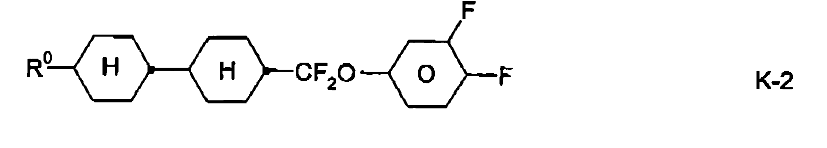 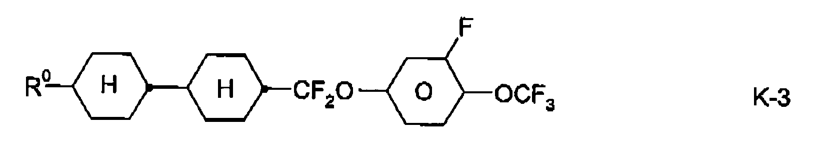 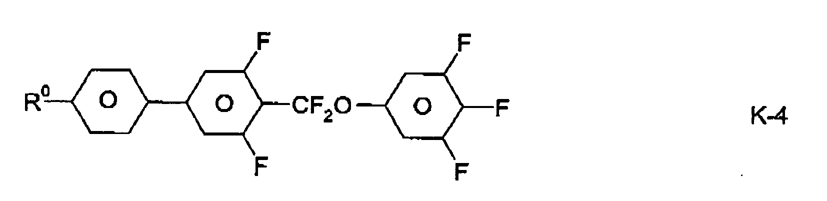 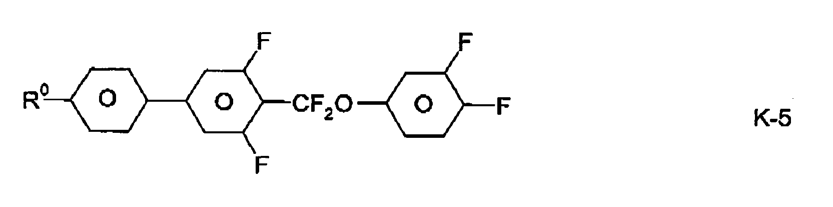 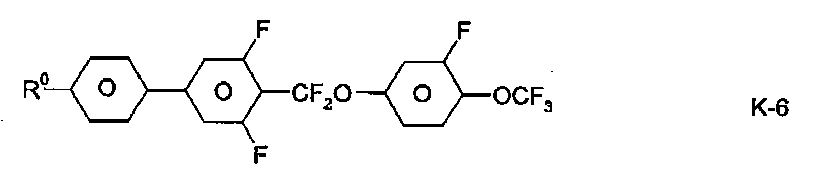 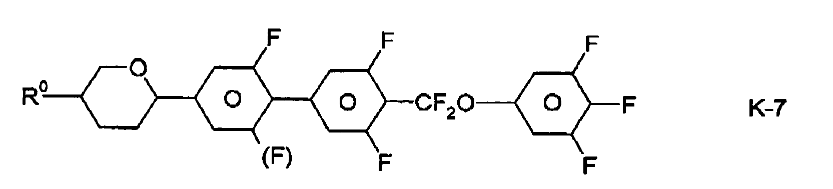 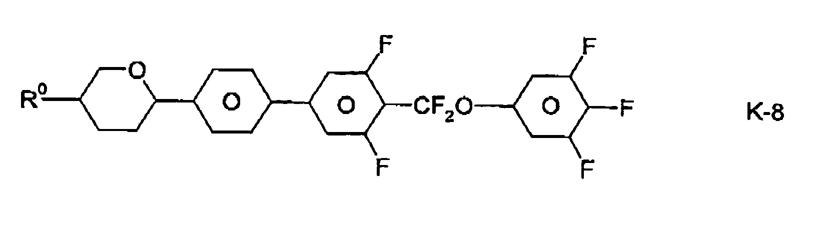 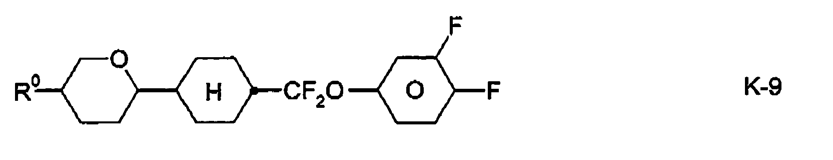 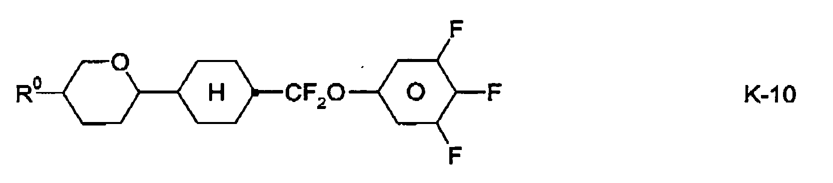 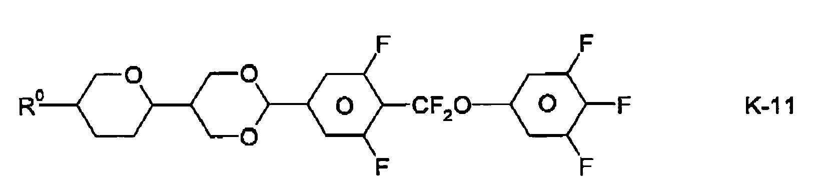 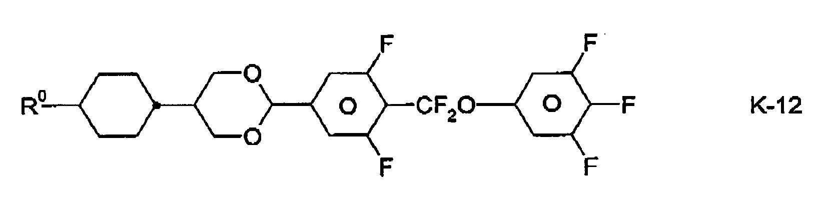 worin
- R⁰: n-Alkyl, Oxaalkyl, Fluoralkyl oder Alkenyl mit jeweils bis zu 9 C- Atomen
bedeutet.

Bevorzugt bedeuten 2, 3 oder 4 der Substituenten L¹, L², L³ und L⁴ in der Formel I Wasserstoff und die übrigen F. Besonders bevorzugt sind Medien mit Verbindungen der Formel I, worin L⁵ und L⁶ = H bedeuten. Für den Fall, dass m = 0 ist, sind besonders bevorzugt L¹ oder L² F. Für den Fall, dass m = 1 ist, sind besonders bevorzugt L¹ und L² H. X bedeutet vorzugsweise F, Cl, OCF₃, CF₃, SF₅, OCHF₂, OC₂F₅, OC₃F₇, OCHFCF₃, OCF₂CHFCF₃ oder einen Alkylrest mit 1 bis 8 C-Atomen. Ganz besonders bevorzugt sind Verbindungen, worin X einen Substituenten F, Cl, OCF₃ oder eine geradkettigen Alkylrest mit 1 bis 6 C-Atomen bedeutet. R¹ steht bevorzugt für einen unsubstituierten, geradkettigen 1-6 C Alkyl- oder Alkoxyrest oder einen entsprechenden 2-6 C Alkenylrest, ganz besonders für einen 1-6 C *n*-Alkylrest.

Zur Erzielung von Mischungen mit besonders hoher dielektrischer Anisotropie bedeutet der Substituent X bevorzugt F, Cl, OCF₃, CF₃, SF₅, OCHF₂, OC₂F₅, OC₃F₇, OCHFCF₃ oder OCF₂CHFCF₃, besonders bevorzugt F, CF₃ oder OCF₃, und ganz besonders bevorzugt F oder OCF₃.

Das Bindeglied Z¹ bedeutet bevorzugt eine Einfachbindung oder -CF=CF-. Das Bindeglied Z² bedeutet bevorzugt -CF=CF- oder -CF₂O-.

Ein weiterer Gegenstand der Erfindung sind Verbindungen der Formel I, worin m 1 und Z² eine Brücke -CF=CF- bedeutet (Verbindungen la). Dabei besitzen in den erfindungsgemäßen Verbindungen der Formel I die übrigen Strukturteile R¹, Ring A, Z¹, X und L¹⁻⁶ die oben angegebenen Bedeutungen sowie die oben angegebenen bevorzugten Bedeutungen.

Die Verbindungen der Formeln I besitzen einen breiten Anwendungsbereich. In Abhängigkeit von der Auswahl der Substituenten können diese Verbindungen als Basismaterialien dienen, aus denen flüssigkristalline Medien zum überwiegenden Teil zusammengesetzt sind; es können aber auch Verbindungen der Formeln I zu flüssigkristallinen Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung und/oder dessen Viskosität zu optimieren.

Die Verbindungen der Formeln I sind in reinem Zustand farblos und bilden flüssigkristalline Mesophasen in einem für die elektrooptische Verwendung günstig gelegenen Temperaturbereich. Chemisch und thermisch sind sie stabil.

Die erfindungsgemäßen Verbindungen besitzen allein und in Mischungen eine besonders niedrige Rotationsviskosität gegenüber anderen Verbindungen mit vergleichbaren physikalisch-chemischen Eigenschaften. Außerdem zeigen sie sehr gute Gesamteigenschaften, insbesondere im Hinblick auf das Verhältnis der Rotationsviskosität zum Klärpunkt (γ1/KIp.).

Für den Fall, dass m = 1 und Z¹ eine Gruppe -CF=CF- bedeutet, so ist der Ring A bevorzugt ein Ringsystem ausgewählt aus den Formeln 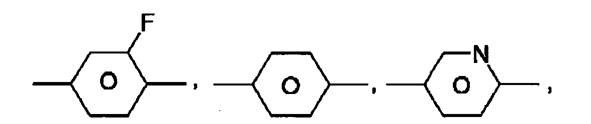 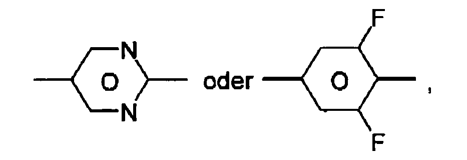 wobei die Ringe nach beiden Seiten ausgerichtet sein können.

Für den Fall, dass m = 1 und Z² eine Gruppe -CF=CF- bedeutet, so ist der Ring A bevorzugt ein Ringsystem ausgewählt aus den Formeln 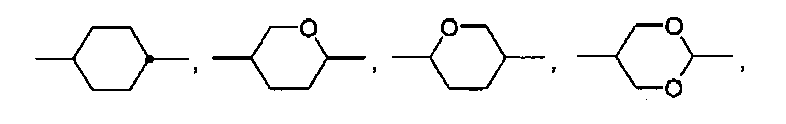 oder 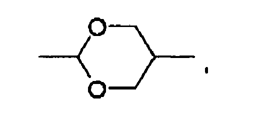 insbesondere der Formeln

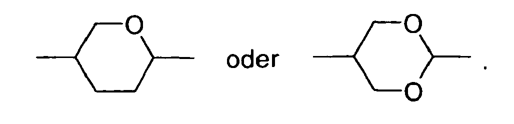

Bevorzugte Verbindungen gemäß der Erfindung sind **dadurch gekennzeichnet, dass**, unabhängig voneinander,
- m: 1 bedeutet,
- Z²: -CF=CF- und Z¹ eine Einfachbindung,
- X: F, -OCF₃, -CF₃, CN, 1-6 C n-Alkyl, oder 1-6 C n-Alkoxy, insbesondere F oder -OCF₃,
- L⁵, L⁶: H, oder
- R¹: 1-7 C Alkyl oder 2-7 C Alkylen
bedeutet.

Für den Fall, dass Z² eine Gruppe -CF=CF- bedeutet, sind L³ und L⁴ bevorzugt H.

Besonders bevorzugte erfindungsgemäße Verbindungen sind **dadurch gekennzeichnet, dass** genau eine Gruppe aus Z¹ und Z² eine Gruppe -CF=CF- darstellt. Folglich sind besonders bevorzugte erfindungsgemäße Verbindungen die der allgemeinen Formel Ib: 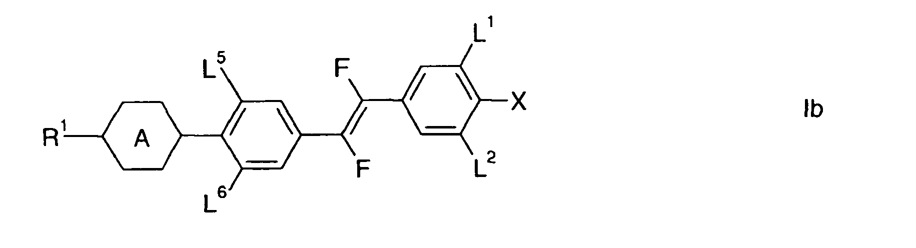 worin R¹, Ring A, X, L¹, L², L⁵ und L⁶ wie oben definiert sind.

Besonders bevorzugte Verbindungen der Formel I, worin der Ring A ein Tetrahydroyranring ist sind Verbindungen der Formel Ic:

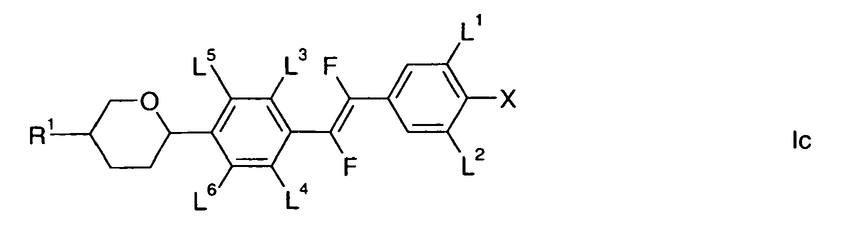

Falls R¹ in Formel I einen Alkylrest und/oder einen Alkoxyrest bedeutet, so kann dieser geradkettig oder verzweigt sein. Vorzugsweise ist er geradkettig, hat 2, 3, 4, 5, 6 oder 7 C-Atome und bedeutet demnach bevorzugt Ethyl, Propyl, Butyl, Pentyl, Hexyl, Heptyl, Ethoxy, Propoxy, Butoxy, Pentoxy, Hexoxy oder Heptoxy, ferner Methyl, Octyl, Nonyl, Decyl, Undecyl, Dodecyl, Tridecyl, Tetradecyl, Pentadecyl, Methoxy, Octoxy, Nonoxy, Decoxy, Undecoxy, Dodecoxy, Tridecoxy oder Tetradedoxy.

Oxaalkyl bedeutet vorzugsweise geradkettiges 2-Oxapropyl (= Methoxymethyl), 2-(= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6-, oder 7-Oxaoctyl, 2-, 3-, 4-, 5-, 6-, 7- oder 8-Oxanonyl, 2-, 3-, 4-, 5-, 6-, 7-, 8- oder 9-Oxadexyl.

Falls R¹ einen Alkylrest bedeutet, in dem eine CH₂-Gruppe durch -CH=CH-ersetzt ist, so kann dieser geradkettig oder verzweigt sein. Vorzugsweise ist er geradkettig und hat 2 bis 10 C-Atome. Er bedeutet demnach besonders Vinyl, Prop-1-, oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl, Oct-1-, 2-, 3-, 4-, 5-, 6- oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl.

Falls R¹ einen Alkylrest bedeutet, in dem eine CH₂-Gruppe durch -O- und eine durch -CO- ersetzt ist, so sind diese bevorzugt benachbart. Somit beinhalten diese eine Acyloxygruppe -CO-O- oder eine Oxycarbonylgruppe -O-CO-. Vorzugsweise sind diese geradkettig und haben 2 bis 6 C-Atome.

Sie bedeuten demnach besonders Acetyloxy, Propionyloxy, Butyryloxy, Pentanoyloxy, Hexanoyloxy, Acetyloxymethyl, Propionyloxymethyl, Butyryloxymethyl, Pentanoyloxymethyl, 2-Acetyloxyethyl, 2-Propionyloxyethyl, 2-Butyryloxyethyl, 2-Acetyloxypropyl, 3-Propionyl-oxypropyl, 4-Acetyl-oxybutyl, Methoxycarbonyl, Ethoxycarbonyl, Propoxy-carbonyl, Butoxycarbonyl, Pentoxycarbonyl, Methoxycarbonylmethyl, Ethoxycarbonylmethly, Propoxycarbonylmethyl, Butoxycarbonylmethyl, 2-(Methoxycarbonyl)ethyl, 2-(Ethoxycarbonyl)ethyl, 2-(Propoxycarbonyl)-ethyl, 3-(Methoxycarbonyl)-propyl, 3-(Ethoxy-carbonyl)-propyl oder 4-(Methoxycarbonyl)-butyl.

Falls R¹ einen Alkylrest bedeutet, in dem eine CH₂-Gruppe durch unsubstituiertes oder substituiertes -CH=CH- und eine benachbarte CH₂-Gruppe durch CO oder CO-O oder O-CO ersetzt ist, so kann dieser geradkettig oder verzweigt sein. Vorzugsweise ist er geradkettig und hat 4 bis 12 C-Atome. Er bedeutet demnach besonders Acryloyloxymethyl, 2-Acryloyloxyethyl, 3-Acryloyloxypropyl, 4-Acryloyloxybutyl, 5-Acryloyloxypentyl, 6-Acryloyloxyhexyl, 7-Acryloyloxyheptyl, 8-Acryloyloxyoctyl, 9-Acryloyloxynonyl, 10-Acryloyloxydecyl, Methacryloyloxymethyl, 2-Methacryloyloxyethyl, 3-Methacryloyloxypropyl, 4-Methacryloyloxybutyl, 5-Methacryloyloxypentyl, 6-Methacryloyloxyhexyl, 7-Methacryloyloxyheptyl, 8-Methacryloyloxyoctyl, 9-Methacryloyloxynonyl.

Falls R¹ einen einfach durch CN oder CF₃ substituerten Alkyl- oder Alkenylrest bedeutet, so ist dieser Rest vorzugsweise geradkettig. Die Substitution durch CN oder CF₃ ist in beliebiger Position.

Falls R¹ einen mindestens einfach durch Halogen substituierten Alkyl- oder Alkenylrest bedeutet, so ist dieser Rest vorzugsweise geradkettig und Halogen ist vorzugsweise F oder Cl. Bei Mehrfachsubstitution ist Halogen vorzugsweise F. Die resultierenden Reste schließen auch perfluorierte Reste ein. Bei Einfachsubstitution kann der Fluor- oder Chlorsubstituent in beliebiger Position sein, vorzugsweise jedoch in ω-Position.

Verbindungen mit verzweigten Flügelgruppen R¹ können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Smektische Verbindungen dieser Art eignen sich als Komponenten für ferroelektrische Materialien.

Verzweigte Gruppen dieser Art enthalten in der Regel nicht mehr als eine Kettenverzweigung. Bevorzugt verzweigte Reste R¹ sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy.

Falls R¹ einen Alkylrest darstellt, in dem zwei oder mehr CH₂-Gruppen durch -O- und/oder -CO-O- ersetzt sind, so kann dieser geradkettig oder verzweigt sind. Vorzugsweise ist er verzweigt und hat 3 bis 12 C-Atome. Er bedeutet demnach besonders Bis-carboxy-methyl, 2,2-Bis-carboxy-ethyl, 3,3-Bis-carboxy-propyl, 4,4-Bis-carboxy-butyl, 5,5-Bis-carboxy-pentyl, 6,6-Bis-carboxy-hexyl, 7,7-Bis-carboxy-heptyl, 8,8-Bis-carboxy-octyl, 9,9-Bis-carboxy-nonyl, 10,10-Bis-carboxy-decyl, Bis-(methoxy-carbonyl)-methyl, 2,2-Bis-(methoxycarbonyl)-ethyl, 3,3-Bis-(methoxy-carbonyl)-propyl, 4,4-Bis-(methoxycarbonyl)-butyl, 5,5-Bis-(methoxy-carbonyl)-pentyl, 6,6-Bis-(methoxycarbonyl)-hexyl, 7,7-Bis-(methoxy-carbonyl)-heptyl, 8,8-Bis-(methoxycarbonyl)-octyl, Bis-(ethoxycarbonyl)-methyl, 2,2-Bis-(ethoxycarbonyl)-ethyl, 3,3-Bis-(ethoxycarbonyl)-propyl, 4,4-Bis-(ethoxycarbonyl)-butyl, 5,5-Bis-(ethoxycarbonyl)-hexyl.

Die Verbindungen der Formeln I werden nach an sich bekannten Methoden dargestellt, wie sie in der Literatur (z. B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Geeignete Verfahren zur Herstellung sind in Schema 1 und Schema 2 skizziert.

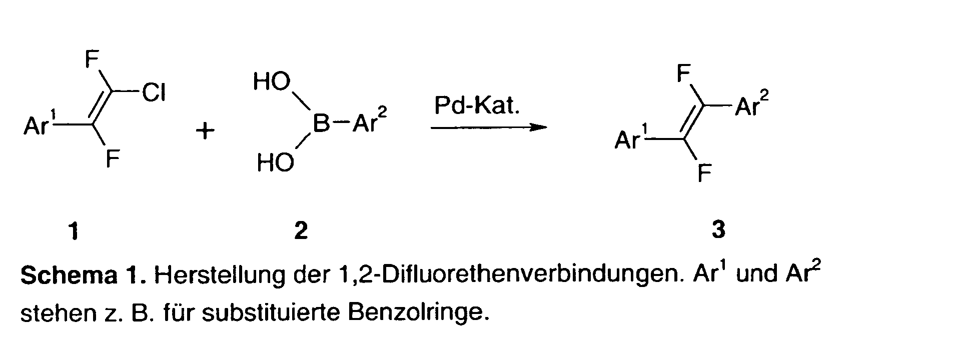

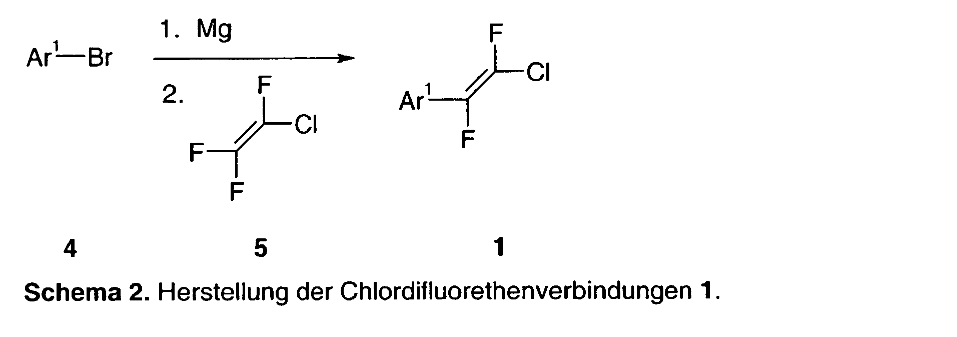

Schema **1** zeigt, wie durch Palladium-katalysierte Verknüpfung einer Chlordifluorethenverbindung der Formel **1** mit einer Boronsäureverbindung der Formel **2** die erfindungsgemäßen Difluorethenverbindungen **3** hergestellt werden können. Die Formel **3** ist analog zu Formel I. Die Reste Ar¹ und Ar² stellen entsprechend substituierte, aromatische Ringsysteme dar.

Die Ausgangsverbindungen der Formel **1** lassen sich aus einem Arylhalogenid **4** durch Halogen-Metall-Austausch und Reaktion mit Chlortrifluorethylen herstellen. Bei der dargestellten Synthesestrategie fällt das gewünschte *E*-Isomer der Formel **3** im Überschuss zum *Z*-Isomer an. Das gewünschte Isomer lässt sich chromatographisch und durch Kristallisation leicht isolieren.

Gegenstand der Erfindung sind auch elektrooptische Anzeigen (insbesondere STN- oder MFK-Anzeigen mit zwei planparallelen Trägerplatten, die mit einer Umrandung eine Zelle bilden, integrierten nicht-linearen Elementen zur Schaltung einzelner Bildpunkte auf den Trägerplatten und einer in der Zelle befindlichen nematischen Flüssigkristallmischung mit positiver dielektrischer Anisotropie und hohem spezifischem Widerstand), die derartige Medien enthalten sowie die Verwendung dieser Medien für elektrooptische Zwecke.

Die erfindungsgemäßen Flüssigkristallmischungen ermöglichen eine bedeutende Erweiterung des zur Verfügung stehenden Parameterraumes. Die erzielbaren Kombinationen aus Klärpunkt, Viskosität bei tiefer Temperatur, thermischer Stabilität und dielektrischer Anisotropie übertreffen bei weitem bisherige Materialien aus dem Stand der Technik.

Die Verbindungen der Formel I werden erfindungsgemäß mit weiteren hochpolaren Komponenten mit Δε > 8 und mit einer oder mehreren neutralen Komponenten (-1,5 < Δε < 3), die - wenigstens zum Teil - gleichzeitig eine geringe optische Anisotropie (Δn < 0,08) besitzen, kombiniert um die flüssigkristallinen Medien zu erhalten.

Die erfindungsgemäßen Flüssigkristallmischungen ermöglichen es unter Beibehaltung der nematischen Phase bis -20 °C und bevorzugt bis -30 °C, besonders bevorzugt bis -40 °C, einen Klärpunkt oberhalb 60 °C, vorzugsweise oberhalb 65 °C, besonders bevorzugt oberhalb 70 °C, gleichzeitig dielektrische Anisotropiewerte Δε ≥ 3, vorzugsweise ≥ 5, insbesondere auch ≥ 7 und einen hohen Wert für den spezifischen Widerstand zu erreichen, wodurch hervorragende STN- und MFK-Anzeigen erzielt werden können. Insbesondere sind die Mischungen durch sehr geringe Rotationsviskositäten gekennzeichnet. Die Rotationsviskositäten γ₁ liegen unterhalb 90 mPa·s, vorzugsweise unterhalb 80 mPa·s, besonders bevorzugt unter 70 mPa·s. Die Operationsspannungen liegen gleichzeitig, abhängig von der gewählten dielektrischen Anisotropie des Mediums, bei niedrigen Werten.

Es versteht sich, dass durch geeignete Wahl der Komponenten der erfindungsgemäßen Mischungen auch höhere Klärpunkte (z.B. oberhalb 90 °C) bei höheren Schwellenspannungen oder niedrigere Klärpunkte bei niedrigeren Schwellenspannungen unter Erhalt der anderen vorteilhaften Eigenschaften realisiert werden können. Ebenso können bei entsprechend wenig erhöhten Viskositäten Mischungen mit größerem Δε und somit geringen Schwellen oder Mischungen mit höheren Klärpunkten erhalten werden. Die erfindungsgemäßen MFK-Anzeigen arbeiten vorzugsweise im ersten Transmissionsminimum nach Gooch und Tarry [C.H. Gooch und H.A. Tarry, Electron. Lett. 10, 2-4, 1974; C.H. Gooch und H.A. Tarry, Appl. Phys., Vol. 8, 1575-1584, 1975], wobei hier neben besonders günstigen elektrooptischen Eigenschaften, wie z.B. hohe Steilheit der Kennlinie und geringe Winkelabhängigkeit des Kontrastes (DE 3022818 A1) bei gleicher Schwellenspannung wie in einer analogen Anzeige im zweiten Minimum, eine kleinere dielektrische Anisotropie ausreichend ist. Hierdurch lassen sich unter Verwendung der erfindungsgemäßen Mischungen im ersten Minimum deutlich höhere spezifische Widerstände verwirklichen als bei Mischungen mit Cyanverbindungen. Der Fachmann kann durch geeignete Wahl der einzelnen Komponenten und deren Gewichtsanteilen mit einfachen Routinemethoden die für eine vorgegebene Schichtdicke der MFK-Anzeige erforderliche Doppelbrechung einstellen.

Die Fließviskosität ν₂₀ bei 20 °C ist vorzugsweise < 60 mm²·s⁻¹, besonders bevorzugt < 50 mm²·s⁻¹. Die Rotationsviskosität γ₁ der erfindungsgemäßen Mischungen bei 20 °C ist vorzugsweise < 80 mPa·s, besonders bevorzugt < 70 mPa·s. Der nematische Phasenbereich hat vorzugsweise eine Breite von mindestens 90°C, insbesondere von mindestens 100°C. Vorzugsweise erstreckt sich dieser Bereich mindestens von -20° bis +70°C.

Bei Flüssigkristallanzeigen ist eine kleine Schaltzeit erwünscht. Dies gilt besonders für Anzeigen für die Videowiedergabe. Für derartige Anzeigen werden Schaltzeiten (Summe: tₒₙ + t_{off}) von maximal 16 ms benötigt. Die Obergrenze der Schaltzeit wird durch die Bildwiederholfrequenz bestimmt. Neben der Rotationsviskosität γ₁ beeinflußt auch der Tiltwinkel die Schaltzeit.

Messungen des "Voltage Holding-ratio" (HR) [S. Matsumoto et al., Liquid Crystals 5,1320 (1989); K. Niwa et al., Proc. SID Conference, San Francisco, June 1984, p. 304 (1984); G. Weber et al., Liquid Crystals 5, 1381 (1989)] haben ergeben, dass erfindungsgemäße Mischungen enthaltend Verbindungen der Formel I eine deutlich kleinere Abnahme des HR mit steigender Temperatur aufweisen als analoge Mischungen enthaltend anstelle den Verbindungen der Formel I Cyanophenylcyclohexane der Formel 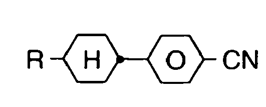 oder Ester der Formel R

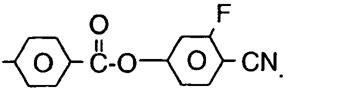

Besonders bevorzugte Flüssigkristalline Medien enthalten eine oder mehrere Verbindungen aus den Formeln I-1 bis I-30: 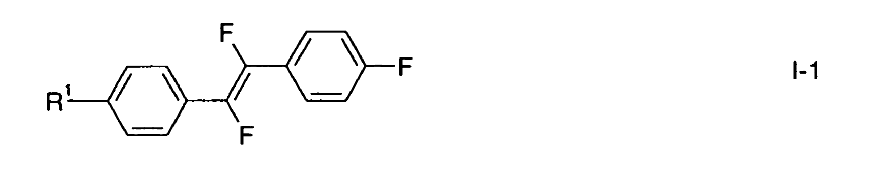 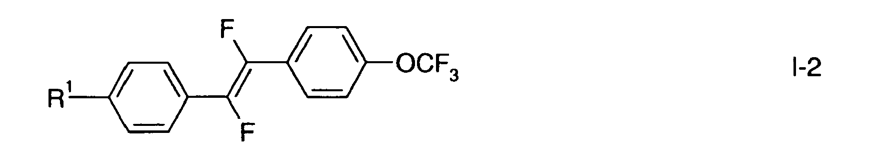 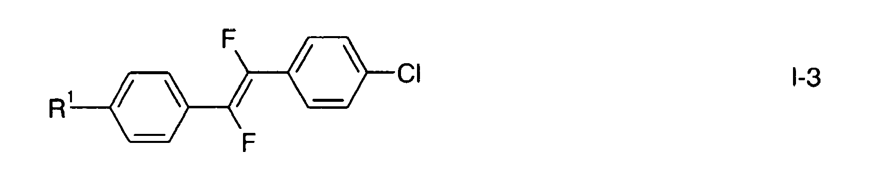 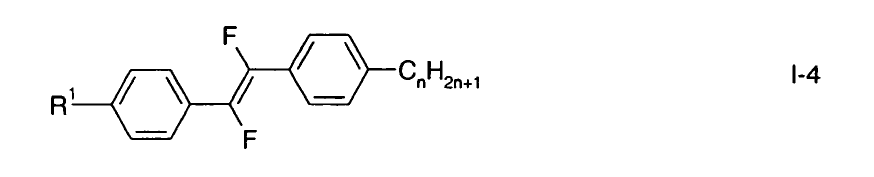 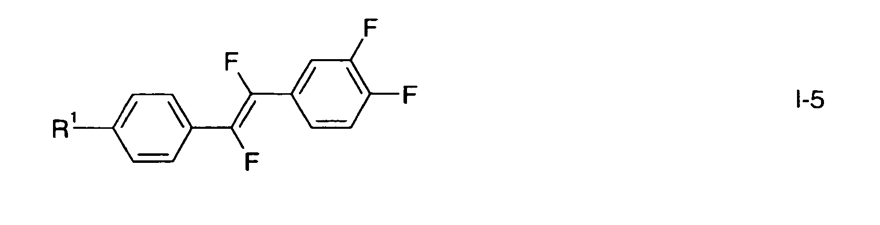 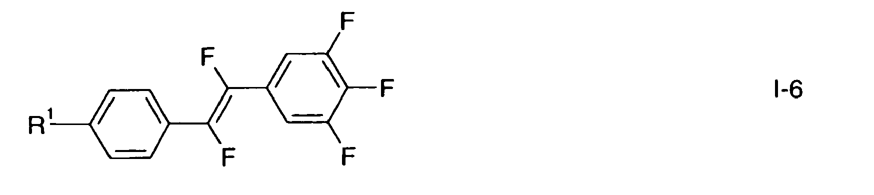 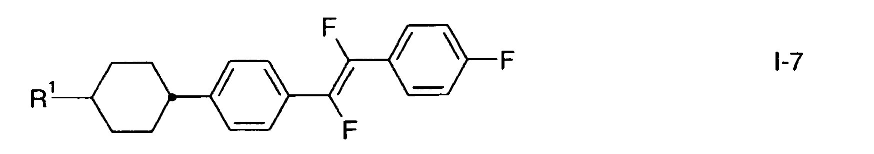 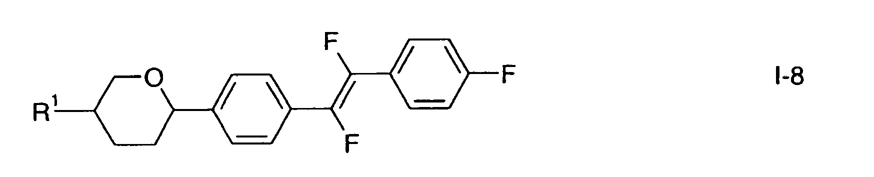 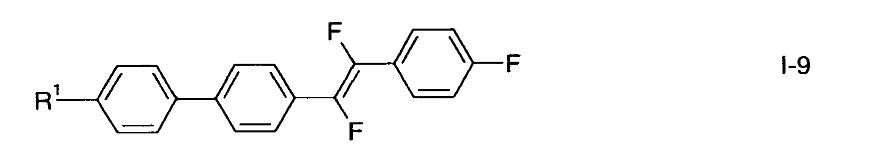 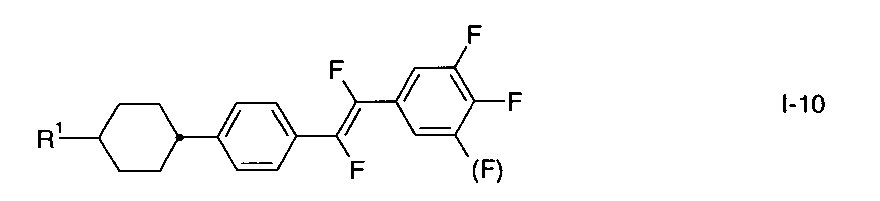 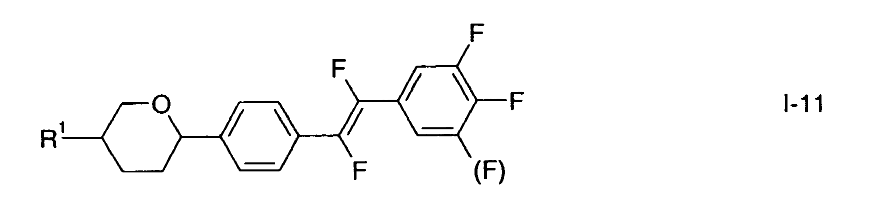 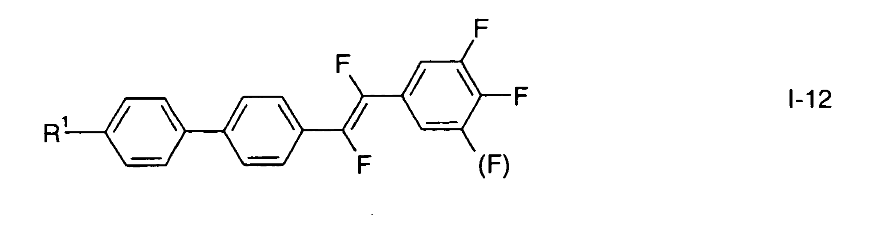 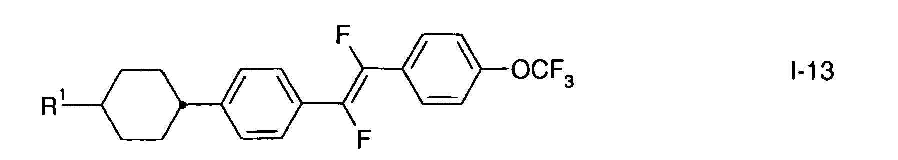 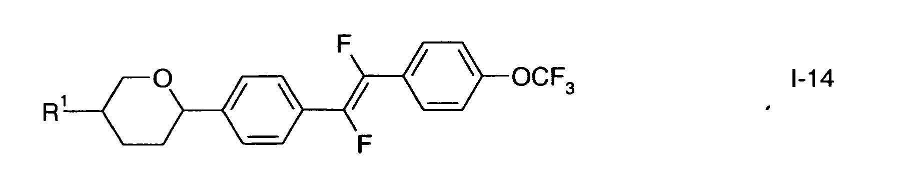 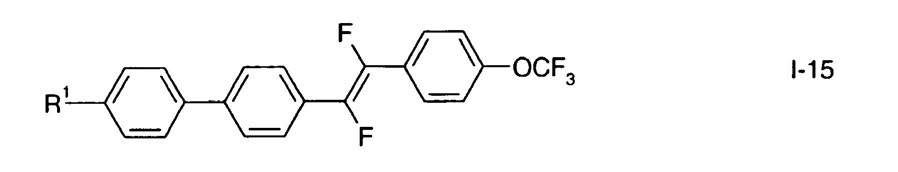 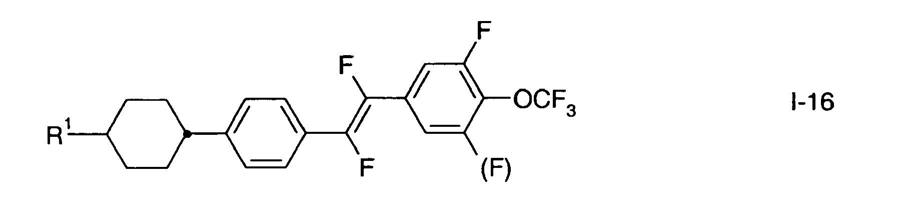 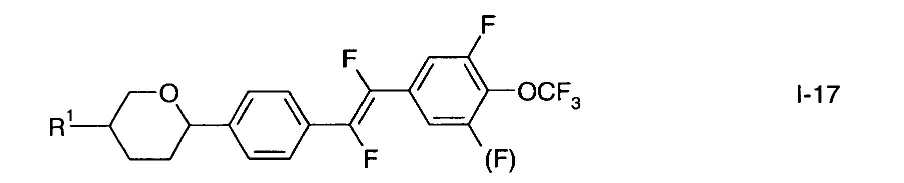 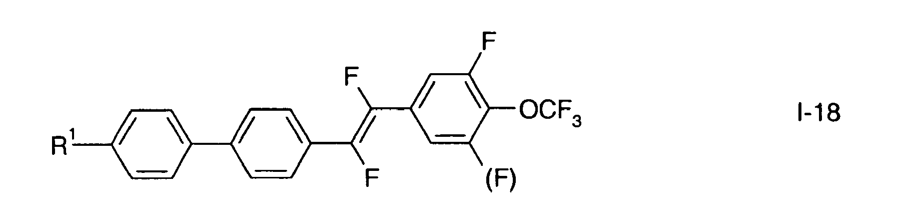 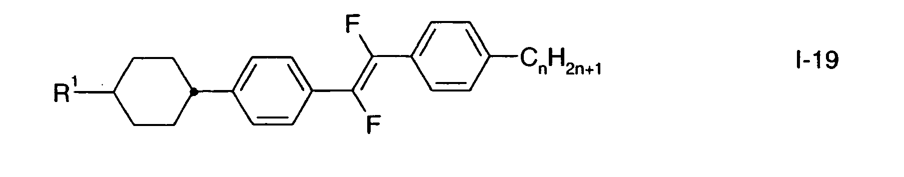 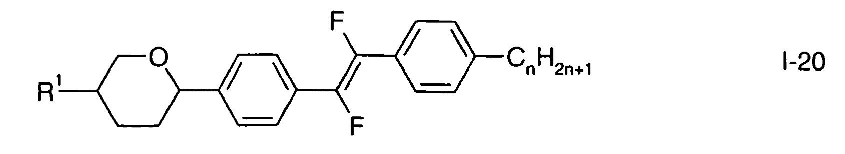 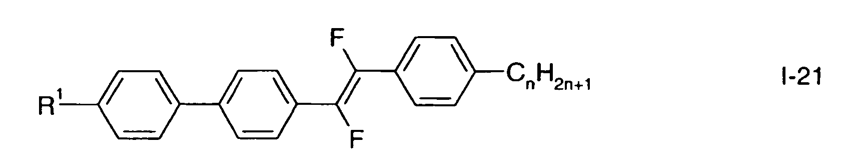 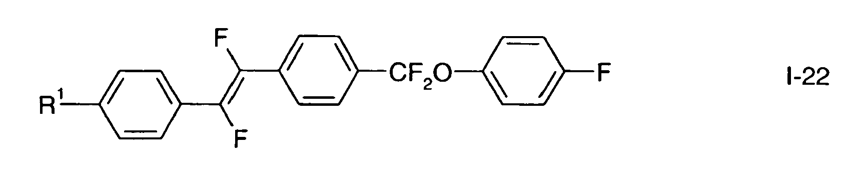 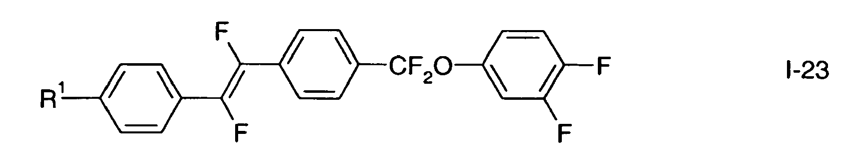 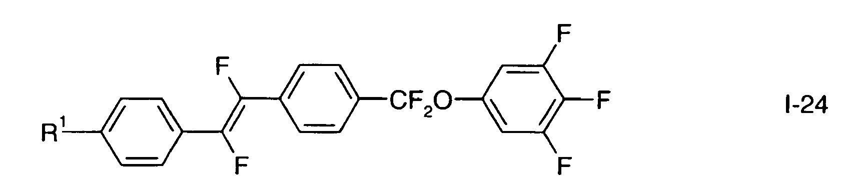 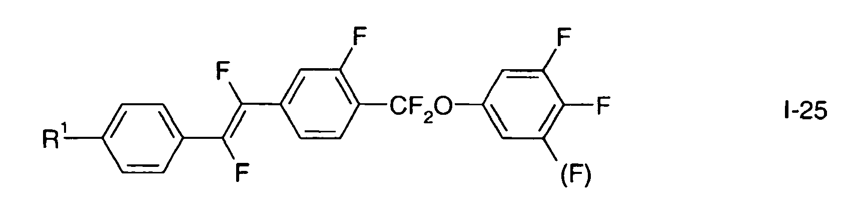 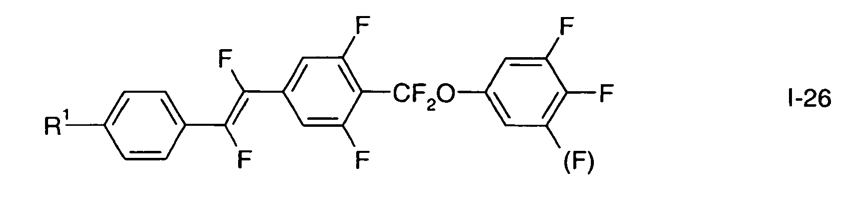 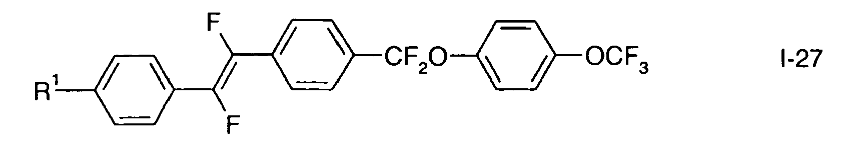 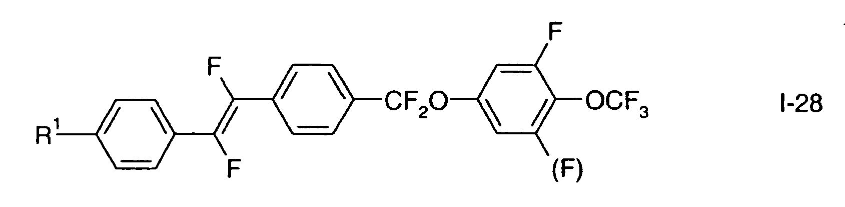 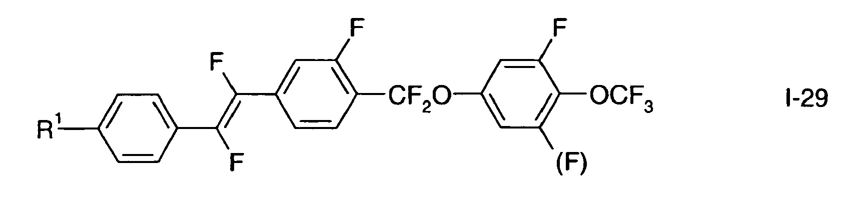 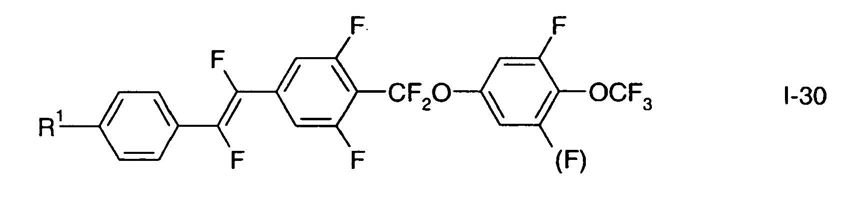 worin R¹ die in Formel I angegebene Bedeutung hat.

Von diesen bevorzugten Verbindungen sind besonders bevorzugt unter Verbindungen mit zwei Ringen (m = 0) solche der Formeln I-1, I-2, I-3, I-4 und I-5, ganz besonders die der Formeln I-1, I-2 und I-4. Besonders bevorzugt unter den Dreiringverbindungen sind solche der Formeln I-7, I-8, I-10, I-13, I-14, I-16, I-19, I-22, I-23, I-24, I-27 und I-28, ganz besonders die der Formeln I-8 und I-14.

Bevorzugte Ausführungsformen der erfindungsgemäßen flüssigkristallinen Medien sind im Folgenden angegeben:
- Das flüssigkristallines Medium ist **dadurch gekennzeichnet, dass** der Anteil an Verbindungen der Formel I im Gesamtgemisch 0,5 bis 40 Gew.% beträgt; bevorzugt beträgt er 4 bis 20 Gew.%.
- Das Medium enthält ein, zwei oder mehrere Verbindungen der Formeln I-1 bis I-30;
- Das Medium enthält zusätzlich eine oder mehrere Verbindungen ausgewählt aus der Gruppe bestehend aus den allgemeinen Formeln II bis VI: 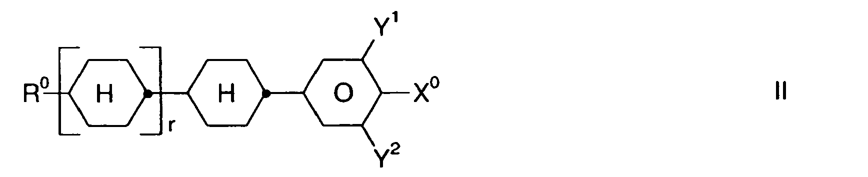 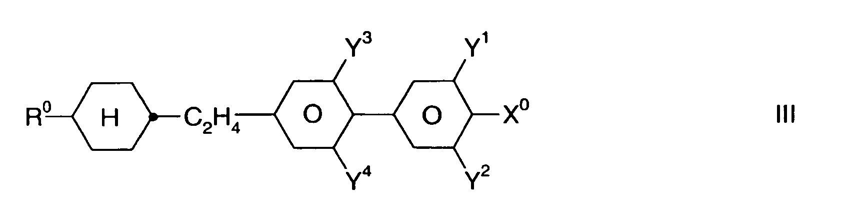 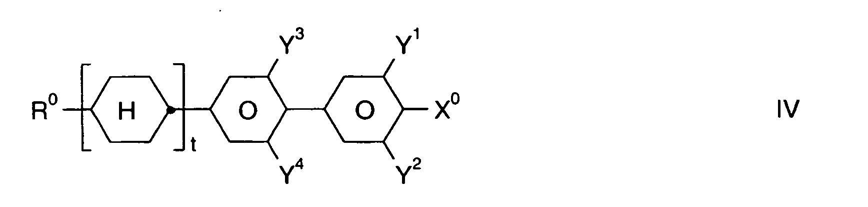 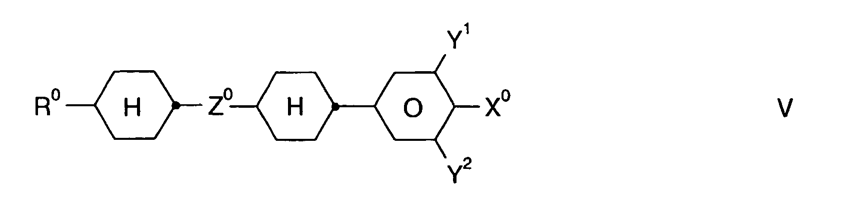 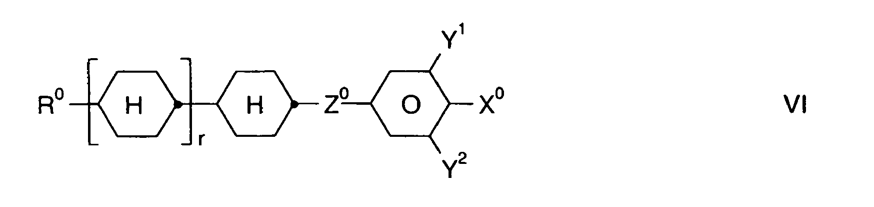 worin die einzelnen Reste die folgenden Bedeutungen haben:
   - R⁰: n-Alkyl, Alkoxy, Oxaalkyl, Fluoralkyl oder Alkenyl mit jeweils bis zu 9 C-Atomen,
   - X⁰: F, Cl, halogeniertes Alkyl, halogeniertes Alkenyl, halogeniertes Oxalkyl, halogeniertes Alkenyloxy oder halogeniertes Alkoxy mit bis zu 6 C-Atomen,
   - Z⁰: -C₂F₄-, -CF=CF-, -C₂H₄-, -(CH₂)₄-, -OCH₂-, -CH₂O-, -CF₂O- oder -OCF₂-,
   - Y¹ bis Y⁴: jeweils unabhängig voneinander H oder F,
   - r: 0 oder 1, und
   - t: 0, 1 oder 2.

Die Verbindung der Formel IV ist vorzugsweise 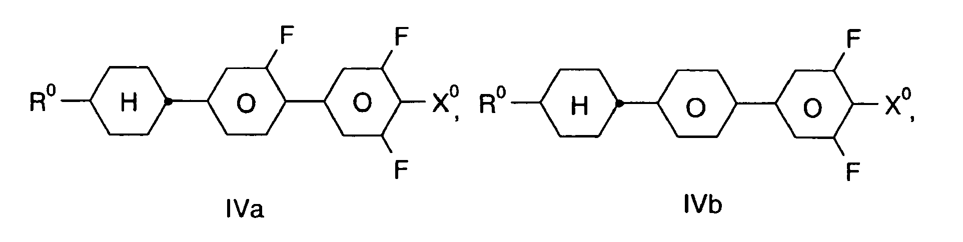 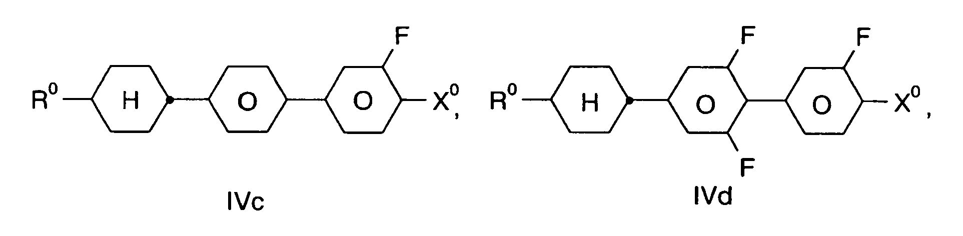 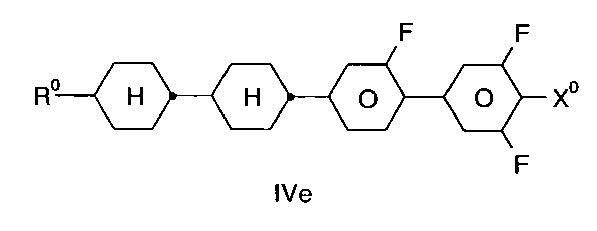 oder 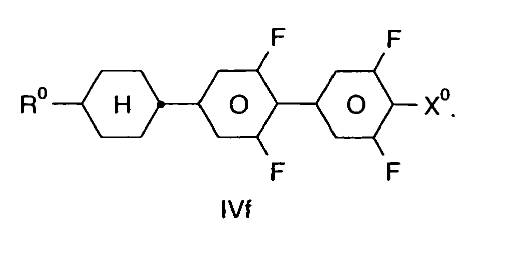
- Das Medium enthält zusätzlich eine oder mehrere Verbindungen ausgewählt aus der Gruppe bestehend aus den allgemeinen Formeln VII bis XIII: 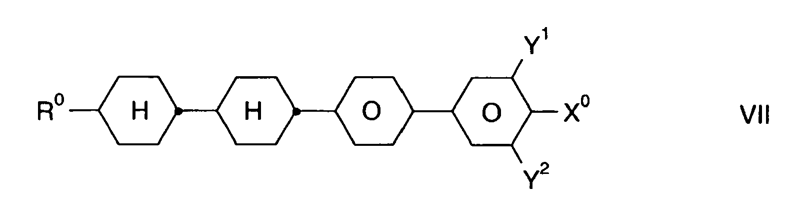 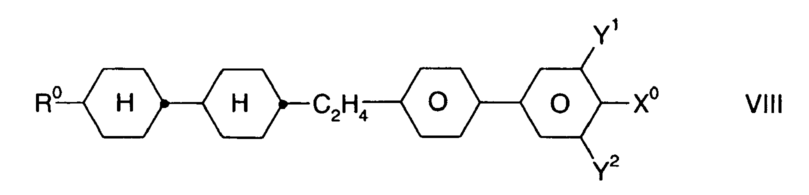 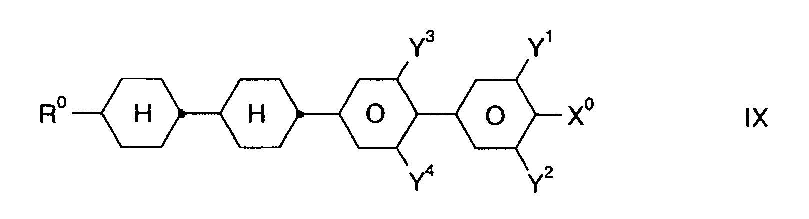 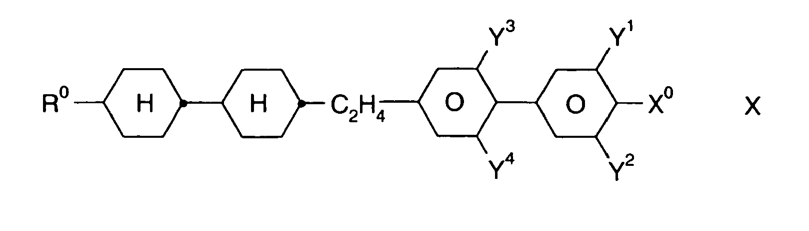 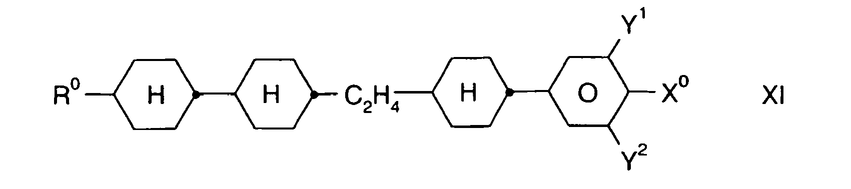 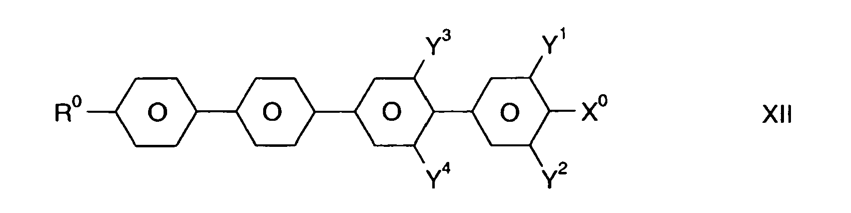 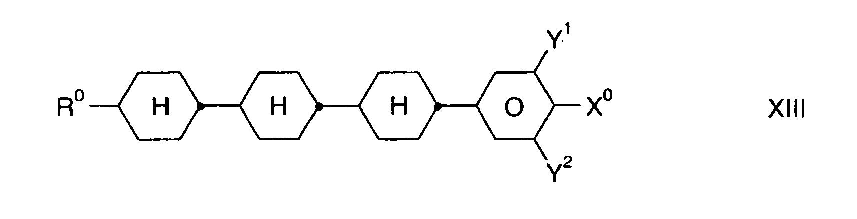 worin
   - R⁰: n-Alkyl, Oxaalkyl, Fluoralkyl oder Alkenyl mit jeweils bis zu 9 C-Atomen,
   - X⁰: F, Cl, halogeniertes Alkyl, halogeniertes Alkenyl, halogeniertes Alkenyloxy oder halogeniertes Alkoxy mit bis zu 6 C-Atomen, und
   - Y¹ bis Y⁴: jeweils unabhängig voneinander H oder F
bedeutet.

X⁰ ist hier vorzugsweise F, Cl, CF₃, OCF₃ oder OCHF₂. R⁰ bedeutet hier vorzugsweise Alkyl, Alkoxy, Oxaalkyl, Fluoralkyl oder Alkenyl mit jeweils bis zu 6 C-Atomen.

Das Medium enthält zusätzlich eine oder mehrere Verbindungen der Formeln E-a bis E-d 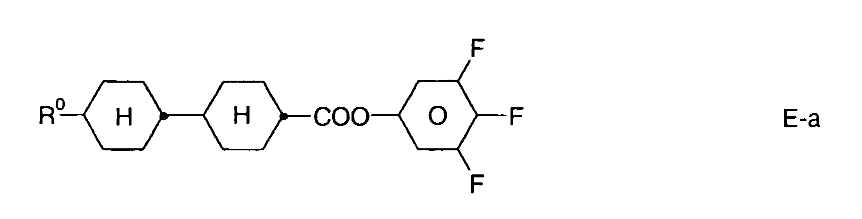 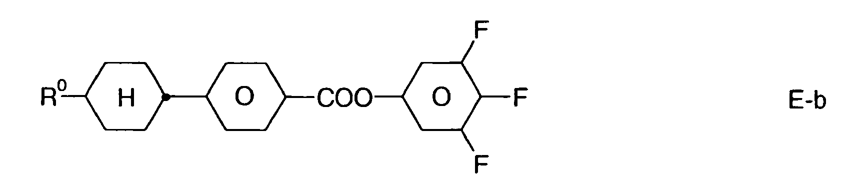 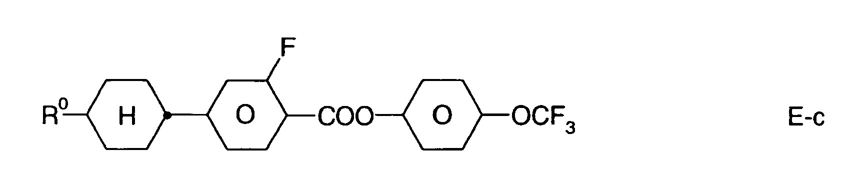 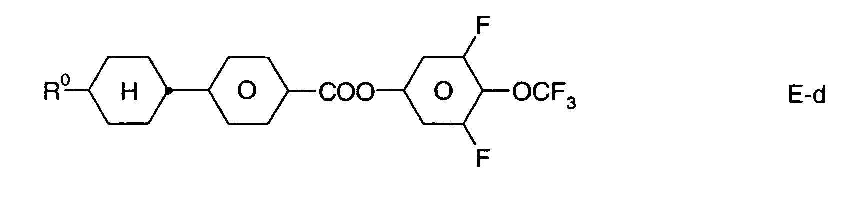 worin
- R⁰: n-Alkyl, Oxaalkyl, Fluoralkyl oder Alkenyl mit jeweils bis zu 9 C-Atomen
bedeutet.
- Der Anteil an Verbindungen der Formel I beträgt im Gesamtgemisch 0,5 bis 40 Gew.%, besonders bevorzugt 1 bis 30 Gew.%;
- Der Anteil der Verbindungen der Formeln E-a bis E-d ist vorzugsweise 5-30 Gew.%, insbesondere 5-25 Gew.%;
- Der Anteil an Verbindungen der Formeln I bis VI zusammen beträgt im Gesamtgemisch mindestens 30 Gew.%;
- Der Anteil an Verbindungen der Formeln II bis VI im Gesamtgemisch beträgt 30 bis 80 Gew.%; 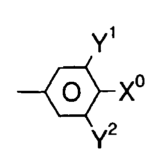 ist vorzugsweise 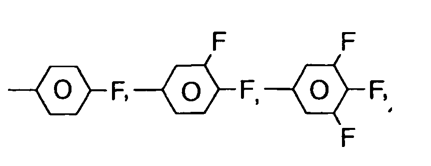 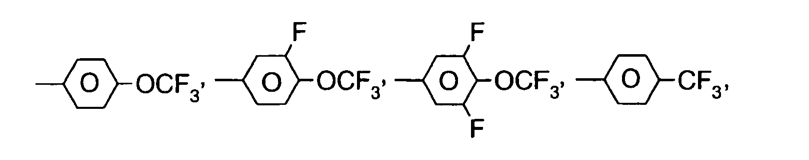 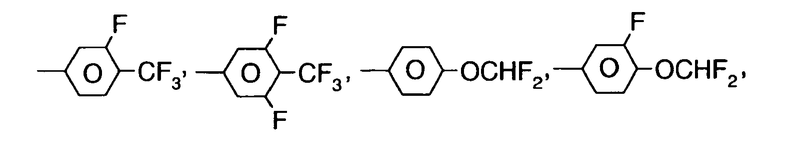 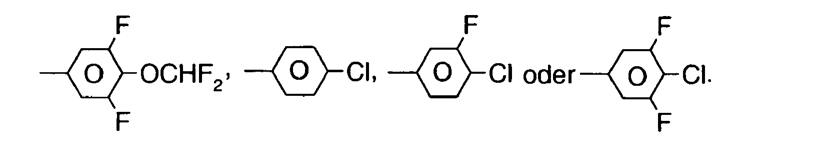
- Das Medium enthält Verbindungen der Formeln II, III, IV, V und/oder VI;
- R⁰ ist in allen Verbindungen bevorzugt geradkettiges Alkyl oder Alkenyl mit 2 bis 7 C-Atomen;
- Das Medium enthält weitere Verbindungen aus der Klasse der fluorierten Terphenyle mit R⁰ und/oder X⁰, wie unten definiert, als Endgruppen, vorzugsweise ausgewählt aus der folgenden Gruppe bestehend aus den allgemeinen Formeln XIV und XV: 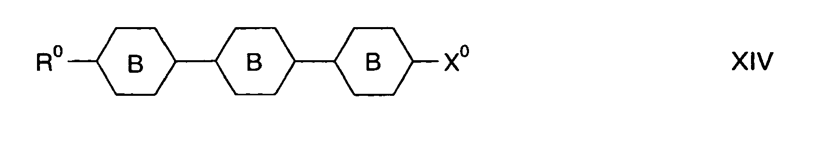 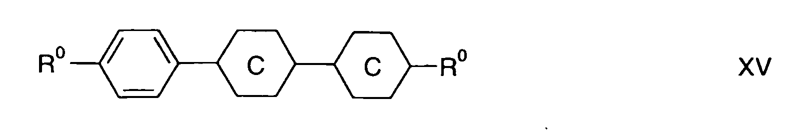 worin
   - R⁰: n-Alkyl, Oxaalkyl, Fluoralkyl oder Alkenyl mit jeweils bis zu 9 C-Atomen,
   - X⁰: F, Cl, halogeniertes Alkyl, halogeniertes Alkenyl, halogeniertes Alkenyloxy oder halogeniertes Alkoxy mit bis zu 6 C-Atomen, und
   - Ring B und C,: unabhängig voneinander, 1,4-Phenylen mit 0, 1, oder 2 Fluor substituiert,
bedeutet.

Vorzugsweise ist in Formel XIV und XV je mindestens einer der 1,4-Phenylenringe ein- oder mehrfach durch Fluoratome substituiert. Bevorzugt sind in Verbindungen der Formel XIV zwei der Phenylene durch mindestens ein Fluoratom substituiert oder eines der Phenylene durch 2 Fluoratome substituiert; in Verbindungen der Formel XV ist bevorzugt eines der Phenylene durch mindestens ein Fluoratom substituiert. X⁰ ist hier vorzugsweise F, Cl, CF₃, OCF₃ oder OCHF₂. R⁰ bedeutet hier vorzugsweise Alkyl, Alkoxy, Oxaalkyl, Fluoralkyl oder Alkenyl mit jeweils bis zu 6 C-Atomen.

Vorzugsweise handelt es sich bei Verbindungen der Formel XIV um Verbindungen der Formeln XIV-1 bis XIV-5: 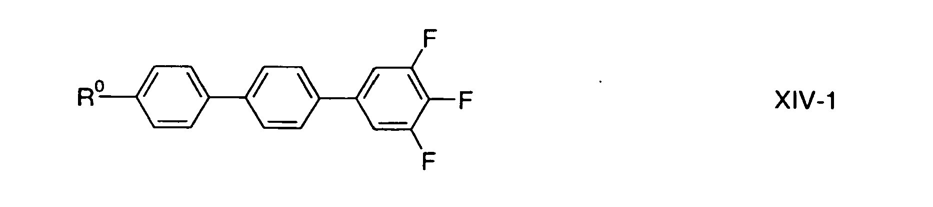 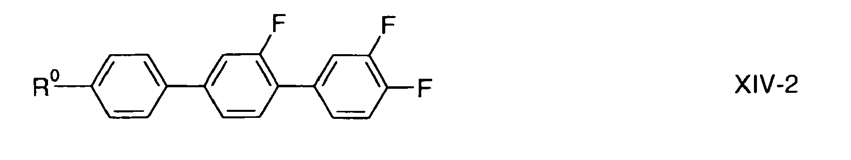 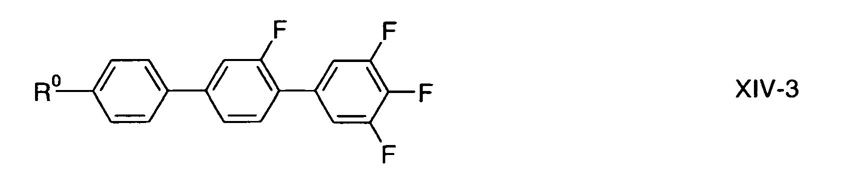 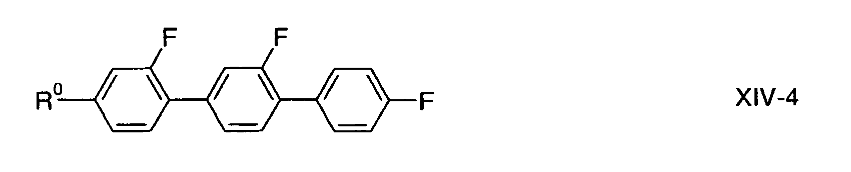 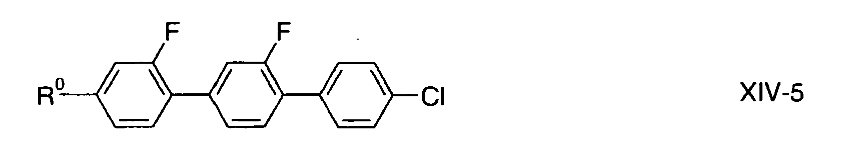 worin R⁰ jeweils unabhängig voneinander wie für die Formel XIV definiert ist.
- Der Anteil der Verbindungen der Formeln XIV und XV ist vorzugsweise 0-25 Gew.%, insbesondere 2-20 Gew.% und ganz besonders 5-15 Gew.%;

Vorzugsweise handelt es sich bei Verbindungen der Formel XV um eine Verbindung der Formel XV-1: 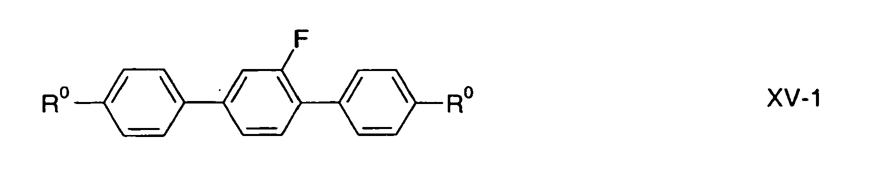 worin R⁰ wie für die Formel XV definiert ist.
- Das Medium enthält weitere Verbindungen, vorzugsweise ausgewählt aus der folgenden Gruppe bestehend aus den allgemeinen Formeln XVI bis XVIII: 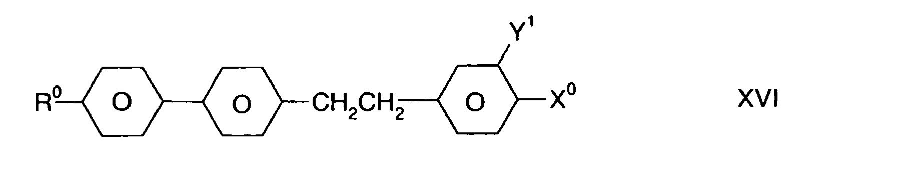 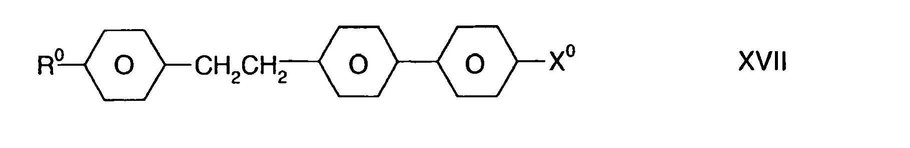 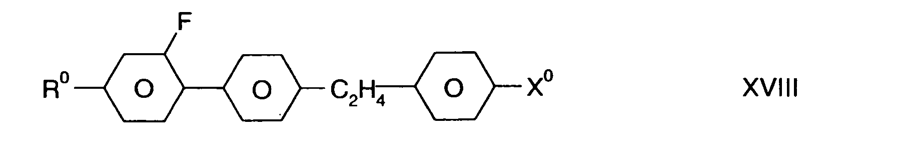 worin
   - R⁰: n-Alkyl, Oxaalkyl, Fluoralkyl oder Alkenyl mit jeweils bis zu 9 C-Atomen,
   - Y¹: H oder F, und
   - X⁰: F, Cl, halogeniertes Alkyl, halogeniertes Alkenyl, halogeniertes Alkenyloxy oder halogeniertes Alkoxy mit bis zu 6 C-Atomen
   bedeutet;
   die 1,4-Phenylenringe können zusätzlich durch CN, Chlor oder Fluor substituiert sein. Vorzugsweise sind die 1,4-Phenylenringe ein- oder mehrfach durch Fluoratome substituiert.
- Das Medium enthält zusätzlich ein, zwei, drei oder mehr, vorzugsweise zwei oder drei Verbindungen der Formeln 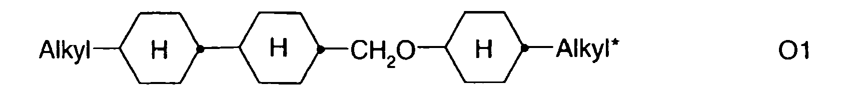 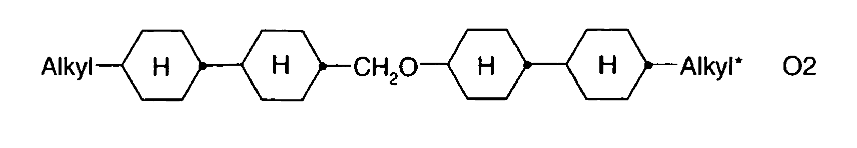 worin "Alkyl" und "Alkyl*" die nachfolgend angegebene Bedeutung haben. Der Anteil der Verbindungen der Formeln O1 und/oder 02 in den erfindungsgemäßen Mischungen beträgt vorzugsweise 0-15 Gew.%, insbesondere 1-12 Gew.% und ganz besonders bevorzugt 3-10 Gew.%.
- Das Medium enthält vorzugsweise 5-35 Gew.% der Verbindung IVa.
- Das Medium enthält vorzugsweise ein, zwei oder drei Verbindungen der Formel IVa, worin X^{o} F oder OCF₃ bedeutet.
- Das Medium enthält vorzugsweise eine oder mehrere Verbindungen der Formeln IIa bis IIg, 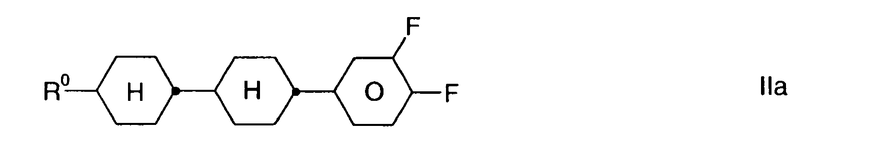 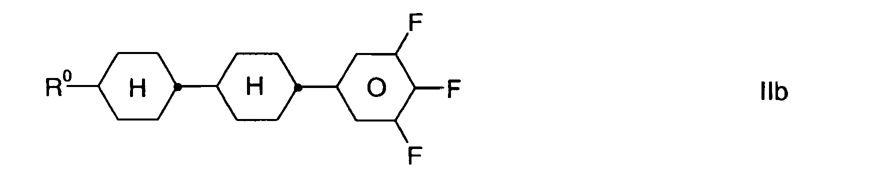 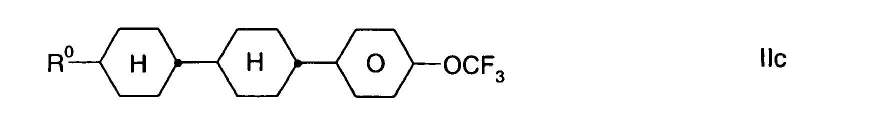 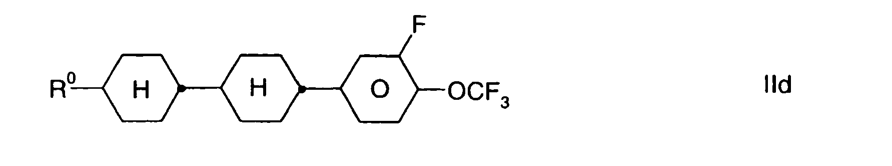 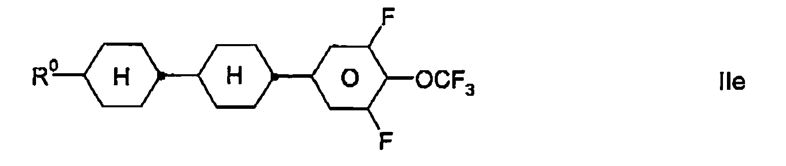 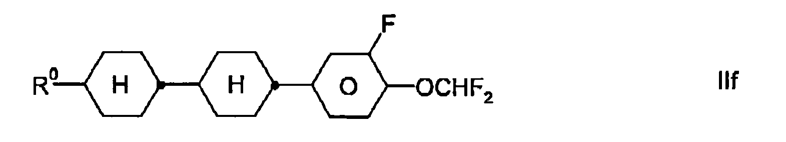 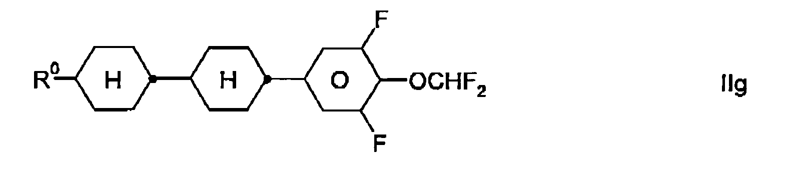
worin
- R⁰: n-Alkyl, Oxaalkyl, Fluoralkyl oder Alkenyl mit jeweils bis zu 9 C-Atomen
bedeutet.

In den Verbindungen der Formeln IIa-IIg bedeutet R⁰ vorzugsweise Methyl, Ethyl, n-Propyl, n-Butyl und n-Pentyl.
- Der Anteil an Verbindungen der Formeln K (K-1 bis K-12) beträgt bevorzugt 5 bis 50 Gew-%, besonders bevorzugt 10 bis 40 Gew.%.
- Der Anteil der Verbindungen der Formel IVb und/oder IVc, worin X^{o} Fluor und R⁰ CH₃, C₂H₅, n-C₃H₇, n-C₄H₉ oder n-C₅H₁₁ bedeutet, beträgt im Gesamtgemisch 2 bis 20 Gew.%, insbesondere 2 bis 15 Gew.%.
- Das Medium enthält vorzugsweise Verbindungen der Formeln II bis VI, worin R⁰ Methyl bedeutet. Besonders bevorzugt enthält das erfindungsgemäße Medium Verbindungen der Formeln 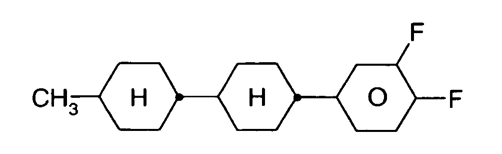 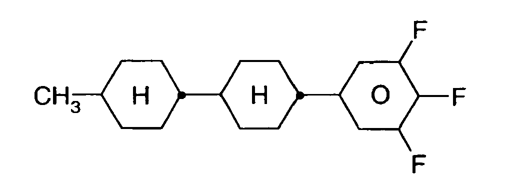 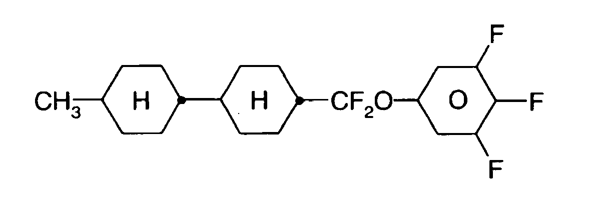 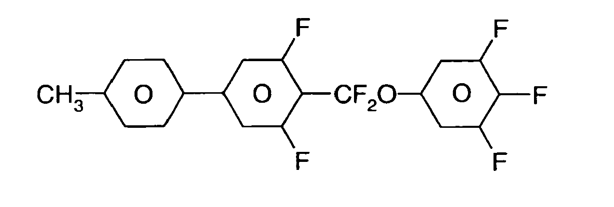
- Das Medium enthält vorzugsweise ein, zwei oder mehr, vorzugsweise ein oder zwei, Dioxan-Verbindungen der Formeln, 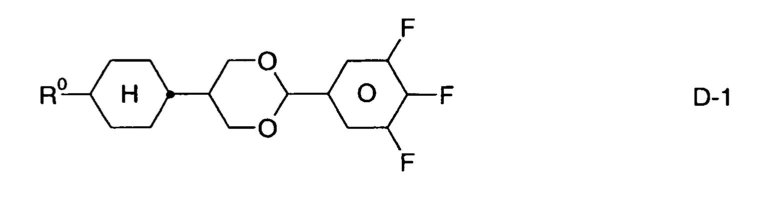 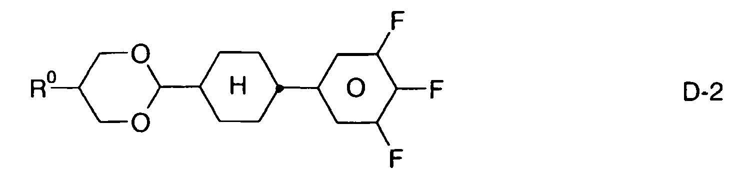
worin
- R⁰: n-Alkyl, Oxaalkyl, Fluoralkyl oder Alkenyl mit jeweils bis zu 9 C-Atomen
bedeutet.

Der Anteil der Dioxan-Verbindungen D-1 und/oder D-2 in den erfindungsgemäßen Mischungen beträgt vorzugsweise 0-25 Gew.%, insbesondere 0-20 Gew.% und ganz besonders bevorzugt 0-15 Gew.%.
- Das Medium enthält vorzugsweise ein, zwei oder mehr, vorzugsweise ein oder zwei Pyranverbindungen der Formeln P-1 bis P-4, 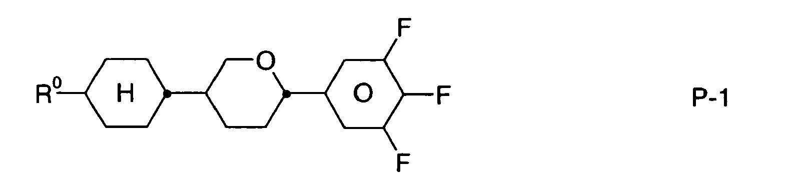 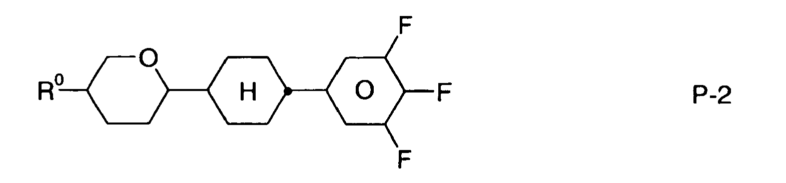 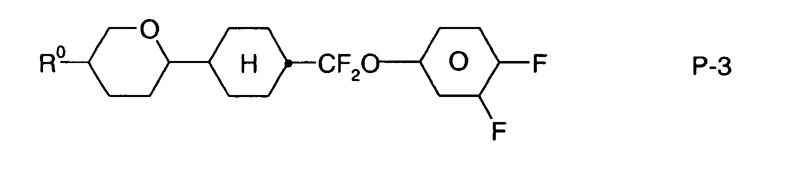 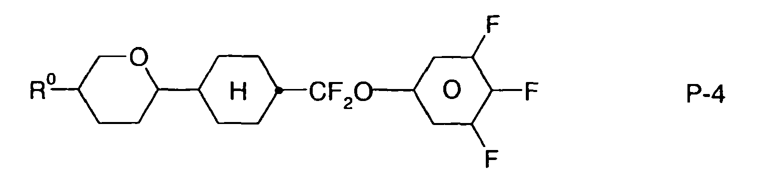 worin
   - R⁰: n-Alkyl, Oxaalkyl, Fluoralkyl oder Alkenyl mit jeweils bis zu 9 C-Atomen
   bedeutet.
- Das Medium enthält zusätzlich ein, zwei oder mehr Zweikern-Verbindungen der Formeln Z-1 bis Z-9 (allgemein Z), 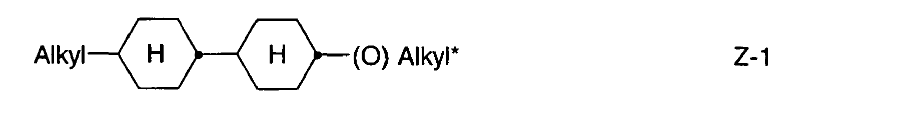 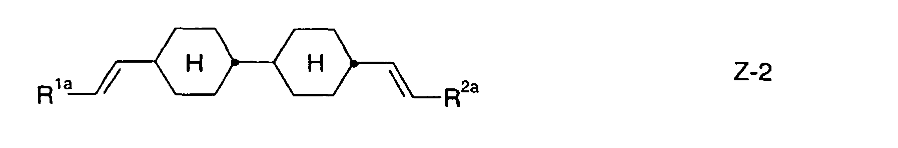 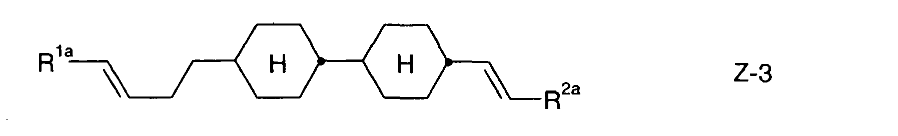 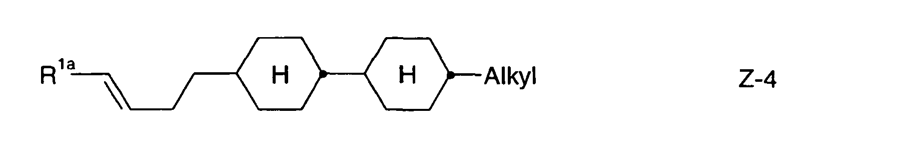 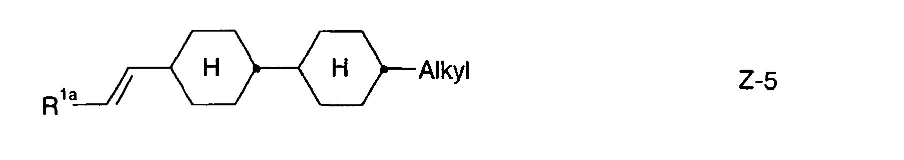 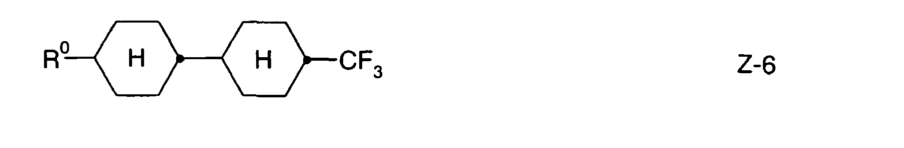 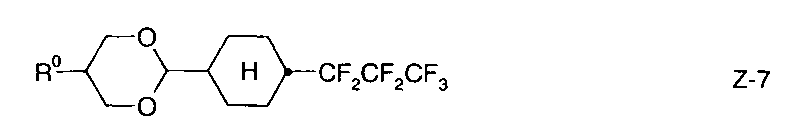 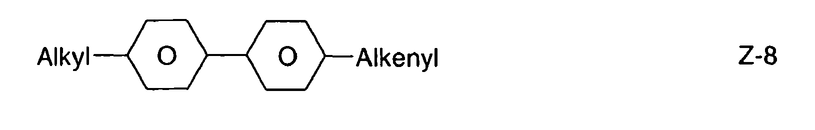 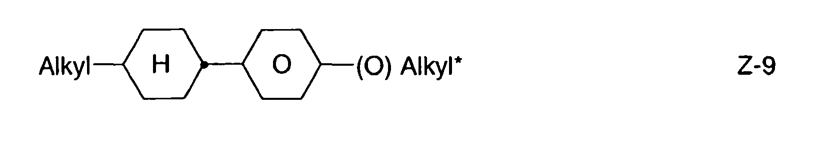
worin
- R^{1a} und R^{2a}: jeweils unabhängig voneinander H, CH₃, C₂H₅ oder n-C₃H₇ bedeuten, und
- R⁰: n-Alkyl, Oxaalkyl, Fluoralkyl oder Alkenyl mit jeweils bis zu 9 C-Atomen
bedeutet.

Alkyl, Alkyl* und Alkenyl besitzen die nachfolgend angegebenen Bedeutungen.

Von den genannten Zweikern-Verbindungen sind besonders bevorzugt die Verbindungen Z-2, Z-5, Z-4 und Z-6, ganz besoders die Verbindungen der Formel Z-5 mit Alkyl gleich Propyl und R^{1a} gleich H oder Methyl, insbesondere mit R^{1a} gleich H.
- Der Anteil an Verbindungen der Formeln Z-1 bis Z-9 beträgt insgesamt 5 bis 70 Gew.%, bevorzugt 15 bis 50 Gew.%. Der Anteil an Verbindungen der Formel Z-5 für sich beträgt vorzugsweise 10 bis 60 Gew.%, bevorzugt 15 bis 50 Gew.%.
- Das Medium besteht im wesentlichen aus Verbindungen ausgewählt aus der Gruppe bestehend aus den allgemeinen Formeln I bis VI, K-1 bis K-121 und aus Z-1 bis Z-9.

Das Medium enthält zusätzlich eine oder mehrere UV-stabilisierende Verbindungen, insbesondere eine Quaterphenylverbindung. Besonders bevorzugt sind einfach oder mehrfach fluorierte Quaterphenylverbindungen der Formel 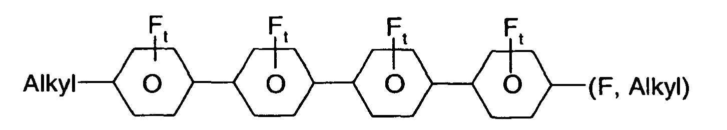 wobei t jeweils unabhängig 0, 1 oder 2 ist,
und ganz besonders der Formel 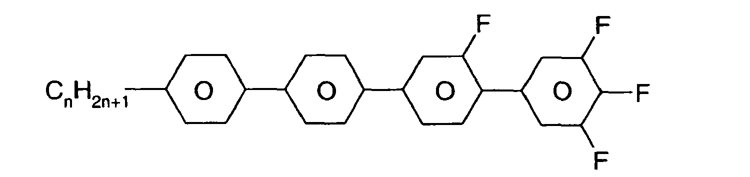 wobei n 1 bis 8 ist.
- Das Medium enthält zusätzlich ein, zwei oder mehr Verbindungen mit anellierten Ringen der Formeln AN1 bis AN11: 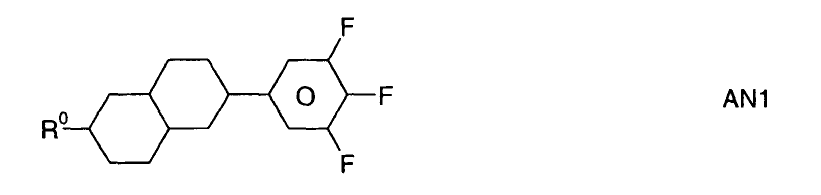 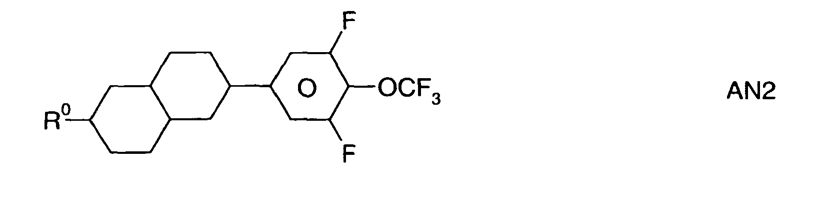 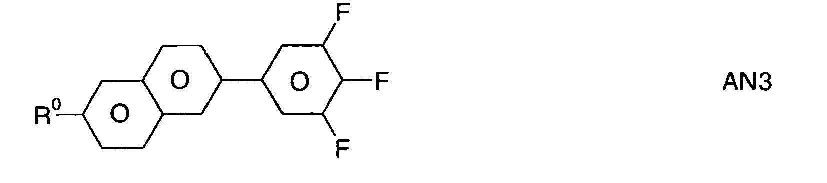 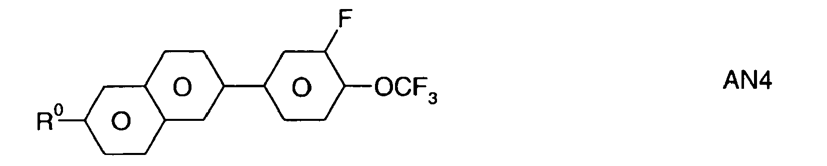 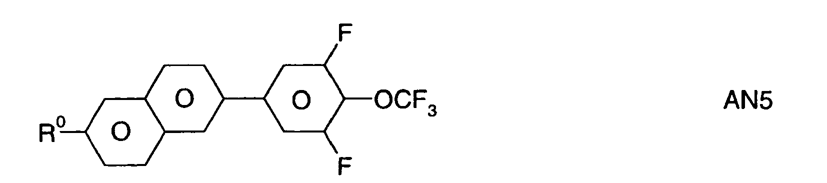 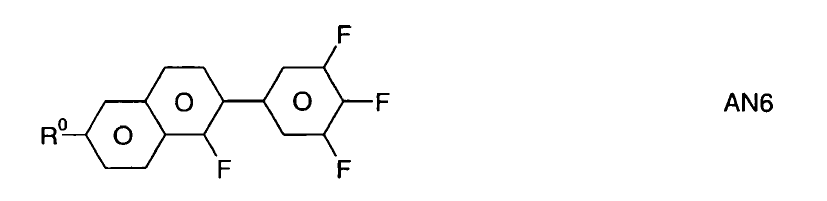 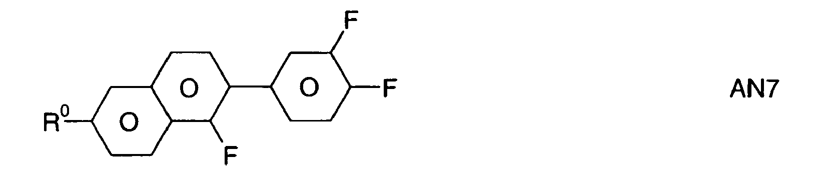 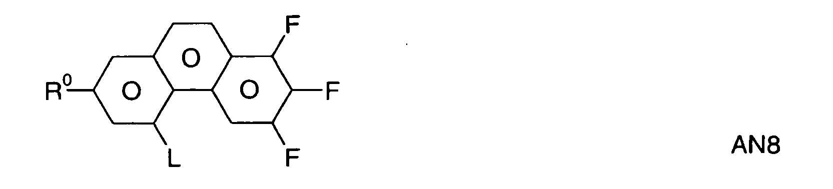 (L = H oder F) 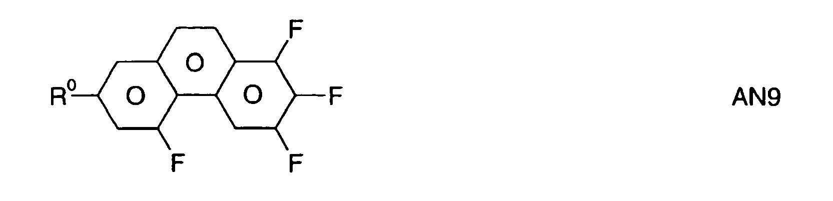 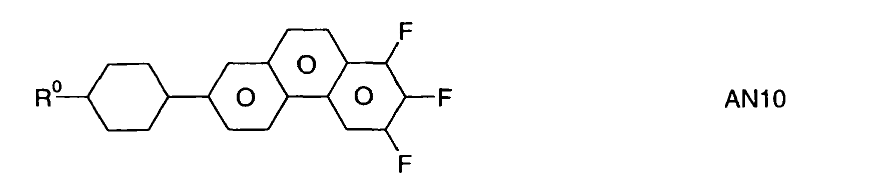 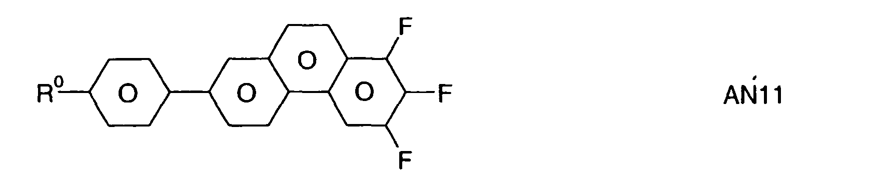 worin R⁰ die oben angegebenen Bedeutungen hat;
- Die erfindungsgemäßen Mischungen zeichnen sich insbesondere dadurch aus, dass sie Klärpunkte von ≥ 70 °C und Schwellenspannungen von < 2,0 V aufweisen.
- Die erfindungsgemäßen Mischungen zeichnen sich insbesondere dadurch aus, dass sie eine dielektrische Anisotropie von Δε > 3 und bevorzugt von Δε > 5 aufweisen.

Es wurde gefunden, dass bereits ein relativ geringer Anteil an Verbindungen der Formeln I im Gemisch mit üblichen Flüssigkristallmaterialien, insbesondere jedoch mit einer oder mehreren Verbindungen der Formeln K, Z, II, III, IV, V und/oder VI zu einer beträchtlichen Erniedrigung der Rotationsviskositäten und der Schaltzeiten führt, wobei gleichzeitig breite nematische Phasen mit tiefen Übergangstemperaturen smektisch-nematisch beobachtet werden, wodurch die Lagerstabilität verbessert wird.

Der Ausdruck "Alkyl" bzw. "Alkyl*" umfasst geradkettige und verzweigte Alkylgruppen mit 1-7 Kohlenstoffatomen, insbesondere die geradkettigen Gruppen Methyl, Ethyl, Propyl, Butyl, Pentyl, Hexyl und Heptyl. Gruppen mit 1-5 Kohlenstoffatomen sind im allgemeinen bevorzugt.

Der Ausdruck "Alkenyl" umfasst geradkettige und verzweigte Alkenylgruppen mit 2-7 Kohlenstoffatomen, insbesondere die geradkettigen Gruppen. Bevorzugte Alkenylgruppen sind C₂-C₇-1E-Alkenyl, C₄-C₇-3E-Alkenyl, C₅-C₇-4-Alkenyl, C₆-C₇-5-Alkenyl und C₇-6-Alkenyl, insbesondere C₂-C₇-1E-Alkenyl, C₄-C₇-3E-Alkenyl und C₅-C₇-4-Alkenyl. Beispiele besonders bevorzugter Alkenylgruppen sind Vinyl, 1 E-Propenyl, 1 E-Butenyl, 1 E-Pentenyl, 1 E-Hexenyl, 1 E-Heptenyl, 3-Butenyl, 3E-Pentenyl, 3E-Hexenyl, 3E-Heptenyl, 4-Pentenyl, 4Z-Hexenyl, 4E-Hexenyl, 4Z-Heptenyl, 5-Hexenyl, 6-Heptenyl und dergleichen. Gruppen mit bis zu 5 Kohlenstoffatomen sind im allgemeinen bevorzugt.

Der Ausdruck "Fluoralkyl" umfasst vorzugsweise geradkettige Gruppen mit endständigem Fluor, d.h. Fluormethyl, 2-Fluorethyl, 3-Fluorpropyl, 4-Fluorbutyl, 5-Fluorpentyl, 6-Fluorhexyl und 7-Fluorheptyl. Andere Positionen des Fluors sind jedoch nicht ausgeschlossen.

Der Ausdruck "Oxaalkyl" bzw. "Alkoxy" umfasst vorzugsweise geradkettige Reste der Formel CₙH₂ₙ₊₁-O-(CH₂)ₘ, worin n und m jeweils unabhängig voneinander 1 bis 6 bedeuten. m kann auch 0 bedeuten. Vorzugsweise ist n = 1 und m 1 -6 oder m = 0 und n = 1-3.

Durch geeignete Wahl der Bedeutungen von R⁰ und X⁰ können die Ansprechzeiten, die Schwellenspannung, die Steilheit der Transmissionskennlinien etc. in gewünschter Weise modifiziert werden. Beispielsweise führen 1 E-Alkenylreste, 3E-Alkenylreste, 2E-Alkenyloxyreste und dergleichen in der Regel zu kürzeren Ansprechzeiten, verbesserten nematischen Tendenzen und einem höheren Verhältnis der elastischen Konstanten k₃₃ (bend) und k₁₁ (splay) im Vergleich zu Alkyl- bzw. Alkoxyresten. 4-Alkenylreste, 3-Alkenylreste und dergleichen ergeben im allgemeinen tiefere Schwellenspannungen und kleinere Werte von k₃₃/k₁₁ im Vergleich zu Alkyl- und Alkoxyresten.

Eine -CH₂CH₂-Gruppe führt im allgemeinen zu höheren Werten von k₃₃/k₁₁ im Vergleich zu einer einfachen Kovalenzbindung. Höhere Werte von k₃₃/k₁₁ ermöglichen z.B. flachere Transmissionskennlinien in TN-Zellen mit 90° Verdrillung (zur Erzielung von Grautönen) und steilere Transmissionskennlinien in STN-, SBE- und OMI-Zellen (höhere Multiplexierbarkeit) und umgekehrt.

Das optimale Mengenverhältnis der Verbindungen der Formeln I und K + Z + II + III + IV + V + VI hängt weitgehend von den gewünschten Eigenschaften, von der Wahl der Komponenten der Formeln I, II, III, IV, V und/oder VI und der Wahl weiterer gegebenenfalls vorhandener Komponenten ab.

Geeignete Mengenverhältnisse innerhalb des oben angegebenen Bereichs können von Fall zu Fall leicht ermittelt werden.

Die Gesamtmenge an Verbindungen der Formeln I und der angegebenen Cokomponenten in den erfindungsgemäßen Gemischen ist nicht kritisch. Die Gemische können daher eine oder mehrere weitere Komponenten enthalten zwecks Optimierung verschiedener Eigenschaften. Der beobachtete Effekt auf die Ansprechzeiten und die Schwellenspannung ist jedoch in der Regel umso größer je höher die Gesamtkonzentration an Verbindungen der Formeln I und der angegebenen Cokomponenten sind.

In einer besonders bevorzugten Ausführungsform enthalten die erfindungsgemäßen Medien Verbindungen der Formel II bis VI (vorzugsweise II, III und/oder IV, insbesondere IVa), worin X⁰ F, OCF₃, OCHF₂, OCH=CF₂, OCF=CF₂ oder OCF₂-CF₂H bedeutet. Eine günstige synergistische Wirkung mit den Verbindungen der Formeln I führt zu besonders vorteilhaften Eigenschaften. Insbesondere Mischungen enthaltend Verbindungen der Formel I und der Formel IVa zeichnen sich durch ihre niedrige Schwellenspannung aus.

Die einzelnen Verbindungen, die in den erfindungsgemäßen Medien verwendet werden können, sind entweder bekannt, oder sie können analog zu den bekannten Verbindungen hergestellt werden.

Der Aufbau der erfindungsgemäßen MFK-Anzeige aus Polarisatoren, Elektrodengrundplatten und Elektroden mit Oberflächenbehandlung entspricht der für derartige Anzeigen üblichen Bauweise. Dabei ist der Begriff der üblichen Bauweise hier weit gefasst und umfasst auch alle Abwandlungen und Modifikationen der MFK-Anzeige, insbesondere auch Matrix-Anzeigeelemente auf Basis poly-Si TFT oder MIM.

Ein wesentlicher Unterschied der erfindungsgemäßen Anzeigen zu den bisher üblichen auf der Basis der verdrillten nematischen Zelle besteht jedoch in der Wahl der Flüssigkristallparameter der Flüssigkristallschicht.

Die Herstellung der erfindungsgemäß verwendbaren Flüssigkristallmischungen erfolgt in an sich üblicher Weise. In der Regel wird die gewünschte Menge der in geringerer Menge verwendeten Komponenten in der den Hauptbestandteil ausmachenden Komponenten gelöst, zweckmäßig bei erhöhter Temperatur. Es ist auch möglich Lösungen der Komponenten in einem organischen Lösungsmittel, z.B. in Aceton, Chloroform oder Methanol, zu mischen und das Lösungsmittel nach Durchmischung wieder zu entfernen, beispielsweise durch Destillation.

Die Dielektrika können auch weitere, dem Fachmann bekannte und in der Literatur beschriebene Zusätze, wie z. B. UV-Stabilisatoren wie Tinuvin^{®} der Fa. Ciba, Antioxidantien, Radikalfänger, etc. enthalten. Beispielsweise können 0-15 % pleochroitische Farbstoffe oder chirale Dotierstoffe zugesetzt werden. Geeignete Stabilisatoren und Dotierstoffe werden nachfolgend in den Tabellen C und D genannt.

Die Schwellenspannung V₁₀ bezeichnet die Spannung für 10 % Transmission (Blickrichtung senkrecht zur Plattenoberfläche). tₒₙ bezeichnet die Einschaltzeit und t_{off} die Ausschaltzeit bei einer Betriebsspannung entsprechend dem 2,0fachen Wert von V₁₀. Δn bezeichnet die optische Anisotropie. Δε bezeichnet die dielektrische Anisotropie (Δε = Δε_{∥} - ε_{⊥}, wobei ε_{∥} die Dielektrizitätskonstante parallel zu den Moleküllängsachsen und ε_{⊥} die Dielektrizitätskonstante senkrecht dazu bedeutet). Die elektro-optischen Daten werden in einer TN-Zelle im 1. Minimum (d.h. bei einem d · Δn-Wert von 0,5 µm) bei 20 °C gemessen, sofern nicht ausdrücklich etwas anderes angegeben wird. Die optischen Daten werden bei 20 °C gemessen, sofern nicht ausdrücklich etwas anderes angegeben wird.

In der vorliegenden Anmeldung und in den folgenden Beispielen sind die Strukturen der Flüssigkristallverbindungen durch Akronyme angegeben, wobei die Transformation in chemische Formeln gemäß folgender Tabellen A und B erfolgt. Alle Reste CₙH₂ₙ₊₁ und CₘH₂ₘ₊₁ sind geradkettige Alkylreste mit n bzw. m C-Atomen; n und m sind ganze Zahlen und bedeuten vorzugsweise 0, 1, 2, 3, 4, 5, 6, 7, 8, 9, 10, 11 oder 12. Die Codierung gemäß Tabelle B versteht sich von selbst. In Tabelle A ist nur das Akronym für den Grundkörper angegeben. Im Einzelfall folgt getrennt vom Akronym für den Grundkörper mit einem Strich ein Code für die Substituenten R^{1*}, R^{2*}, L^{1*} und L^{2*}:

| Code für R^{1*}, R^{2*}, L^{1*}, L^{2*}, L^{3*} | R^{1*} | R^{2*} | L^{1*} | L^{2*} |
|---|---|---|---|---|
| nm | CₙH₂ₙ₊₁ | CₘH₂ₘ₊₁ | H | H |
| n0m | OCₙH₂ₙ₊₁ | CₘH₂ₘ₊₁ | H | H |
| n0.m | CₙH₂ₙ₊₁ | OCₘH₂ₘ₊₁ | H | H |
| n | CₙH₂ₙ₊₁ | CN | H | H |
| nN.F | CₙH₂ₙ₊₁ | CN | F | H |
| nN.F.F | CₙH₂ₙ₊₁ | CN | F | F |
| nF | CₙH₂ₙ₊₁ | F | H | H |
| nCl | CₙH₂ₙ₊₁ | Cl | H | H |
| n0F | OCₙH₂ₙ₊₁ | F | H | H |
| nF.F | CₙH₂ₙ₊₁ | F | F | H |
| nF.F.F | CₙH₂ₙ₊₁ | F | F | F |
| nmF | CₙH₂ₙ₊₁ | CₘH₂ₘ₊₁ | F | H |
| n0CF₃ | CₙH₂ₙ₊₁ | OCF₃ | H | H |
| n0CF₃.F | CₙH₂ₙ₊₁ | OCF₃ | F | H |
| n0CF₃.F.F | CₙH₂ₙ₊₁ | OCF₃ | F | F |
| n-Vm | CₙH₂n+1 | -CH=CH-CₘH₂ₘ₊₁ | H | H |
| nV-Vm | CₙH₂ₙ₊₁-CH=CH- | -CH=CH-CₘH₂ₘ₊₁ | H | H |

Bevorzugte Mischungskomponenten finden sich in den Tabellen A und B.

**Tabelle A**

| |
|---|
| 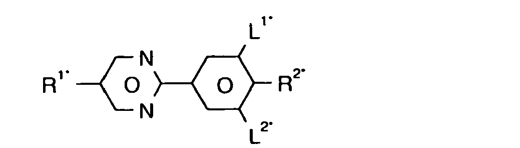 |
| **PYP** |
| 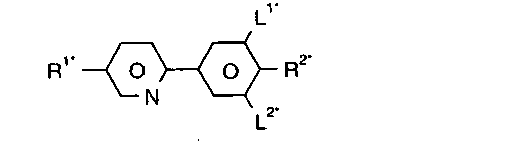 |
| **PYRP** |
| 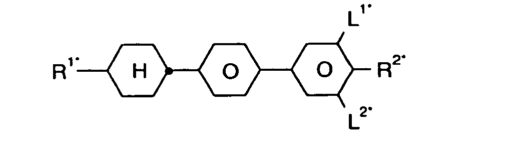 |
| **BCH** |
| 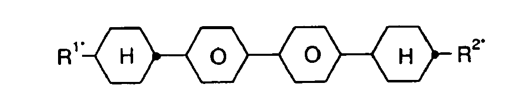 |
| **CBC** |
| 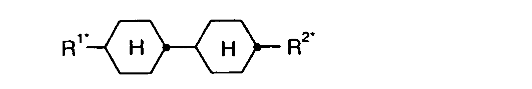 |
| **CCH** |
| 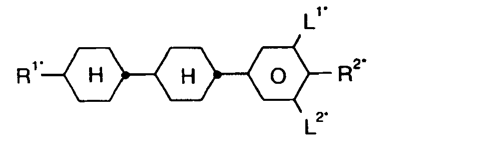 |
| **CCP** |
| 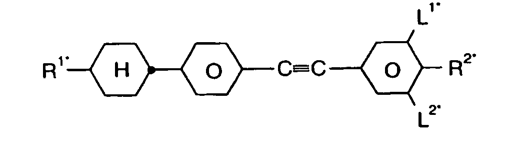 |
| **CPTP** |
| 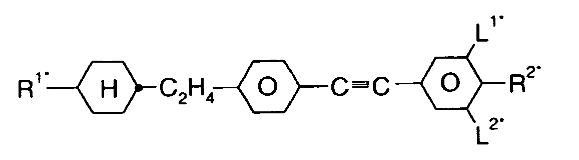 |
| **CEPTP** |
| 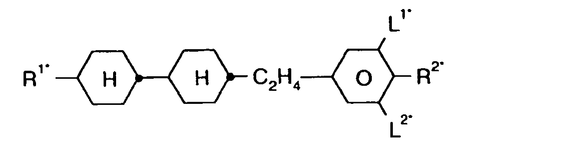 |
| **ECCP** |
| 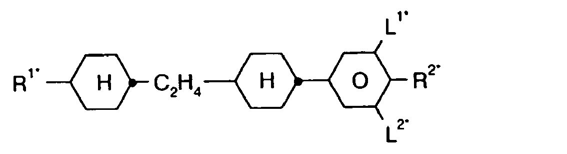 |
| **CECP** |
| 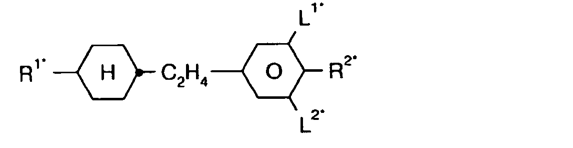 |
| **EPCH** |
| 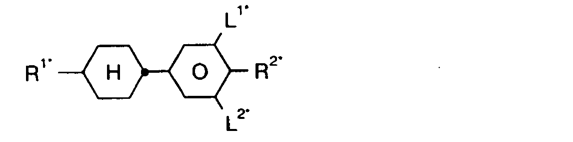 |
| **PCH** |
| 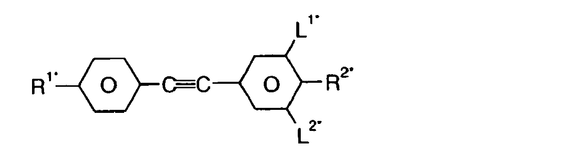 |
| **PTP** |
| 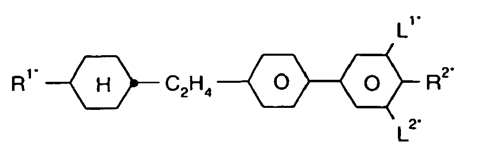 |
| **BECH** |
| 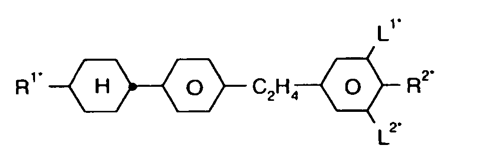 |
| **EBCH** |
| 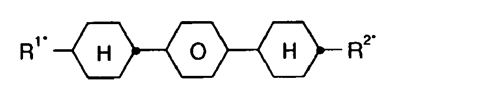 |
| **CPC** |
| 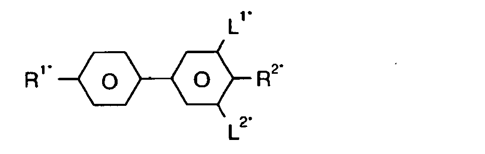 |
| **B** |
| 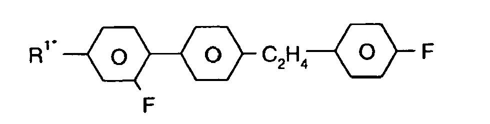 |
| **FET-nF** |
| 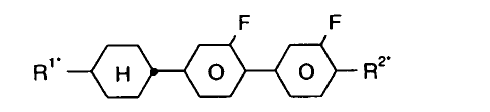 |
| **CGG** |
| 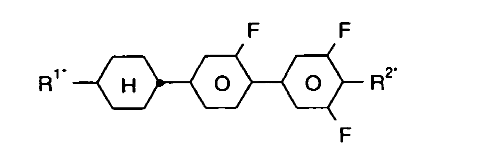 |
| **CGU** |
| 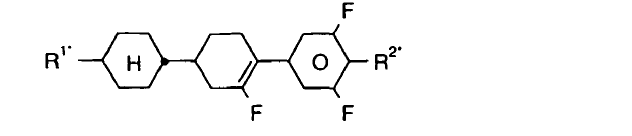 |
| **CFU** |

**Tabelle B**

| |
|---|
| 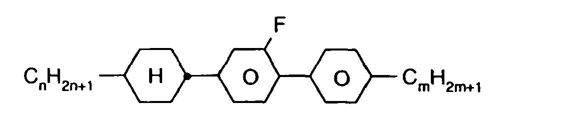 |
| **BCH-n.Fm** |
| 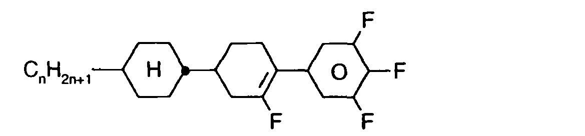 |
| **CFU-n-F** |
| 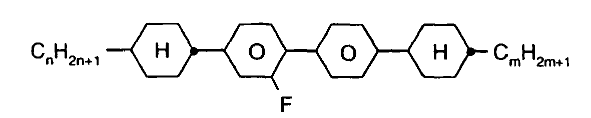 |
| **CBC-nmF** |
| 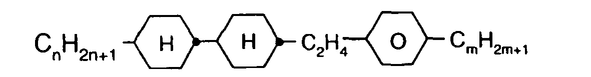 |
| **ECCP-nm** |
| 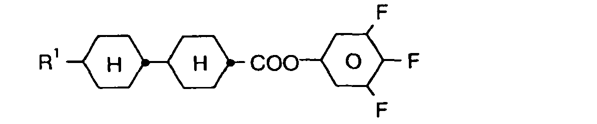 |
| **CCZU-n-F** |
| 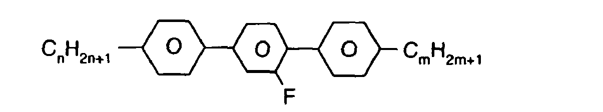 |
| **PGP-n-m** |
| 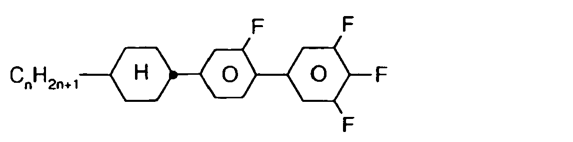 |
| **CGU-n-F** |
| 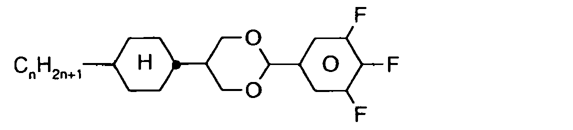 |
| **CDU-n-F** |
| 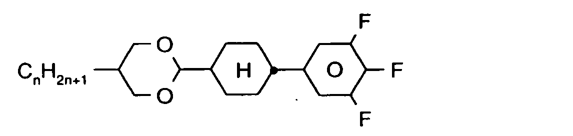 |
| **DCU-n-F** |
| 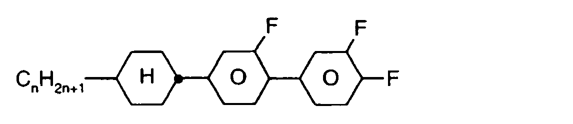 |
| **CGG-n-F** |
| 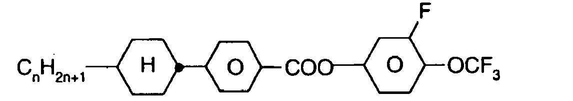 |
| **CPZG-n-OT** |
| 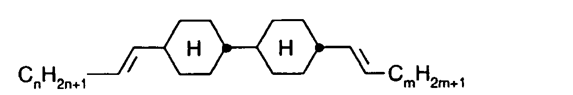 |
| **CC-nV-Vm** |
| 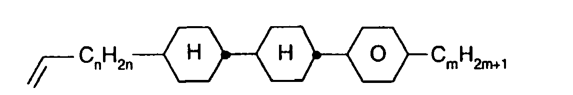 |
| **CCP-Vn-m** |
| 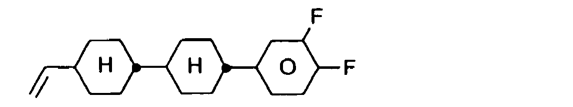 |
| **CCG-V-F** |
| 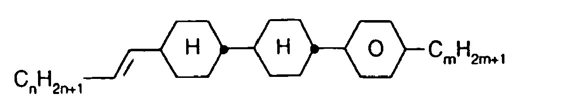 |
| **CCP-nV-m** |
| 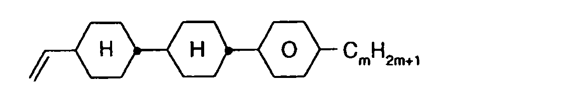 |
| **CCP-V-m** |
| 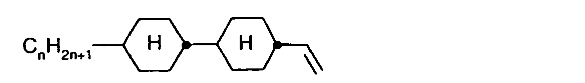 |
| **CC-n-V** |
| 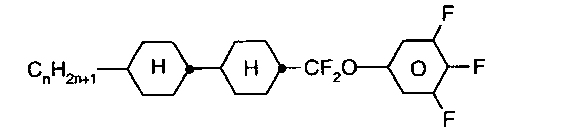 |
| **CCQU-n-F** |
| 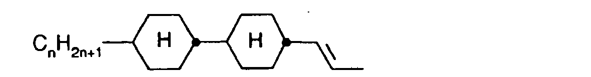 |
| **CC-n-V1** |
| 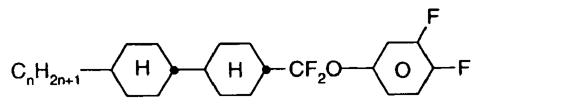 |
| **CCQG-n-F** |
| 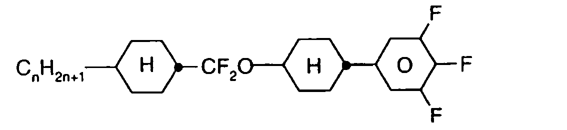 |
| **CQCU-n-F** |
| 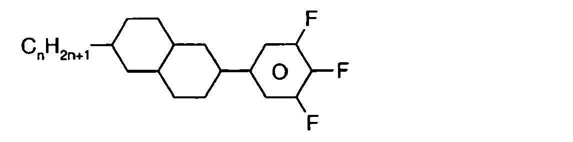 |
| **Dec-U-n-F** |
| 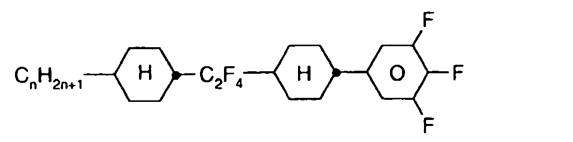 |
| **CWCU-n-F** |
| 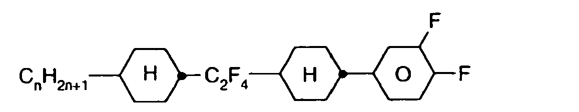 |
| **CWCG-n-F** |
| 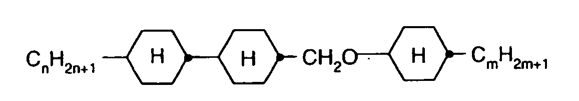 |
| CCOC-n-m |
| 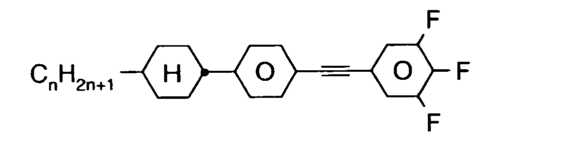 |
| **CPTU-n-F** |
| 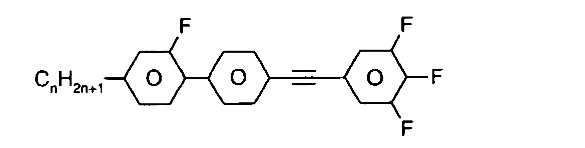 |
| **GPTU-n-F** |
| 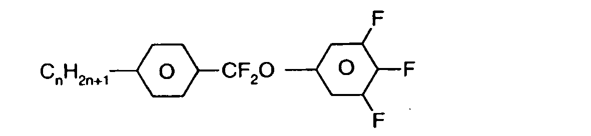 |
| **PQU-n-F** |
| 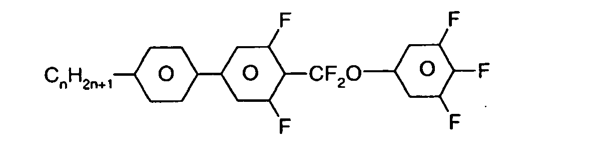 |
| **PUQU-n-F** |
| 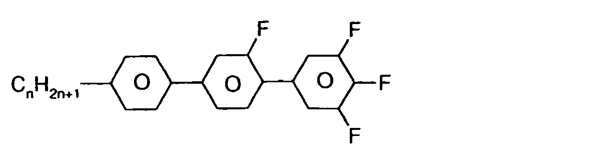 |
| **PGU-n-F** |
| 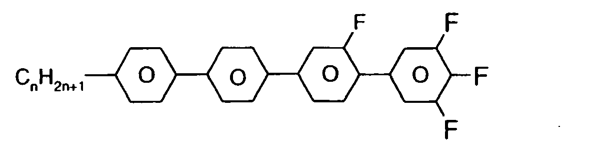 |
| **PPGU-n-F** |
| 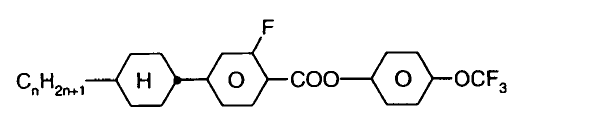 |
| **CGZP-n-OT** |
| 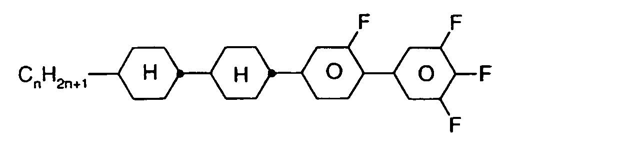 |
| **CCGU-n-F** |
| 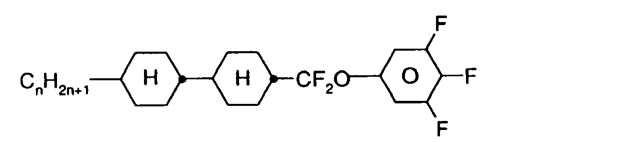 |
| **CCQU-n-F** |
| 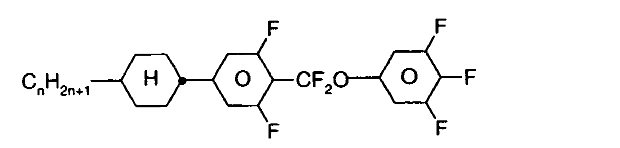 |
| **CUQU-n-F** |
| 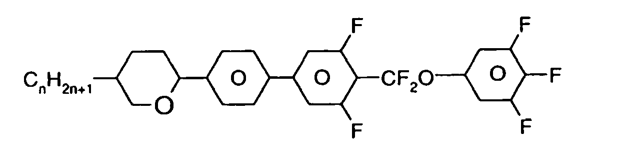 |
| **APUQU-n-F** |
| 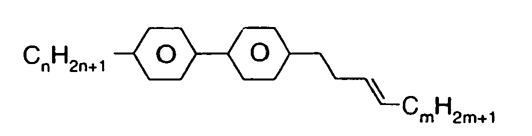 |
| **PP-n-2Vm** |

Besonders bevorzugt sind flüssigkristalline Mischungen, die neben den Verbindungen der Formeln I mindestens ein, zwei, drei oder vier Verbindungen aus der Tabelle B enthalten.

**Tabelle C**

| |
|---|
| In der Tabelle C werden mögliche Dotierstoffe angegeben, die in der Regel den erfindungsgemäßen Mischungen zugesetzt werden. Vorzugsweise enthalten die Mischungen 0-10 Gew.%, insbesondere 0,01-5 Gew.% und besonders bevorzugt 0,01-3 Gew.% an Dotierstoffen. |
| 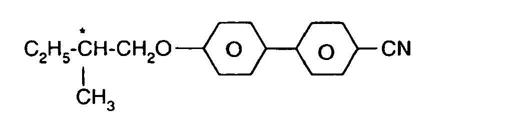 |
| **C 15** |
| 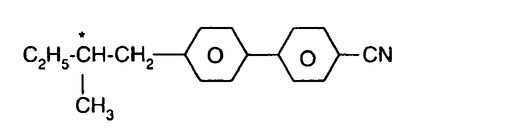 |
| **CB 15** |
| 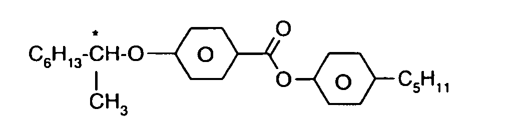 |
| **CM 21** |
| 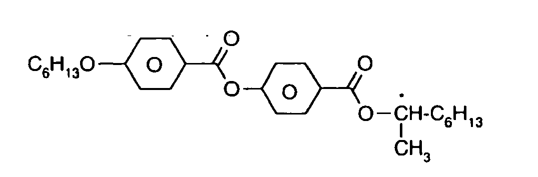 |
| **R/S-811** |
| 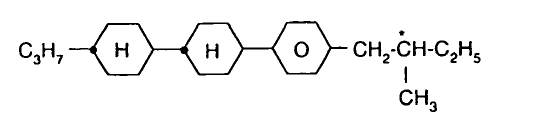 |
| **CM 44** |
| 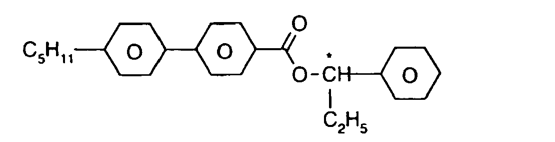 |
| **CM 45** |
| 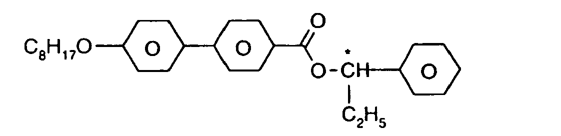 |
| **CM 47** |
| 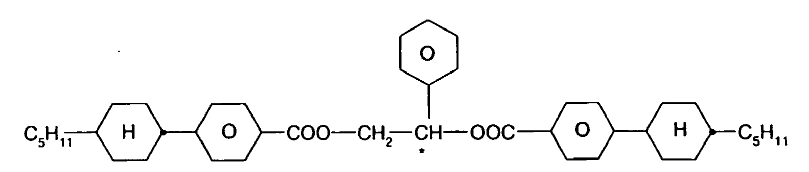 |
| **R/S-1011** |
| 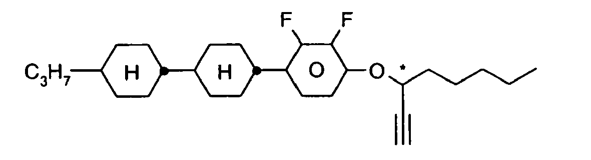 |
| **R/S-3011** |
| 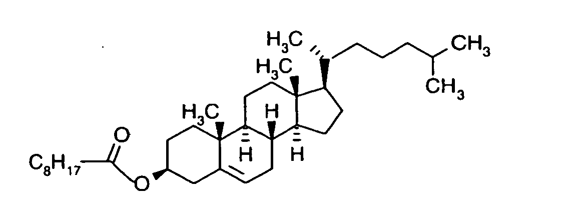 |
| **CN** |
| 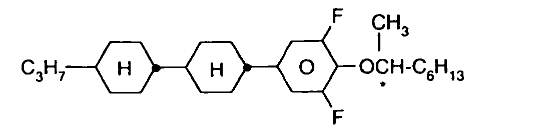 |
| **R/S-2011** |
| 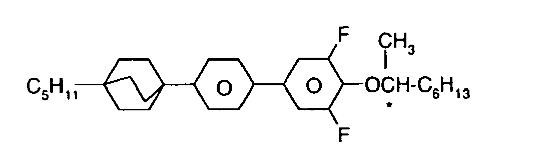 |
| **R/S-4011** |
| 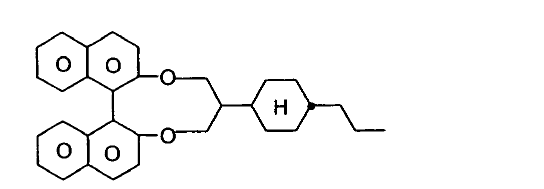 |
| **R/S-5011** |

**Tabelle D**

| | |
|---|---|
| Stabilisatoren, die beispielsweise den erfindungsgemäßen Mischungen zugesetzt werden können, werden nachfolgend genannt. | |
| 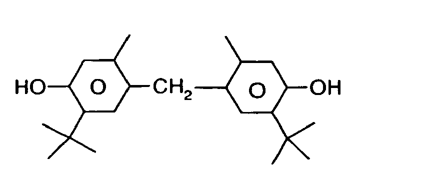 | 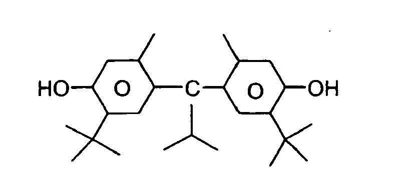 |
| 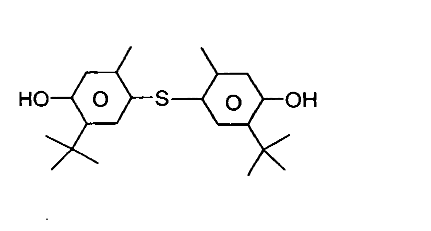 | 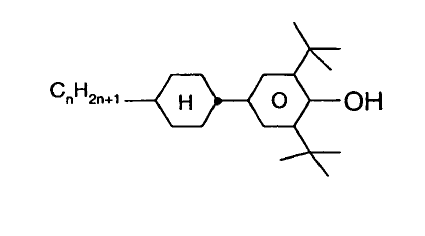 |
| 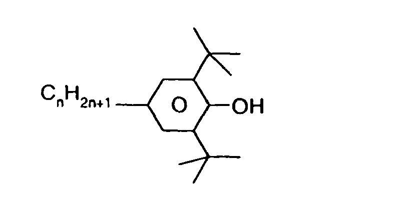 | 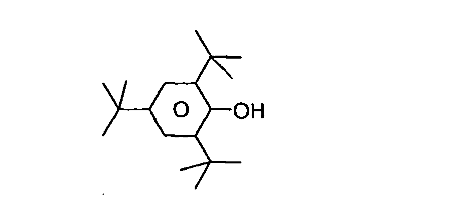 |
| 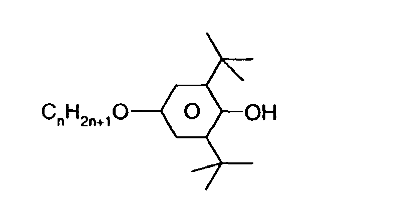 | 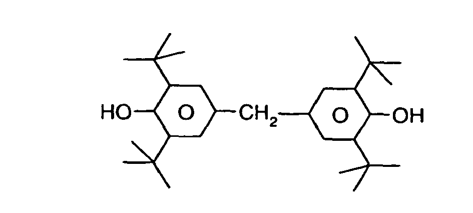 |
| 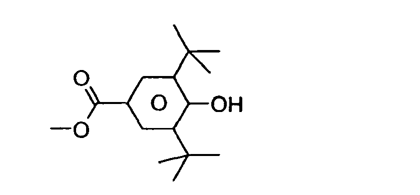 | 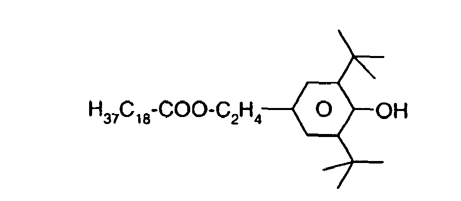 |
| 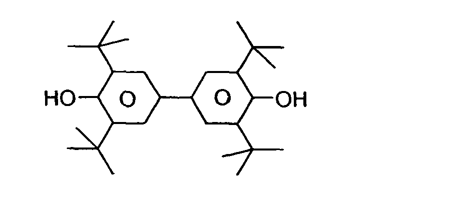 | |
| 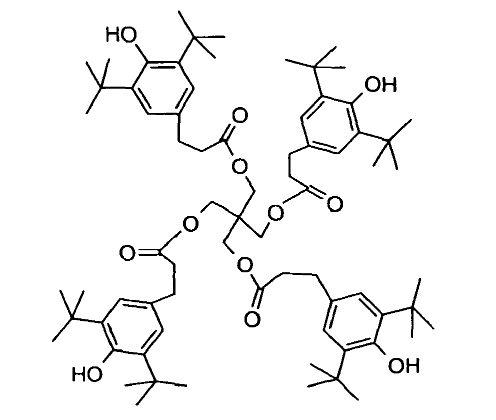 | |
| 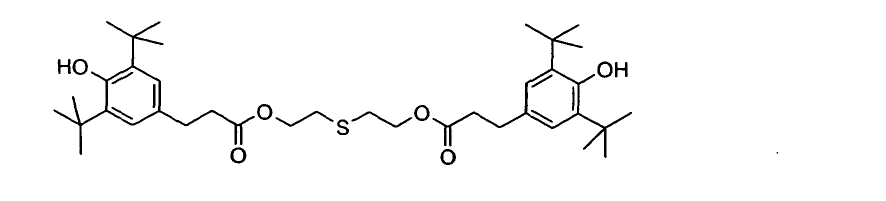 | |
| 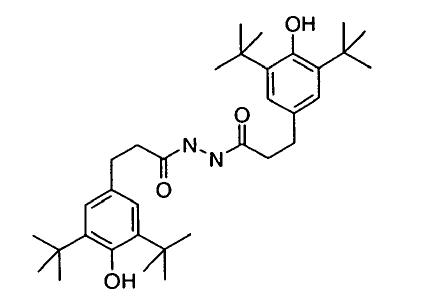 | |
| 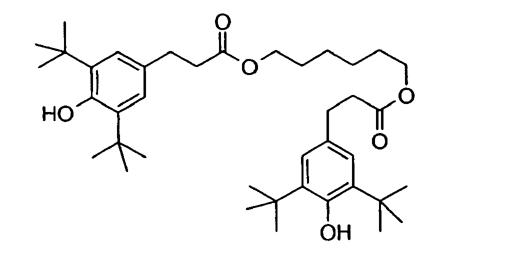 | |
| 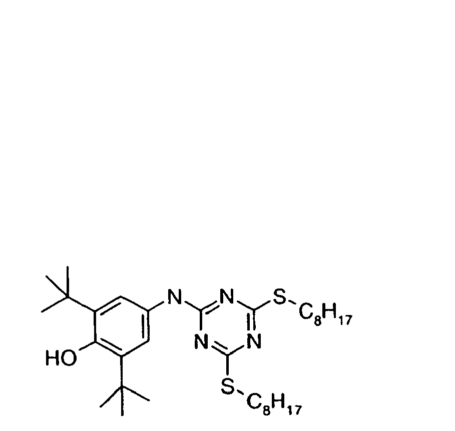 | 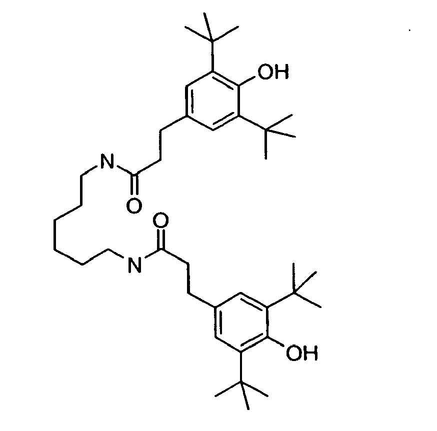 |
| 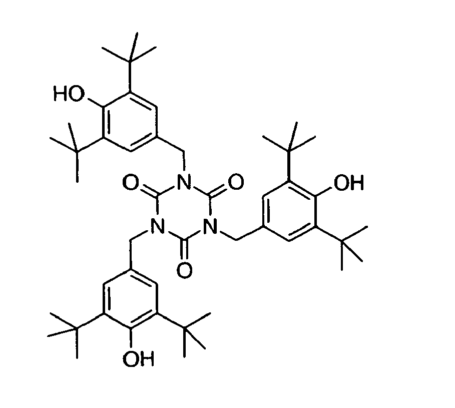 | 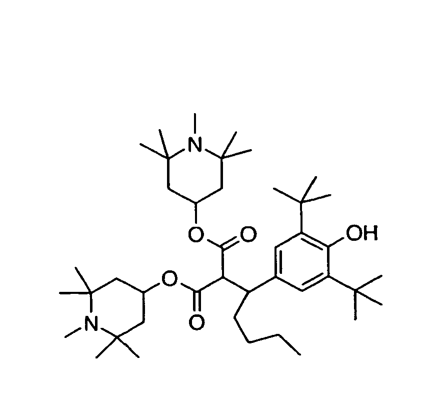 |
| 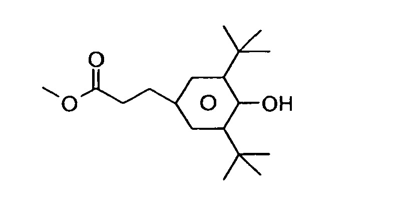 | 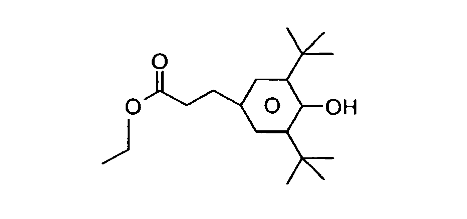 |
| 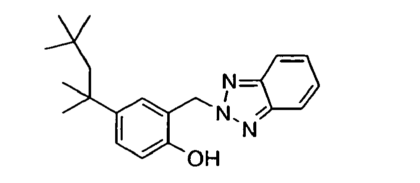 | 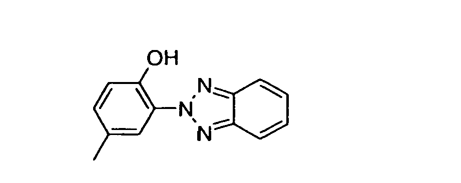 |
| 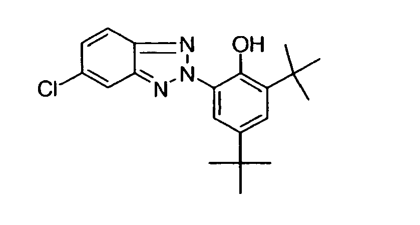 | 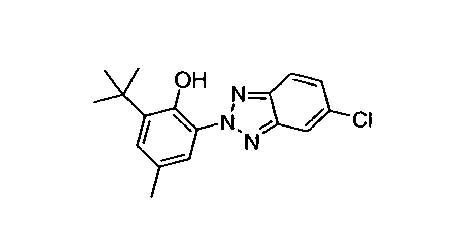 |
| 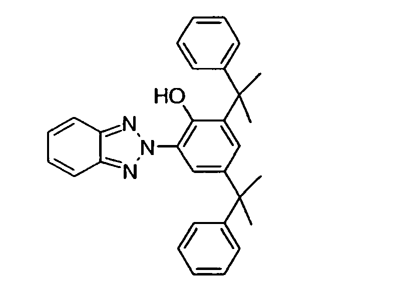 | 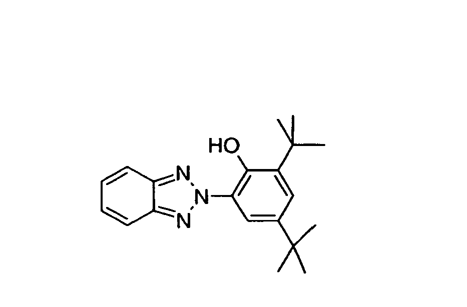 |
| 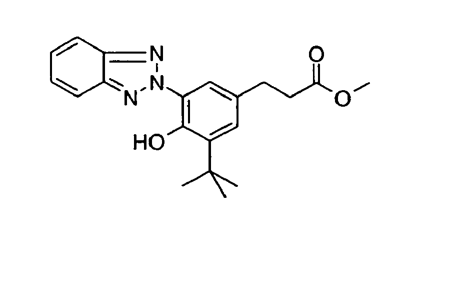 | 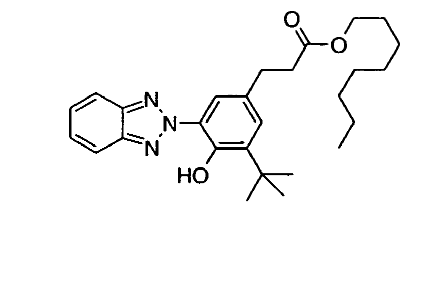 |
| 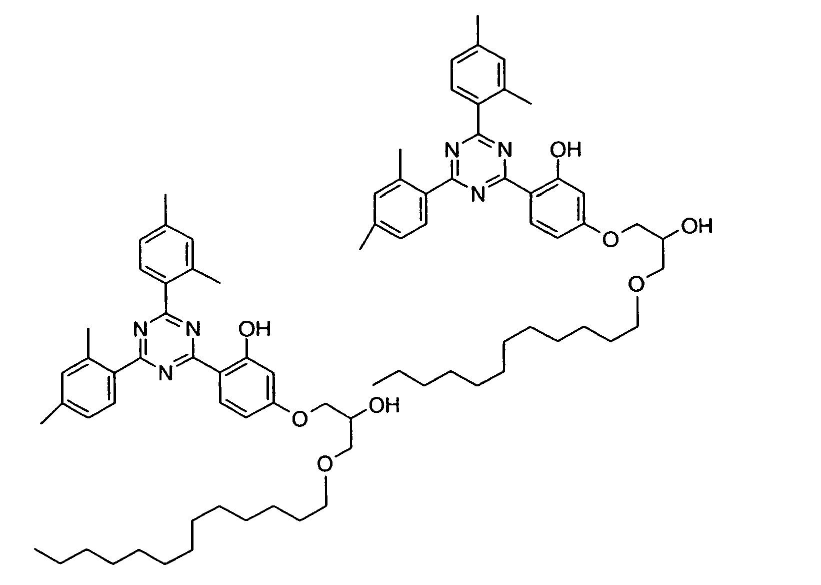 | |
| 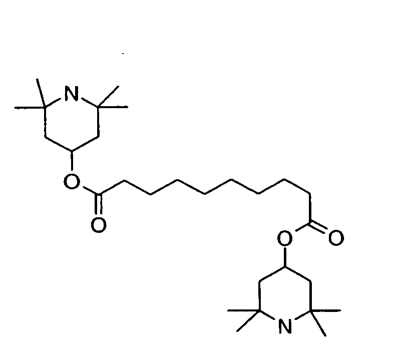 | 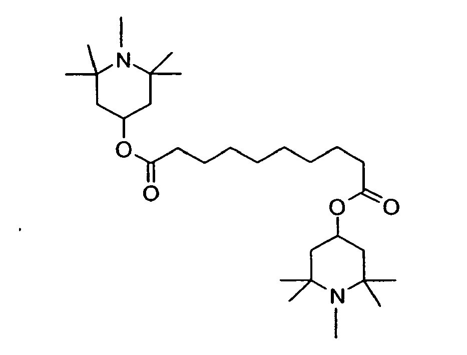 |
| 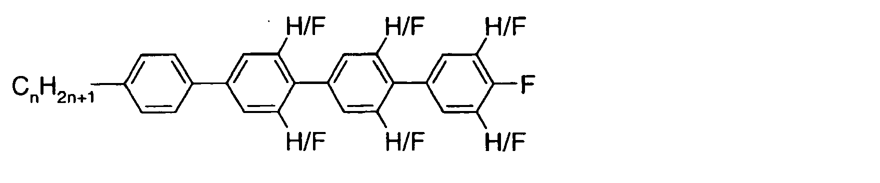 | |

Die folgenden Beispiele erläutern die Erfindung, ohne sie einschränken zu sollen. Der Fachmann wird in der Lage sein, den Beispielen besonders geeignete, nicht näher beschriebene Ausführungsformen zu entnehmen und sie an unterschiedliche Randbedingungen anzupassen.

Vor- und nachstehend bedeuten Prozentangaben Gewichtsprozent. Alle Temperaturen sind in Grad Celsius angegeben. Es bedeuten K = kristalliner Zustand, N = nematische Phase, Sm = smektische Phase und I = isotrope Phase. Die Angaben zwischen diesen Symbolen stellen die Übergangstemperaturen für die Reinstoffe dar.

Die physikalischen Messmethoden an Mischungen sind in beschrieben in "Merck Liquid Crystals, Physical Properties of Liquid Crystals", Nov. 1997, Merck KGaA.

Die dielektrische Anisotrope Δε der einzelnen Substanzen wird bei 20 °C und 1 kHz bestimmt. Dazu werden 10 Gew.% der zu untersuchenden Substanzen in der dielektrisch positiven Mischung ZLI-4792 (Merck KGaA) gelöst gemessen und der Messwert auf eine Konzentration von 100 % extrapoliert. Die optische Anisotropie Δn wird bei 20 °C und einer Wellenlänge von 589,3 nm bestimmt. Sie wird ebenfalls durch Extrapolation der Werte bei 10 Gew.% bestimmt.

Es bedeuten:
- Klp.: Klärpunkt (Phasenübergangstemperatur nematisch-isotrop),
- Δn: optische Anisotropie (589 nm, 20°C),
- Δε: dielektrische Anisotropie (1 kHz, 20°C), ε_{∥} - ε_{⊥}
- ε_{∥}: Anteil der Dielektrizitätskonstante parallel zur Moleküllängsachse (1 kHz, 20 °C),
- ε_{⊥}: Anteil der Dielektrizitätskonstante senkrecht zur Moleküllängsachse (1 kHz, 20 °C)
- γ₁: Rotationsviskosität (20°C),
- tₛₜₒᵣₑ: Tieftemperatur-Lagerstabilität in Stunden (-20°C, -30°C, -40°C),
- V₁₀: Schwellenspannung = charakteristische Spannung bei einem relativen Kontrast von 10 %,
- V₉₀: Sättigungsspannung = charakteristische Spannung bei einem relativen Kontrast von 90 %,
- k₁: elastische Konstante Spreizdeformation (splay deformation, auch k₁₁)
- k₃: elastische Konstante Biegedeformation (bend deformation, auch k₃₃)
- k₃/k₁: Verhältnis von k₃ zu k₁,
- V₀: kapazitive bzw. Freederickzs-Schwellenspannung

### Synthesebeispiel 1.1

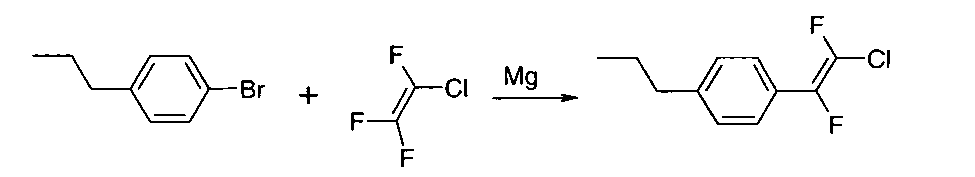

Durch langsame Zugabe von 141 g (70,9 mmol) 1-Brom-4-propylbenzol zu 20 g (823 mmol) Magnesium in 200 ml trockenem THF wird eine Grignard- Lösung hergestellt. Nach 1 h Rühren unter Rückfluss wird mit 1 I THF verdünnt und auf -35°C gekühlt. Am Trockeneiskühler werden 100 g (0,86 mol) Chlortrifluorethylen langsam eingeleitet und 1,5 h gerührt. Die Reaktionslösung wird auf RT erwärmt, 12 h gerührt und in eine Mischung aus Eis/2N HCl eingerührt. Die organische Phase wird abgetrennt. Die wässrige Phase wird mit MTB-Ether extrahiert. Die vereinigten organischen Phasen werden mit Wasser und NaCl-Lösung gewaschen, getrocknet und eingeengt. Das Produkt ist eine farblose Flüssigkeit.

### Synthesebeispiel 1.2

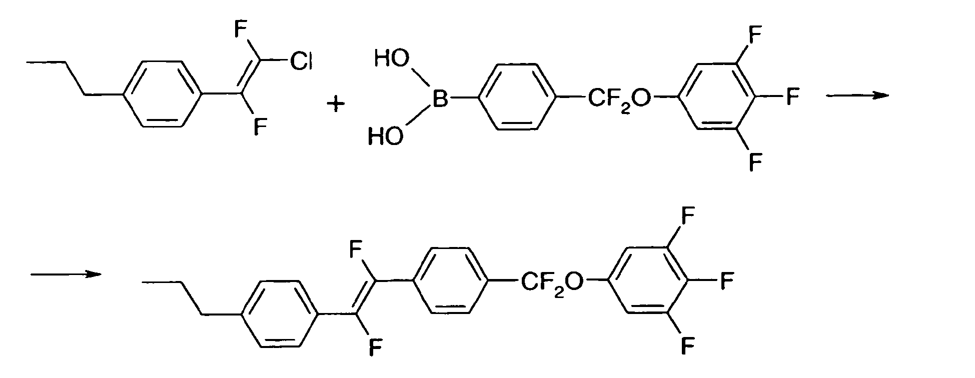

Zu 18,2 g (97 mmol) Natriumorthosilikat in 50 ml Wasser werden unter Stickstoff 2,02 g (30 mmol) Bis(tricyclohexylphosphin)palladium(II)chlorid und 0,154 ml (30 mmol) Hydraziniumhydroxid gegeben und 5 min gerührt. Dazu werden 32,5 g (142 mmol) des Produktes aus Beispiel 1.1 und 50 g (141 mmol) der Boronsäureverbindung gegeben und 12 h am Rückfluss gerührt. Anschließend wird die organische Phase abgetrennt, der Rest ausgeschüttelt und alle organischen Phasen vereinigt. Die Reinigung erfolgt durch Fraktionierung über 1 I Kieselgel mit Pentan. Die Produktfraktion wird aus kaltem Isopropanol kristallisiert. Farblose Kristalle (Schmp. 55°C, > 99 % GC/HPLC). K 55 N 75 I.

### Synthesebeispiel 2

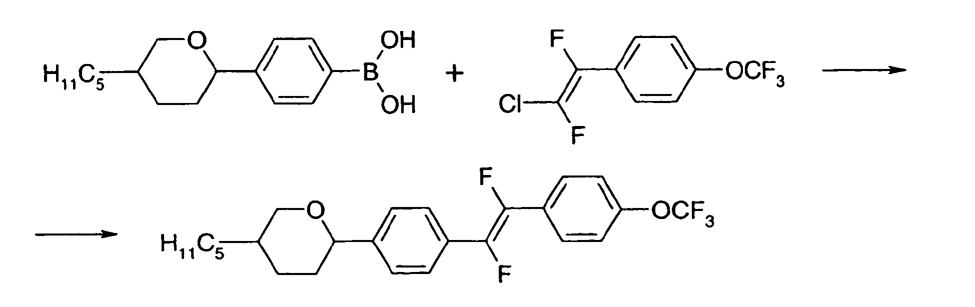

Zu 3,75 g (19,9 mmol) Natriumorthosilikat in 10 ml Wasser werden unter Stickstoff 0,40 g (0,72 mmol) Bis(tricyclohexylphosphin)palladium(II)chlorid und 0,35 ml (0,72 mmol) Hydraziniumchlorid gegeben und 5 min gerührt. Dazu werden 7,23 g (28 mmol) der Boronsäure und 7,50 g (27 mmol) der Chlordifluorethenverbindung gegeben und 12 h am Rückfluss gerührt. Nach der Aufarbeitung analog Beispiel 1.1 wird aus Toluol/Methanol kristallisiert (Schmp. 39°C; > 99 % GC/HPLC). K 39 SmB 110 SmA 221 N 226 I.

| | |
|---|---|
| Klp. | 215,5 °C |
| Δε | 8,4 |
| Δn | 0,223 |
| γ₁ | 249 mPa·s |

### Mischungsbeispiel 1

| | | | |
|---|---|---|---|
| CC-3-V | 26 % | Klärpunkt [°C]: | 73,5 |
| CC-3-V1 | 7% | Δn [589 nm, 20°C]: | 0,1007 |
| CCQU-3-F | 12 % | Δε [1 kHz, 20°C]: | +7,8 |
| PUQU-2-F | 9 % | γ₁ [mPa·s, 20°C]: | 57 |
| PUQU-3-F | 12 % | V₁₀ [V]: | 1,48 |
| CCP-V-1 | 14 % | | |
| CCP-30CF3 | 8 % | | |
| CCGU-3-F | 3 % | | |
| CBC-33 | 1 % | | |
| 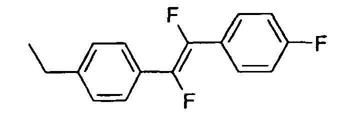 | 8% | | |
| | 100% | | |

### Mischungsbeispiel 2

| | | | |
|---|---|---|---|
| CCP-30CF3 | 4 % | Klärpunkt [°C]: | 78,0 |
| CCQU-3-F | 12 % | Δn [589 nm, 20 °C]: | 0,102 |
| CC-4-V | 14 % | Δε [1 kHz, 20 °C]: | +9,7 |
| CC-3-V1 | 14 % | γ₁ [mPa·s, 20 °C]: | 72 |
| PUQU-2-F | 14 % | V₁₀ [V]: | 1,34 |
| PUQU-3-F | 13 % | | |
| CCP-V-1 | 20 % | | |
| CCGU-3-F | 5 % | | |
| 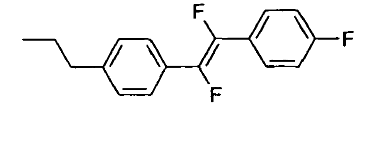 | 4% | | |
| | 100% | | |

### Mischungsbeispiel 3

| | | | |
|---|---|---|---|
| CC-3-V1 | 17 % | Klärpunkt [°C]: | 76,0 |
| CC-3-V | 31 % | Δn [589 nm, 20 °C]: | 0,102 |
| PUQU-2-F | 9 % | Δε [1 kHz, 20°C]: | +5,5 |
| PUQU-3-F | 8 % | γ₁ [mPa·s, 20°C]: | 52 |
| BCH-32 | 4% | V₁₀ [V]: | 1,86 |
| CCP-V-1 | 14 % | | |
| CCGU-3-F | 9 % | | |
| 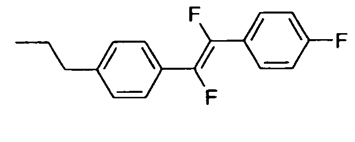 | 8% | | |
| | 100% | | |

### Mischungsbeispiel 4

| | | | |
|---|---|---|---|
| CC-3-V1 | 17 % | Klärpunkt [°C]: | 76 |
| CC-3-V | 31 % | Δn [589 nm, 20°C]: | 0,1023 |
| PUQU-2-F | 9 % | Δε [1 kHz, 20 °C]: | +5,5 |
| PUQU-3-F | 8 % | γ₁ [mPa·s, 20°C]: | 52 |
| BCH-32 | 4% | V₁₀ [V]: | 1,86 |
| CCP-V-1 | 14 % | | |
| CCGU-3-F | 9 % | | |
| 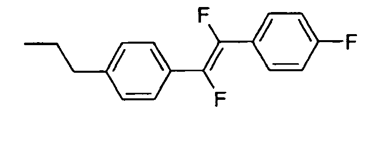 | 8% | | |
| | 100% | | |

### Mischungsbeispiel 5

| | | | |
|---|---|---|---|
| CC-3-V1 | 15 % | Klärpunkt [°C]: | 75,5 |
| CC-3-V | 31 % | Δn [589 nm, 20°C]: | 0,1066 |
| PUQU-2-F | 8 % | Δε [1 kHz, 20 °C]: | +5,3 |
| PUQU-3-F | 8 % | γ₁ [mPa·s, 20°C]: | 49 |
| BCH-32 | 8 % | V₁₀ [V]: | 1,90 |
| CCP-V-1 | 11 % | | |
| CCGU-3-F | 8 % | | |
| PP-1-2V1 | 3 % | | |
| 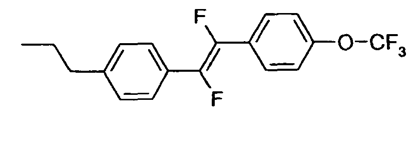 | 8% | | |
| | 100% | | |

### Mischungsbeispiel 6

| | | | |
|---|---|---|---|
| PUQU-2-F | 7 % | Klärpunkt [°C]: | 77.5 |
| PUQU-3-F | 10% | Δn [589 nm, 20°C]: | 0,1158 |
| CC-3-V | 36 % | Δε [1 kHz, 20 °C]: | +7,1 |
| CCP-V-1 | 15 % | γ₁ [mPa·s, 20 °C]: | 57 |
| CCGU-3-F | 8% | V₁₀ [V]: | 1,60 |
| BCH-32 | 10 % | | |
| PGU-3-F | 6 % | | |
| 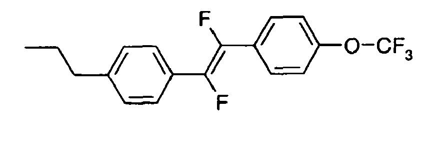 | 8% | | |
| | 100% | | |

### Mischungsbeispiel 7

| | | | |
|---|---|---|---|
| CC-3-V1 | 18 % | Klärpunkt [°C]: | 75 |
| CC-3-V | 31 % | Δn [589 nm, 20°C]: | 0,1082 |
| PUQU-2-F | 8 % | Δε [1 kHz, 20 °C]: | +5,5 |
| PUQU-3-F | 11 % | γ₁ [mPa·s, 20 °C]: | 50 |
| BCH-32 | 10 % | V₁₀ [V]: | 1,82 |
| CCP-V-1 | 6 % | | |
| CCGU-3-F | 8 % | | |
| 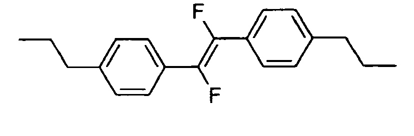 | 8% | | |
| | 100% | | |

### Mischungsbeispiel 8

| | | | |
|---|---|---|---|
| PUQU-3-F | 18 % | Klärpunkt [°C]: | 75,5 |
| CC-3-V | 31% | Δn [589 nm, 20°C]: | 0,1090 |
| CC-3-V1 | 10 % | Δε [1 kHz, 20 °C]: | +7,0 |
| CCP-V-1 | 8% | γ₁ [mPa·s, 20°C]: | 57 |
| CCGU-3-F | 8% | V₁₀ [V]: | 1,65 |
| BCH-32 | 10 % | | |
| PGU-3-F | 7 % | | |
| 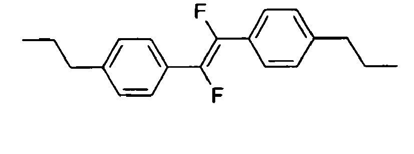 | 8% | | |
| | 100% | | |

### Mischungsbeispiel 9

| | | | |
|---|---|---|---|
| PUQU-3-F | 16 % | Klärpunkt [°C]: | 75,5 |
| CC-3-V | 43% | Δn [589 nm, 20°C]: | 0,1226 |
| CCGU-3-F | 7 % | Δε [1 kHz, 20 °C]: | +7,1 |
| BCH-32 | 6 % | γ₁ [mPa·s, 20°C]: | 56 |
| APUQU-3-F | 6 % | V₁₀ [V]: | 1,66 |
| PGP-2-4 | 7 % | | |
| PGP-2-3 | 4 % | | |
| CBC-33 | 3 % | | |
| 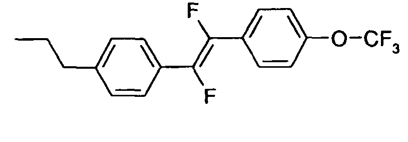 | 8% | | |
| | 100% | | |

### Mischungsbeispiel 10

| | | | |
|---|---|---|---|
| PUQU-3-F | 16 % | Klärpunkt [°C]: | 74,5 |
| CC-3-V | 43.5% | Δn [589 nm, 20 °C]: | 0,1201 |
| CCGU-3-F | 9 % | Δε [1 kHz, 20 °C]: | +7,1 |
| BCH-32 | 5.5 % | γ₁ [mPa·s, 20°C]: | 57 |
| APUQU-3-F | 6 % | V₁₀ [V]: | 1,64 |
| PGP-2-4 | 5 % | | |
| PGP-2-3 | 4 % | | |
| CBC-33 | 3 % | | |
| 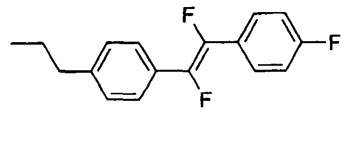 | 8% | | |
| | 100% | | |

### Mischungsbeispiel 11

| | | | |
|---|---|---|---|
| PUQU-3-F | 10 % | Klärpunkt [°C]: | 75 |
| CC-3-V | 43% | Δn [589 nm, 20 °C]: | 0,1084 |
| CC-3-V1 | 13 % | Δε [1 kHz, 20 °C]: | +5,3 |
| CCGU-3-F | 7 % | γ₁ [mPa·s, 20°C]: | 48 |
| APUQU-3-F | 6 % | V₁₀ [V]: | 1,88 |
| PGP-2-4 | 6 % | | |
| PGP-2-3 | 4 % | | |
| CBC-33 | 3 % | | |
| 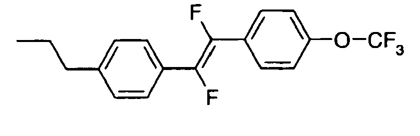 | 8% | | |
| | 100% | | |

### Mischungsbeispiel 12

| | | | |
|---|---|---|---|
| CC-3-V | 40 % | Klärpunkt [°C]: | 74,5 |
| CC-3-V1 | 11 % | Δn [589 nm, 20°C]: | 0,1187 |
| PUQU-3-F | 3 % | Δε [1 kHz, 20°C]: | +4,6 |
| PGU-2-F | 7% | γ₁ [mPa·s, 20°C]: | 49 |
| PGU-3-F | 10% | V₁₀ [V]: | 1,94 |
| PGP-2-3 | 5 % | | |
| PGP-2-4 | 3.5% | | |
| CCP-30CF3 | 2 % | | |
| CCP-V-1 | 6.5% | | |
| CCGU-3-F | 3 % | | |
| CBC-33 | 3 % | | |
| 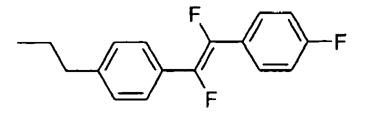 | 6% | | |
| | 100% | | |

### Mischungsbeispiel 13

| | | | |
|---|---|---|---|
| PUQU-3-F | 17 % | Klärpunkt [°C]: | 76 |
| CC-3-V | 37% | Δn [589 nm, 20 °C]: | 0,1193 |
| CC-3-V1 | 5 % | Δε [1 kHz, 20°C]: | +7,3 |
| CCP-V-1 | 6 % | γ₁ [mPa·s, 20 °C]: | 56 |
| CCGU-3-F | 8% | V₁₀ [V]: | 1,64 |
| BCH-32 | 10 % | | |
| PGU-3-F | 5 % | | |
| PPGU-4-F | 3 % | | |
| PGP-2-3 | 3 % | | |
| 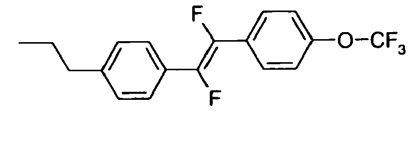 | 6% | | |
| | 100% | | |

### Mischungsbeispiel 14

| | | | |
|---|---|---|---|
| CC-3-V1 | 15 % | Klärpunkt [°C]: | 76 |
| CC-3-V | 36 % | Δn [589 nm, 20 °C]: | 0,1092 |
| PUQU-3-F | 16 % | Δε [1 kHz, 20 °C]: | +5,6 |
| BCH-32 | 10% | γ₁ [mPa·s, 20°C]: | 51 |
| CCP-V-1 | 4% | V₁₀ [V]: | 1,87 |
| CCGU-3-F | 7 % | | |
| PPGU-4-F | 3 % | | |
| PGP-2-4 | 3 % | | |
| 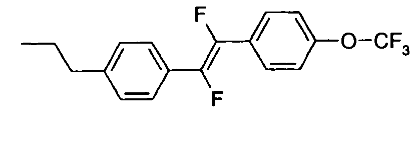 | 6% | | |
| | 100% | | |

### Mischungsbeispiel 15

| | | | |
|---|---|---|---|
| PUQU-3-F | 17 % | Klärpunkt [°C]: | 75 |
| CC-3-V | 38 % | Δn [589 nm, 20 °C]: | 0,1184 |
| CCP-V-1 | 9 % | Δε [1 kHz, 20 °C]: | +7,0 |
| CCGU-3-F | 10 % | γ₁ [mPa·s, 20°C]: | 58 |
| BCH-32 | 10 % | V₁₀ [V]: | 1,65 |
| PGU-3-F | 6 % | | |
| PGP-2-4 | 4 % | | |
| 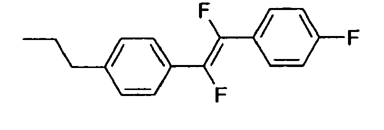 | 6% | | |
| | 100% | | |

### Mischungsbeispiel 16

| | | | |
|---|---|---|---|
| PGU-2-F | 5% | Klärpunkt [°C]: | 75,0 |
| CDU-2-F | 6 % | Δn [589 nm, 20 °C]: | 0,0972 |
| CCZU-3-F | 15 % | Δε [1 kHz, 20 °C]: | +8,5 |
| CC-3-V1 | 13 % | γ₁ [mPa·s, 20°C]: | 61 |
| CC-3-V | 21 % | k₁ [pN, 20°C] | 13,1 |
| CCP-V-1 | 6 % | k₃/k₁ [pN, 20°C] | 0,95 |
| CCP-30CF3 | 8% | V₀ [V, 20°C] | 1,30 |
| CCP-40CF3 | 6 % | | |
| PUQU-2-F | 7 % | | |
| PUQU-3-F | 7 % | | |
| 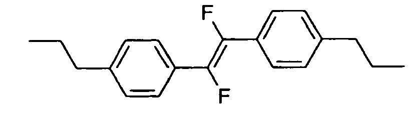 | 6% | | |
| | 100% | | |

### Mischungsbeispiel 17

| | | | |
|---|---|---|---|
| PGU-2-F | 6.5% | Klärpunkt [°C]: | 75,5 |
| CDU-2-F | 4.5% | Δn [589 nm, 20°C]: | 0,0976 |
| CCZU-3-F | 12 % | Δε [1 kHz, 20°C]: | +8,4 |
| CC-3-V1 | 13 % | γ₁ [mPa·s, 20°C]: | 61 |
| CC-3-V | 21 % | k₁ [pN, 20°C] | 12,6 |
| CCP-V-1 | 8% | k₃/k₁ [pN, 20 °C] | 1,02 |
| CCP-30CF3 | 8% | V₀ [V, 20°C] | 1,29 |
| CCP-40CF3 | 8 % | | |
| PUQU-2-F | 7 % | | |
| PUQU-3-F | 6 % | | |
| 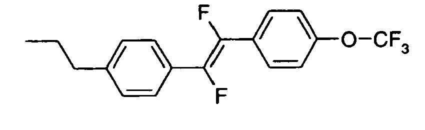 | 6% | | |
| | 100% | | |

### Mischungsbeispiel 18

| | | | |
|---|---|---|---|
| CC-3-V | 19 % | Klärpunkt [°C]: | 75,5 |
| CC-3-V1 | 13 % | Δn [589 nm, 20°C]: | 0,0975 |
| CCP-30CF3 | 8 % | Δε [1 kHz, 20 °C]: | +8,6 |
| CCP-40CF3 | 8 % | γ₁ [mPa·s, 20°C]: | 65 |
| CCP-V-1 | 8% | k₁ [pN, 20 °C] | 12,5 |
| CCZU-3-F | 13 % | k₃/k₁ [pN, 20°C] | 1,01 |
| CDU-2-F | 6.5% | V₀ [V, 20°C] | 1,27 |
| PGU-2-F | 5.5% | | |
| PUQU-2-F | 7 % | | |
| PUQU-3-F | 6 % | | |
| 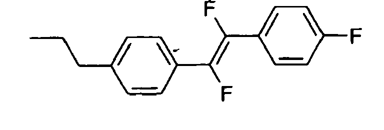 | 6% | | |
| | 100% | | |

### Mischungsbeispiel 19

| | | | |
|---|---|---|---|
| CC-3-V | 13 % | Klärpunkt [°C]: | 78,5 |
| CC-3-V1 | 12 % | Δn [589 nm, 20 °C]: | 0,1105 |
| CCGU-3-F | 5 % | Δε [1 kHz, 20°C]: | +11,4 |
| CCP-30CF3 | 8 % | γ₁ [mPa·s, 20 °C]: | 78 |
| CCP-V-1 | 10.5% | k₁ [pN, 20 °C] | 13,0 |
| CCZU-3-F | 12 % | k₃/k₁ [pN, 20°C] | 0,98 |
| CDU-2-F | 9% | V₀ [V, 20°C] | 1,12 |
| PGU-2-F | 7.5% | | |
| PUQU-2-F | 8.5% | | |
| PUQU-3-F | 8.5% | | |
| 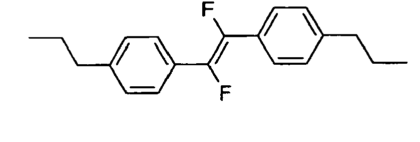 | 6% | | |
| | 100% | | |

### Mischungsbeispiel 20

| | | | |
|---|---|---|---|
| CC-3-V | 14.5% | Klärpunkt [°C]: | 79,0 |
| CC-3-V1 | 12 % | Δn [589 nm, 20°C]: | 0,1097 |
| CCGU-3-F | 7% | Δε [1 kHz, 20 °C]: | +11,3 |
| CCP-30CF3 | 7 % | γ₁ [mPa·s, 20°C]: | 78 |
| CCP-V-1 | 12.5% | k₁ [pN, 20 °C] | 12,4 |
| CCZU-3-F | 9% | k₃/k₁ [pN, 20°C] | 1,06 |
| CDU-2-F | 7% | V₀ [V, 20 °C] | 1,10 |
| PGU-2-F | 8% | | |
| PUQU-2-F | 8.5% | | |
| PUQU-3-F | 8.5% | | |
| 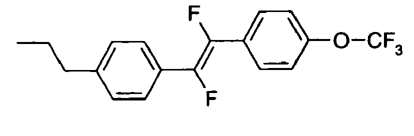 | 6% | | |
| | 100% | | |

### Mischungsbeispiel 21

| | | | |
|---|---|---|---|
| CC-3-V | 12.5 % | Klärpunkt [°C]: | 79 |
| CC-3-V1 | 12 % | Δn [589 nm, 20°C]: | 0,1100 |
| CCGU-3-F | 7 % | Δε [1 kHz, 20°C]: | +11,4 |
| CCP-30CF3 | 8 % | γ₁ [mPa·s, 20°C]: | 81 |
| CCP-V-1 | 12.5% | k₁ [pN, 20°C] | 12,2 |
| CCZU-3-F | 9% | k₃/k₁ [pN, 20°C] | 1,07 |
| CDU-2-F | 9% | V₀ [V, 20 °C] | 1,09 |
| PGU-2-F | 7 % | | |
| PUQU-2-F | 8.5% | | |
| PUQU-3-F | 8.5 % | | |
| 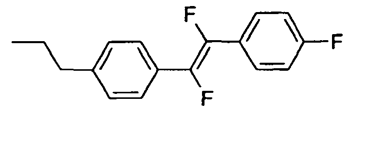 | 6% | | |
| | 100% | | |

### Mischungsbeispiel 22

| | | | |
|---|---|---|---|
| APUQU-2-F | 8 % | Klärpunkt [°C]: | 73,0 |
| CC-3-V | 25% | Δn [589 nm, 20°C]: | 0,1005 |
| CC-3-V1 | 13 % | Δε [1 kHz, 20 °C]: | +8,6 |
| CCP-V-1 | 10.5% | γ₁ [mPa·s, 20°C]: | 59 |
| CCP-V2-1 | 10 % | k₁ [pN, 20°C] | 12,8 |
| CDU-2-F | 10 % | k₃/k₁ [pN, 20°C] | 1,01 |
| PUQU-2-F | 8.5% | V₀ [V, 20 °C] | 1,28 |
| PUQU-3-F | 9 % | | |
| 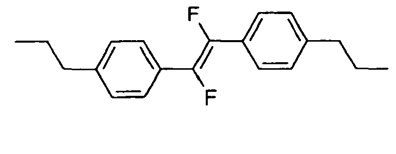 | 6% | | |
| | 100% | | |

### Mischungsbeispiel 23

| | | | |
|---|---|---|---|
| APUQU-2-F | 8 % | Klärpunkt [°C]: | 74,5 |
| CC-3-V | 26 % | Δn [589 nm, 20 °C]: | 0,0996 |
| CC-3-V1 | 12 % | Δε [1 kHz, 20 °C]: | +8,5 |
| CCP-V-1 | 10.5% | γ₁ [mPa·s, 20°C]: | 59 |
| CCP-V2-1 | 12 % | k₁ [pN, 20 °C] | 12,7 |
| CDU-2-F | 8 % | k₃/k₁ [pN, 20°C] | 1,05 |
| PGU-2-F | 2 % | V₀ [V, 20 °C] | 1,28 |
| PUQU-2-F | 7.5 % | | |
| PUQU-3-F | 8 % | | |
| 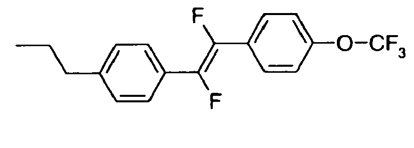 | 6% | | |
| | 100% | | |

### Mischungsbeispiel 24

| | | | |
|---|---|---|---|
| APUQU-2-F | 8% | Klärpunkt [°C]: | 73 |
| CC-3-V | 25 % | Δn [589 nm, 20°C]: | 0,1000 |
| CC-3-V1 | 12 % | Δε [1 kHz, 20 °C]: | +8,5 |
| CCP-V-1 | 10.5% | γ₁ [mPa·s, 20°C]: | 60 |
| CCP-V2-1 | 12 % | k₁ [pN, 20 °C] | 12,2 |
| CDU-2-F | 9.5% | k₃/k₁ [pN, 20°C] | 1,07 |
| PGU-2-F | 1.5% | V₀ [V, 20 °C] | 1,25 |
| PUQU-2-F | 7.5% | | |
| PUQU-3-F | 8 % | | |
| 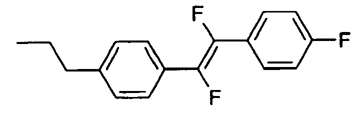 | 6% | | |
| | 100% | | |

### Mischungsbeispiel 25

| | | | |
|---|---|---|---|
| APUQU-2-F | 9% | Klärpunkt [°C]: | 78 |
| CC-3-V | 16 % | Δn [589 nm, 20°C]: | 0,1118 |
| CC-3-V1 | 12 % | Δε [1 kHz, 20°C]: | +11,1 |
| CCP-30CF3 | 7 % | γ₁ [mPa·s, 20 °C]: | 74 |
| CCP-V-1 | 11 % | | |
| CCP-V2-1 | 10% | | |
| CDU-2-F | 5.5% | | |
| PGU-2-F | 5% | | |
| PUQU-2-F | 10 % | | |
| PUQU-3-F | 10 % | | |
| 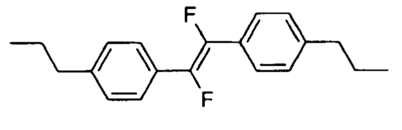 | 4.5% | | |
| | 100% | | |

### Mischungsbeispiel 26

| | | | |
|---|---|---|---|
| APUQU-2-F | 9 % | Klärpunkt [°C]: | 79,5 |
| CC-3-V | 15.5% | Δn [589 nm, 20 °C]: | 0,1112 |
| CC-3-V1 | 12 % | Δε [1 kHz, 20 °C]: | +11,1 |
| CCP-30CF3 | 7.5 % | γ₁ [mPa·s, 20 °C]: | 72 |
| CCP-V-1 | 11.5% | | |
| CCP-V2-1 | 11 % | | |
| CDU-2-F | 3.5% | | |
| PGU-2-F | 5 % | | |
| PUQU-2-F | 10 % | | |
| PUQU-3-F | 10 % | | |
| 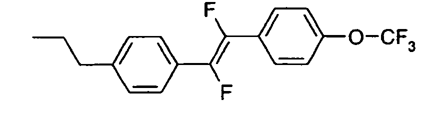 | 5% | | |
| | 100% | | |

### Mischungsbeispiel 27

| | | | |
|---|---|---|---|
| APUQU-2-F | 9% | Klärpunkt [°C]: | 78,5 |
| CC-3-V | 14.5% | Δn [589 nm, 20 °C]: | 0,1114 |
| CC-3-V1 | 12 % | Δε [1 kHz, 20 °C]: | +11,2 |
| CCP-30CF3 | 7.5 % | γ₁ [mPa·s, 20 °C]: | 76 |
| CCP-V-1 | 11.5% | k₁ [pN, 20°C] | 12,7 |
| CCP-V2-1 | 11 % | k₃/k₁ [pN, 20°C] | 1,08 |
| CDU-2-F | 5% | V₀ [V, 20 °C] | 1,12 |
| PGU-2-F | 4.5% | | |
| PUQU-2-F | 10% | | |
| PUQU-3-F | 10% | | |
| 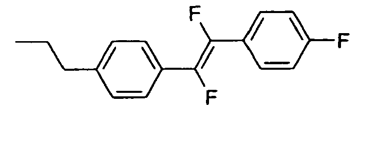 | 5% | | |
| | 100% | | |

### Mischungsbeispiel 28

| | | | |
|---|---|---|---|
| CC-3-V | 21 % | Klärpunkt [°C]: | 74 |
| CC-3-V1 | 6 % | Δn [589 nm, 20 °C]: | 0,1192 |
| CCQU-2-F | 11 % | Δε [1 kHz, 20°C]: | +11,2 |
| PUQU-3-F | 17 % | ε_{∥} [1 kHz, 20 °C]: | +15,0 |
| PGU-2-F | 7 % | γ₁ [mPa·s, 20°C]: | 77 |
| PGU-3-F | 12 % | V₁₀ [V]: | 1,23 |
| CCP-V-1 | 17 % | V₉₀ [V]: | 1,88 |
| CCGU-3-F | 3 % | | |
| 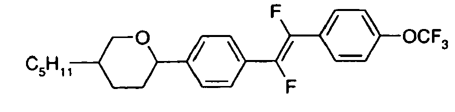 | 6% | | |
| | 100% | | |

### Mischungsbeispiel 29

| | | | |
|---|---|---|---|
| CC-3-V | 24 % | Klärpunkt [°C]: | 75,5 |
| CCQU-2-F | 6 % | Δn [589 nm, 20 °C]: | 0,1210 |
| PUQU-3-F | 17 % | Δε [1 kHz, 20 °C]: | +12,7 |
| PGU-2-F | 5% | ε_{∥} [1 kHz, 20°C]: | +16,4 |
| PGU-3-F | 6 % | γ₁ [mPa·s, 20 °C]: | 82 |
| CCP-30CF3 | 8% | V₁₀ [V]: | 1,21 |
| PGP-2-3 | 1% | V₉₀ [V]: | 1,88 |
| CCP-V-1 | 18 % | | |
| CCGU-3-F | 3 % | | |
| 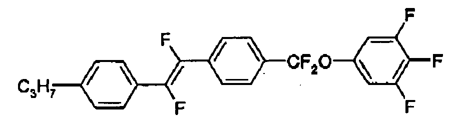 | 12% | | |
| | 100 % | | |

### Mischungsbeispiel 30

| | | | |
|---|---|---|---|
| CC-3-V | 24 % | Klärpunkt [°C]: | 77,5 |
| CCQU-2-F | 6% | Δn [589 nm, 20 °C]: | 0,1209 |
| PUQU-3-F | 17 | Δε [1 kHz, 20°C]: | +12,2 |
| PGU-2-F | 5% | ε_{∥} [1 kHz, 20°C]: | +15,9 |
| PGU-3-F | 6% | γ₁ [mPa·s, 20°C]: | 78 |
| CCP-30CF3 | 8% | V₁₀ [V]: | 1,25 |
| PGP-2-3 | 2 % | V₉₀ [V] | 1,90 |
| CCP-V-1 | 18 % | | |
| CCGU-3-F | 4 % | | |
| 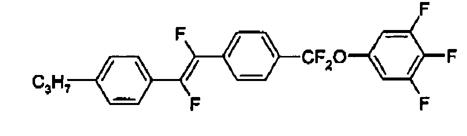 | 10% | | |
| | 100% | | |

### Mischungsbeispiel 31

| | | | |
|---|---|---|---|
| PGU-2-F | 4% | Klärpunkt [°C]: | 69 |
| PUQU-2-F | 8% | Δn [589 nm, 20 °C]: | 0,1091 |
| GGP-3-CL | 4 % | Δε [1 kHz, 20°C]: | +4,1 |
| CC-3-V | 35.5% | ε_{∥} [1 kHz, 20 °C]: | +6,9 |
| CC-3-V1 | 13 % | γ₁ [mPa·s, 20°C]: | 47 |
| PP-1-2V1 | 9% | k₁ [pN, 20 °C] | 13,0 |
| CCP-V-1 | 11 % | k₃/k₁ [pN, 20 °C] | 1,06 |
| CCP-V2-1 | 2.5 % | V₀ [V, 20°C] | 1,88 |
| BCH-32 | 8 % | | |
| 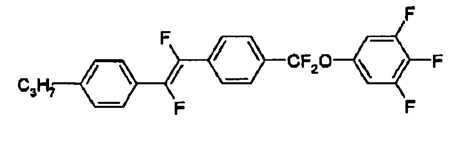 | 5% | | |
| | 100 % | | |

### Mischungsbeispiel 32

| | | | |
|---|---|---|---|
| CDU-2-F | 2 % | Klärpunkt [°C]: | 75,5 |
| PGU-2-F | 3 % | Δn [589 nm, 20 °C]: | 0,0996 |
| PUQU-2-F | 7.5% | Δε [1 kHz, 20 °C]: | +8,7 |
| PUQU-3-F | 8% | ε_{∥} [1 kHz, 20 °C]: | +12,1 |
| CCP-V-1 | 11 % | γ₁ [mPa·s, 20 °C]: | 59 |
| CCP-V2-1 | 11.5% | k₁ [pN, 20°C] | 12,4 |
| CC-3-V1 | 12.5% | k₃/k₁ [pN, 20 °C] | 1,12 |
| CC-3-V | 30.5% | V₀ [V, 20 °C] | 1,25 |
| APUQU-2-F | 8 % | | |
| 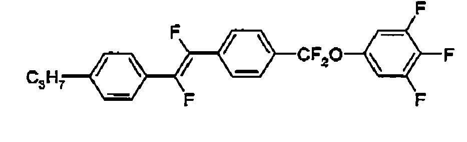 | 6% | | |
| | 100 % | | |

### Mischungsbeispiel 33

| | | | |
|---|---|---|---|
| CDU-2-F | 4.5% | Klärpunkt [°C]: | 74 |
| PGU-2-F | 5% | Δn [589 nm, 20 °C]: | 0,1000 |
| PUQU-2-F | 8 % Δε | [1 kHz, 20 °C]: | +8,6 |
| PUQU-3-F | 8 % | ε_{∥} [1 kHz, 20 °C]: | +12,1 |
| CCP-V-1 | 11 % | γ₁ [mPa·s, 20 °C]: | 60 |
| CCP-V2-1 | 6 % | k₁ [pN, 20°C] | 12,1 |
| CC-3-V1 | 12 % | k₃/k₁ [pN, 20 °C] | 1,10 |
| CC-3-V | 31.5% | V₀ [V, 20°C] | 1,25 |
| APUQU-2-F | 8% | | |
| 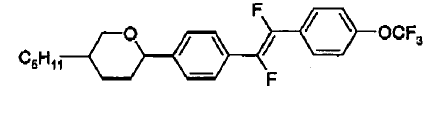 | 6% | | |
| | 100% | | |

### Mischungsbeispiel 34

| | | | |
|---|---|---|---|
| CCP-30CF3 | 7 % | Klärpunkt [°C]: | 80 |
| PGU-2-F | 5.5% | Δn [589 nm, 20 °C]: | 0,1096 |
| PUQU-2-F. | 8.5 % | Δε [1 kHz, 20 °C]: | +10,9 |
| PUQU-3-F | 9% | ε_{∥} [1 kHz, 20°C]: | +14,4 |
| CC-3-V1 | 12 % | γ₁ [mPa·s, 20 °C]: | 71 |
| CC-3-V | 21.5% | k₁ [pN, 20 °C] | 13,0 |
| CCP-V-1 | 11.5 % | k₃/k₁ [pN, 20 °C] | 1,08 |
| CCP-V2-1 | 10% | V₀ [V, 20 °C] | 1,15 |
| APUQU-2-F | 8.5% | | |
| 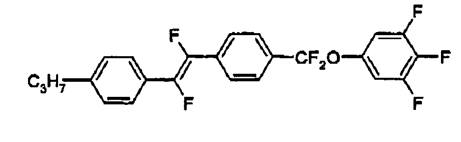 | 6.5% | | |
| | 100 % | | |

### Mischungsbeispiel 35

| | | | |
|---|---|---|---|
| PUQU-2-F | 11 % | Klärpunkt [°C]: | 75 |
| PUQU-3-F | 9.5% | Δn [589 nm, 20 °C]: | 0,1001 |
| CCP-30CF3 | | 8% Δε [1 kHz, 20 °C]: | +13,9 |
| CC-3-V | 25% | ε_{∥} [1 kHz, 20 °C]: | +17,9 |
| CC-3-V1 | 11 % | γ₁ [mPa·s, 20°C]: | 79 |
| CCZU-3-F | 1.5% | k₁ [pN, 20 °C] | 11,4 |
| CCQU-3-F | 10 % | k₃/k₁ [20°C] | 1,11 |
| CCGU-3-F | 10 % | V₀ [V, 20°C] | 0,96 |
| APUQU-2-F | 9 % | | |
| 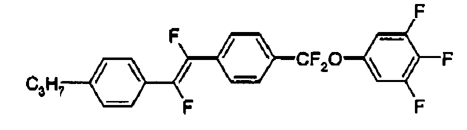 | 5% | | |
| | 100 % | | |

### Mischungsbeispiel 36

| | | | |
|---|---|---|---|
| PUQU-2-F | 11 % | Klärpunkt [°C]: | 75,5 |
| PUQU-3-F | 9.5 % | Δn [589 nm, 20 °C]: | 0,0996 |
| CCZU-3-F | 9.5% | Δε [1 kHz, 20°C]: | +13,4 |
| CC-3-V | 35% | ε_{∥} [1 kHz, 20°C]: | +17,5 |
| CCQU-3-F | 10 % | γ₁ [mPa·s, 20 °C]: | 80 |
| CCGU-3-F | 9 % | k₁ [pN, 20°C] | 11,0 |
| APUQU-2-F | 9 % | k₃/k₁ [20 °C] | 1,07 |
| 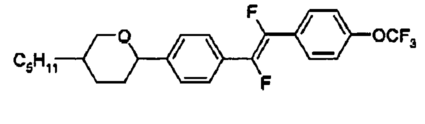 | 7 % | V₀ [V, 20 °C] | 0,95 |
| | 100% | | |

## Patentansprüche

1. Flüssigkristallines Medium mit positiver dielektrischer Anisotropie auf der Basis eines Gemisches von Verbindungen, **dadurch gekennzeichnet, dass** es eine oder mehrere Verbindungen der Formel I 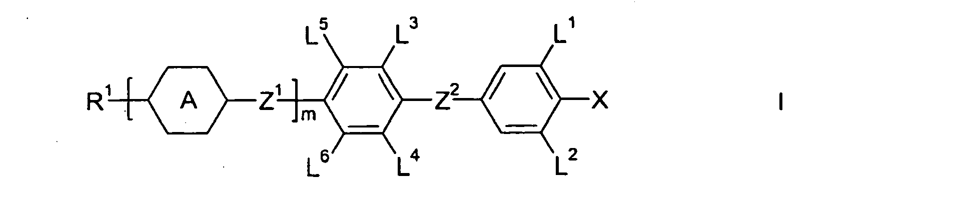 enthält, worin
R¹ einen halogenierten oder unsubstituierten Alkyl- oder Alkoxyrest mit 1 bis 15 C-Atomen, wobei in diesen Resten auch eine oder mehrere CH₂-Gruppen jeweils unabhängig voneinander durch -C≡C-, -CH=CH-, -O-, -CO-O- oder -O-CO- so ersetzt sein können, dass O-Atome nicht direkt miteinander verknüpft sind,
A ein nach links oder rechts ausgerichtetes Ringsystem der Formeln 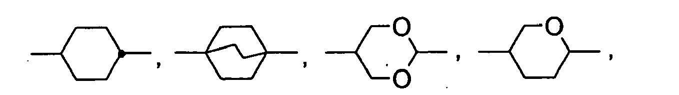 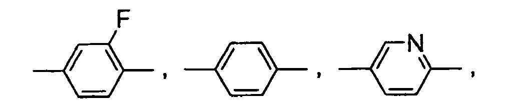 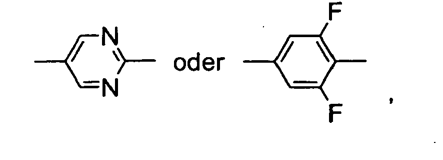
Z¹, Z² eine Einfachbindung; -C≡C-, -CF=CF-, -CH=CH-, -CF₂O-, oder -CH₂CH₂-, wobei mindestens eine Gruppe aus Z¹ und Z² die Gruppe -CF=CF- bedeutet,
X F, Cl, CN, SF₅, oder einen halogenierten oder unsubstituierten Alkyl- oder Alkoxyrest mit 1 bis 15 C- Atomen, wobei in diesen Resten auch eine oder mehrere CH₂-Gruppen jeweils unabhängig voneinander durch -C≡C-, -CH=CH-, -O-, -CO-O- oder -O-CO- so ersetzt sein können, dass O-Atome nicht direkt miteinander verknüpft sind,
L¹, L², L³, L⁴, L⁵ und L⁶ jeweils unabhängig voneinander H oder F, und
m 0 oder 1
bedeuten,
und
zusätzlich eine oder mehrere ausgewählte Verbindungen der allgemeinen Formeln K-1 bis K-12 enthält: 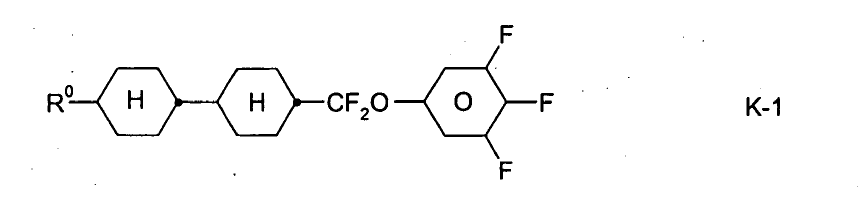 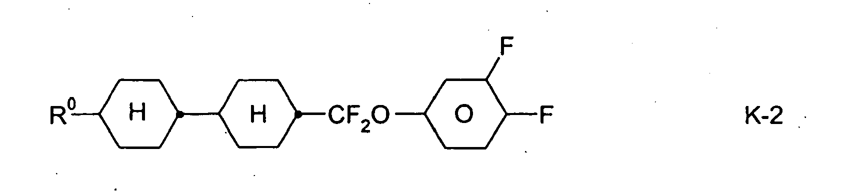 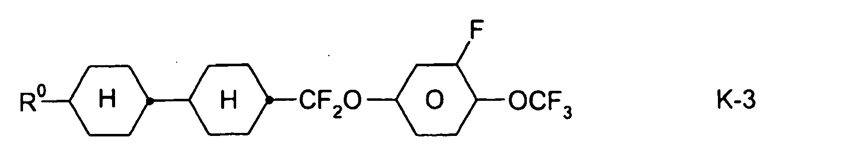 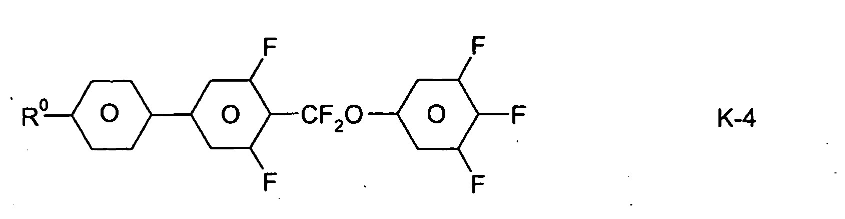 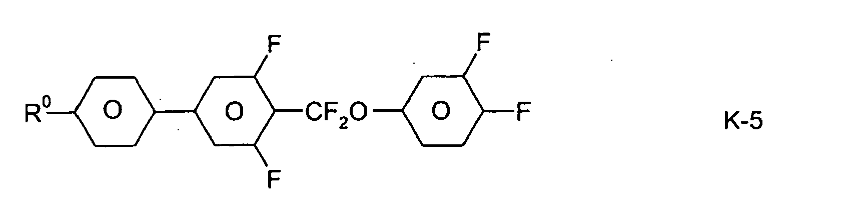 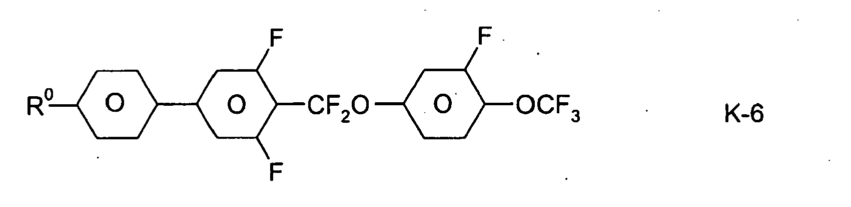 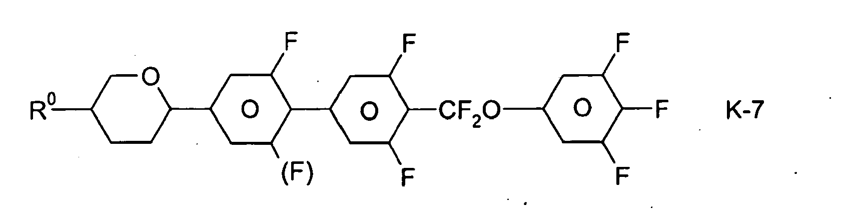 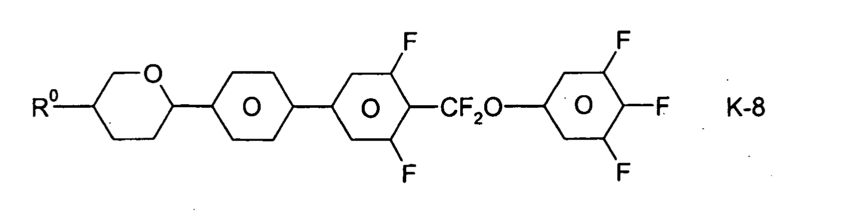 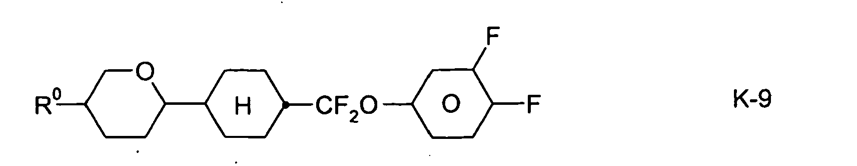 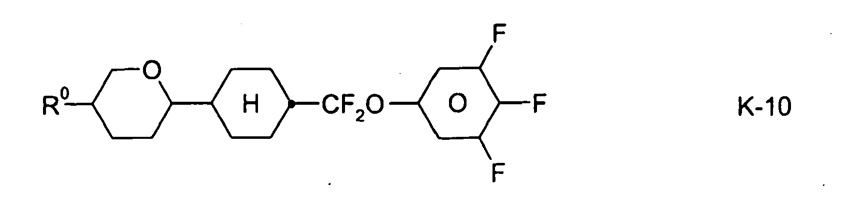 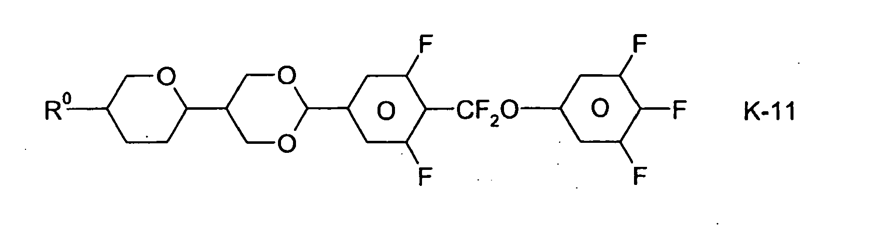 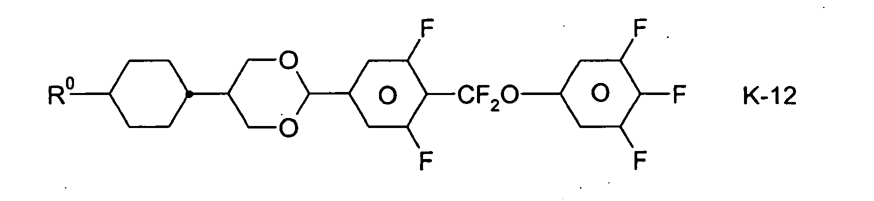 worin
R⁰ n-Alkyl, Oxaalkyl, Fluoralkyl oder Alkenyl mit jeweils bis zu 9 C-Atomen
bedeutet.

2. Flüssigkristallines Medium nach Anspruch 1, **dadurch gekennzeichnet, dass**
X F, Cl, CN, oder einen halogenierten Alkyl- oder Alkoxyrest mit 1 bis 15 C-Atomen, wobei in diesen Resten auch eine oder mehrere CH₂-Gruppen jeweils unabhängig voneinander durch -C≡C-, -CH=CH-, -O-, -CO-O- oder -O-CO- so ersetzt sein können, dass O-Atome nicht direkt miteinander verknüpft sind,
bedeutet.

3. Flüssigkristallines Medium nach Anspruch 1 oder 2, **dadurch gekennzeichnet, dass**
m 1
bedeutet.

4. Flüssigkristallines Medium gemäß mindestens einem der Ansprüche 1 bis 3, **dadurch gekennzeichnet, dass** es ein, zwei oder mehr Verbindungen der Formeln I-1 bis I-30 enthält: 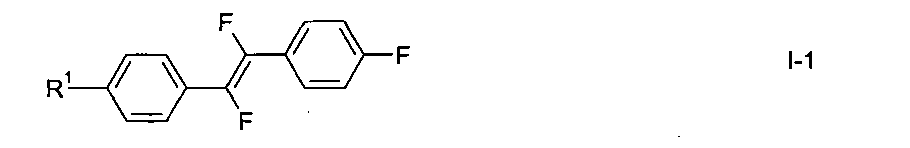 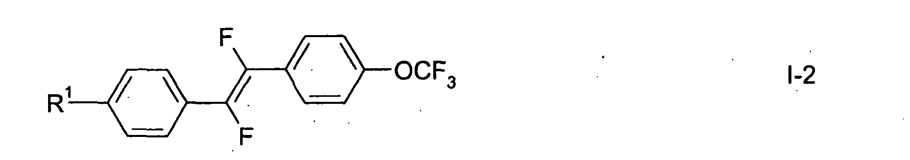 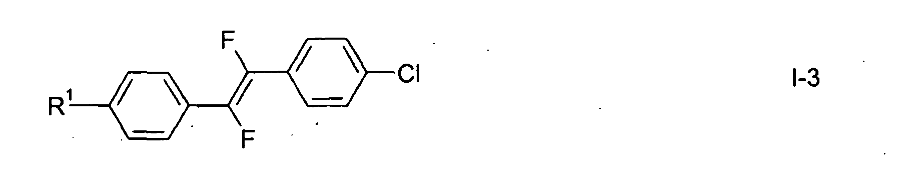 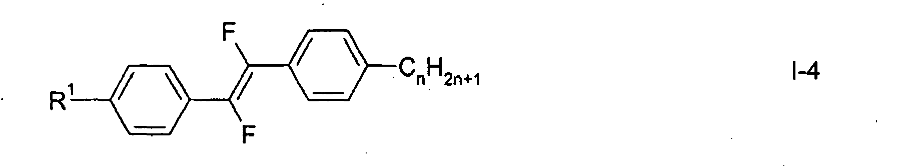 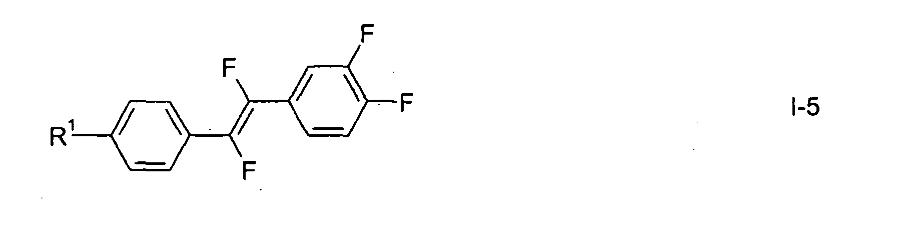 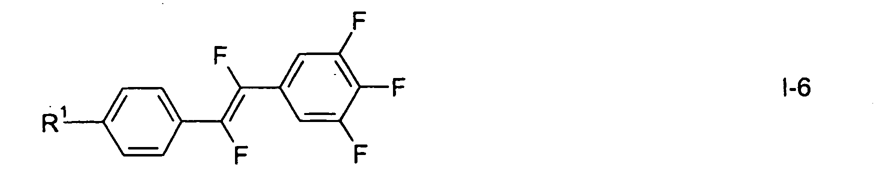 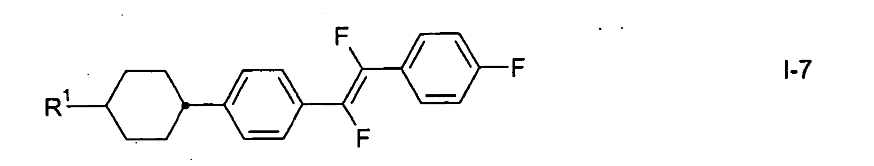 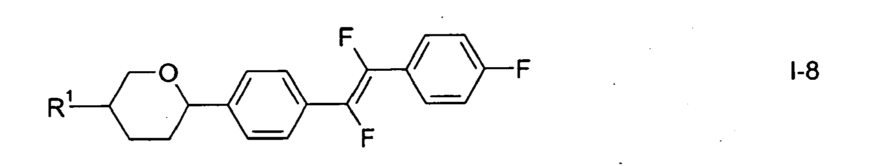 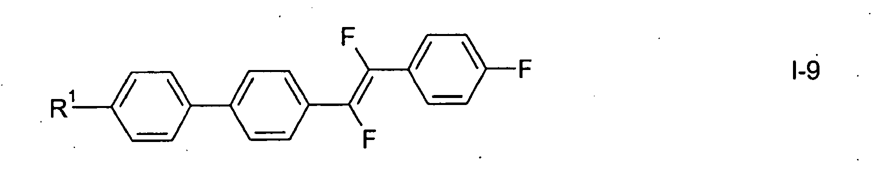 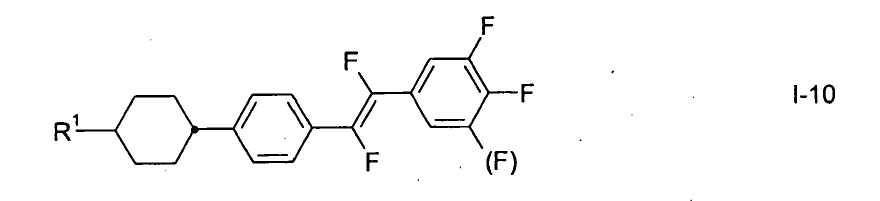 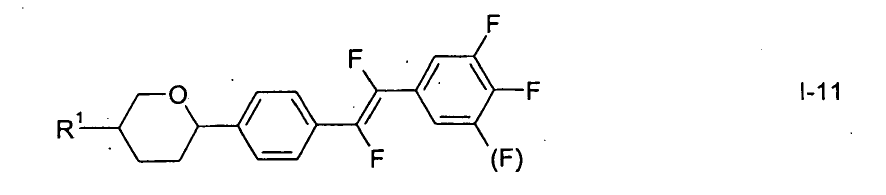 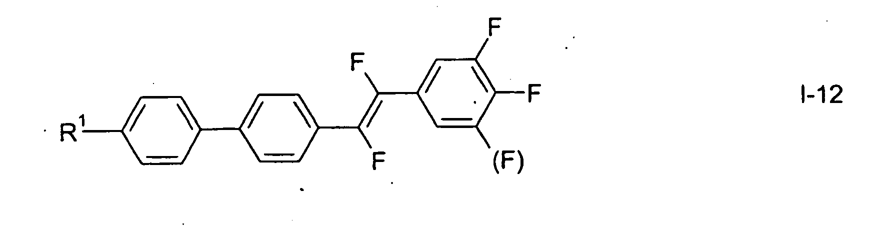 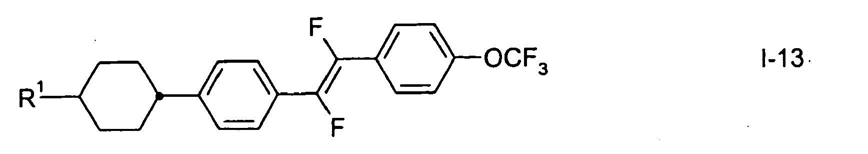 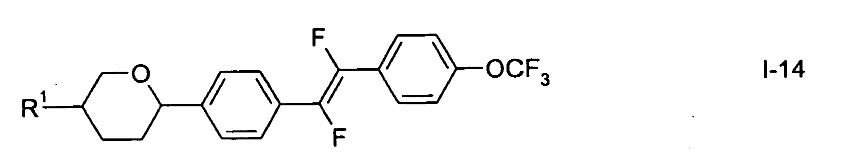 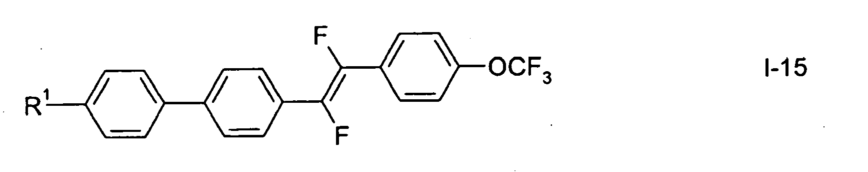 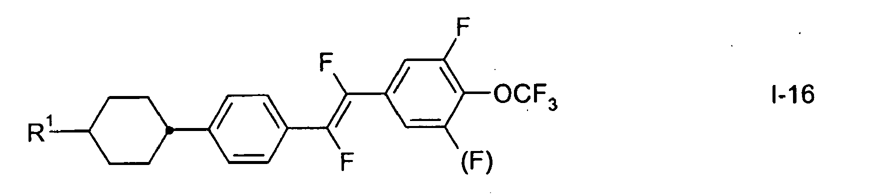 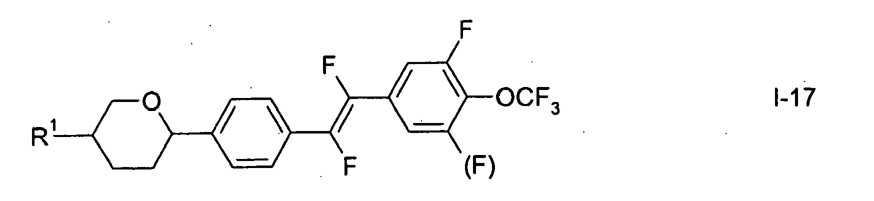 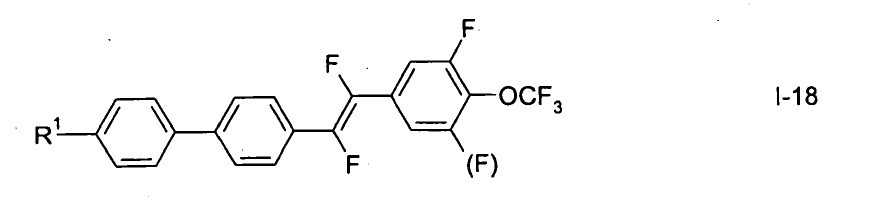 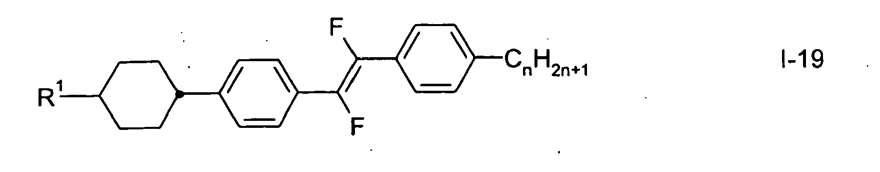 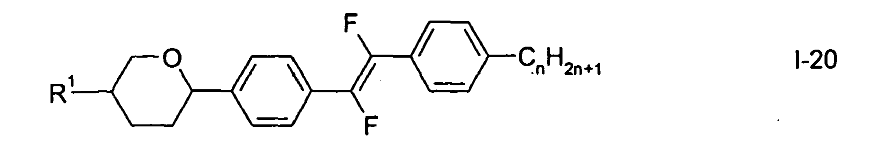 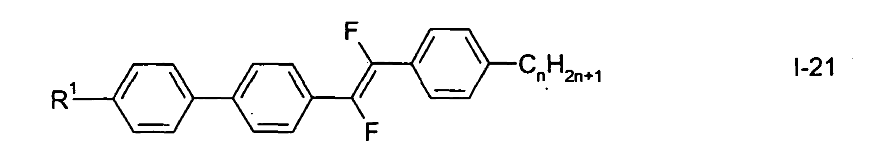 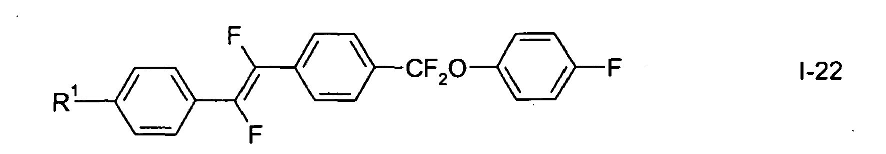 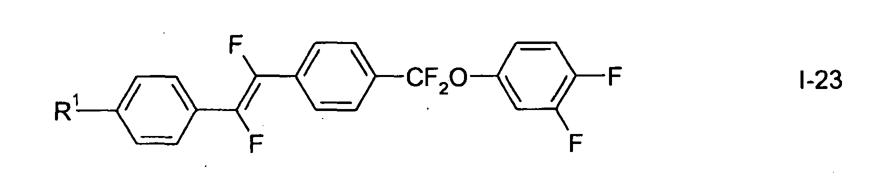 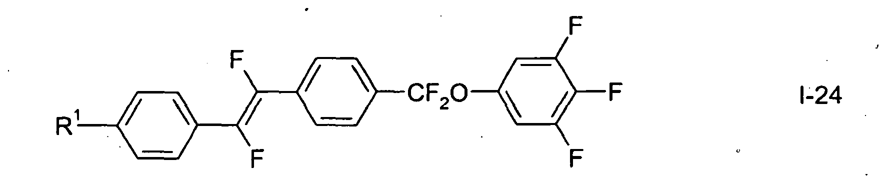 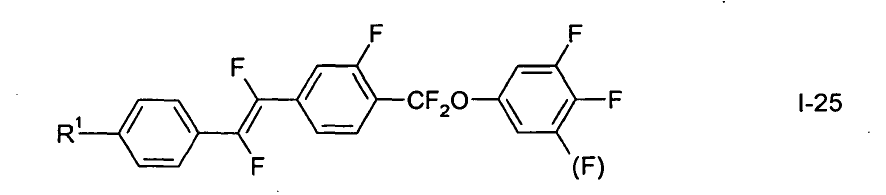 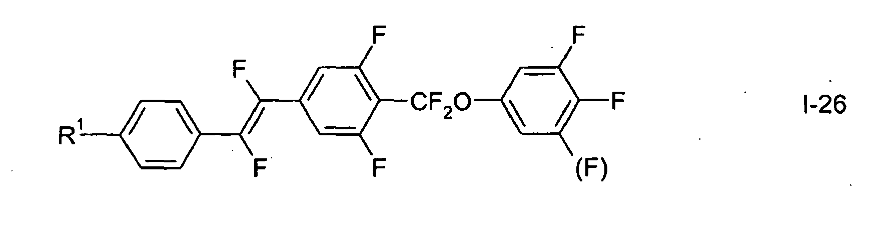 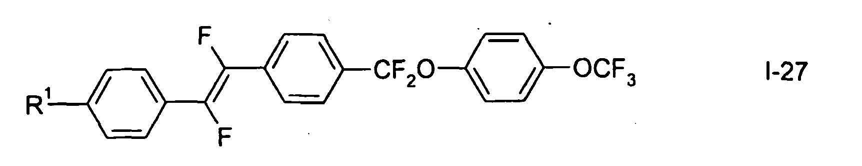 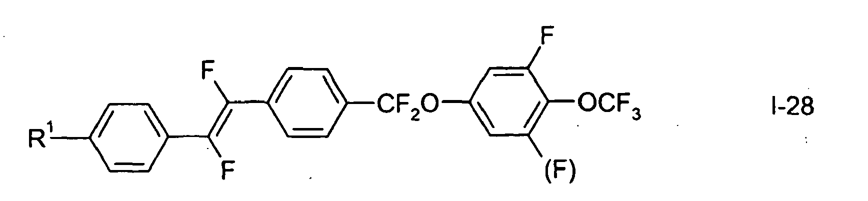 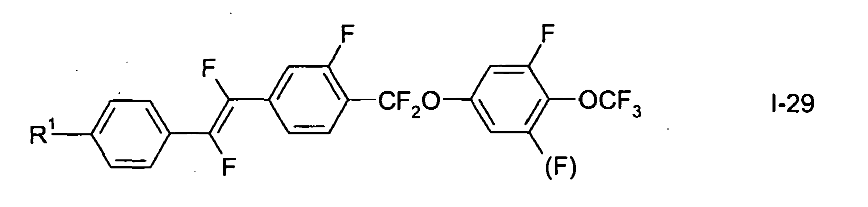 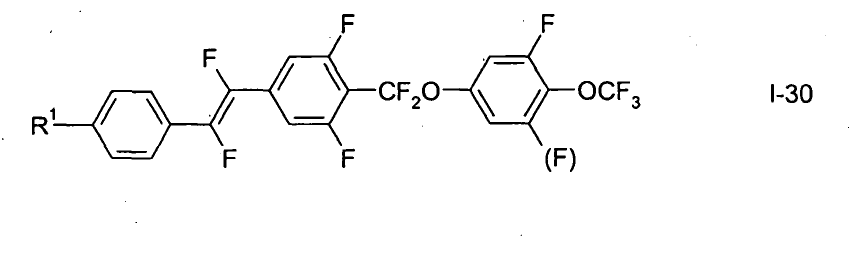 worin
R¹ die in Anspruch 1 angegebene Bedeutung hat und n für 1, 2, 3, 4, 5, 6, 7 oder 8 steht.

5. Flüssigkristallines Medium gemäß mindestens einem der Ansprüche 1 bis 4, **dadurch gekennzeichnet, dass** es zusätzlich eine oder mehrere ausgewählte Verbindungen der allgemeinen Formeln Z-1 bis Z-9 enthält: 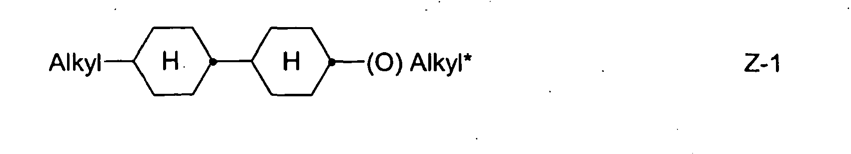 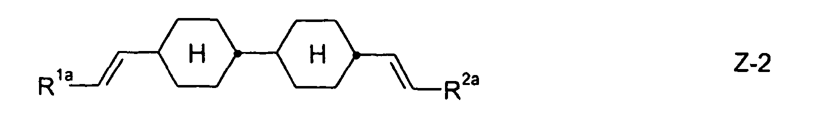 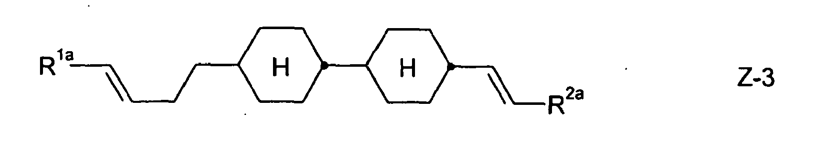 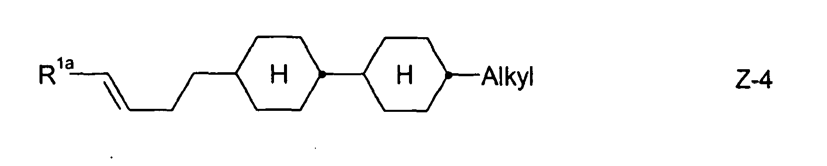 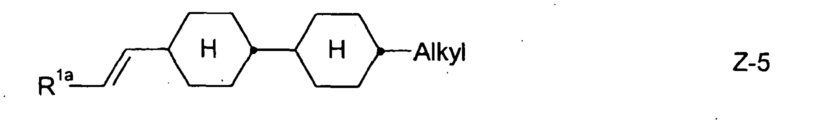 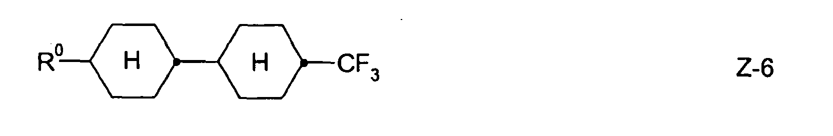 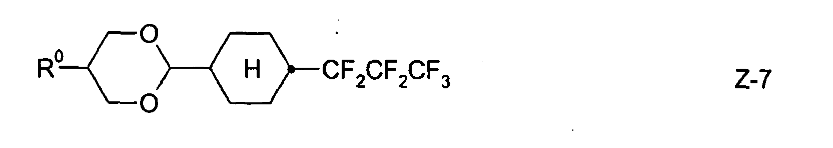 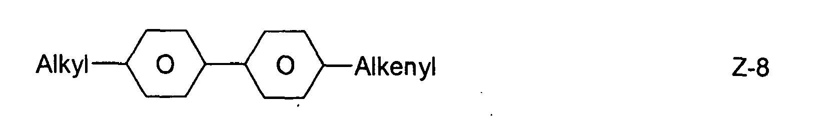 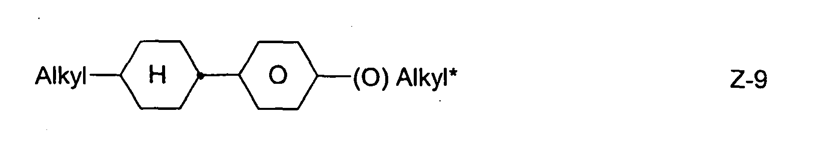 worin
R^{1a} und R^{2a} jeweils unabhängig voneinander H, CH₃, C₂H₅ oder n-C₃H₇,
R⁰ n-Alkyl, Oxaalkyl, Fluoralkyl oder Alkenyl mit jeweils bis zu 9 C-Atomen,
Alkyl, Alkyl* einen unsubstituierten n-Alkylrest mit 1 bis 7 C-Atomen, und
Alkenyl einen unsubstituierten Alkenylrest mit 2-7 C-Atomen
bedeutet.

6. Flüssigkristallines Medium gemäß mindestens einem der Ansprüche 1 bis 5, **dadurch gekennzeichnet, dass** es zusätzlich eine oder mehrere Verbindungen ausgewählt aus der Gruppe bestehend aus den allgemeinen Formeln II, III, IV, V und VI enthält: 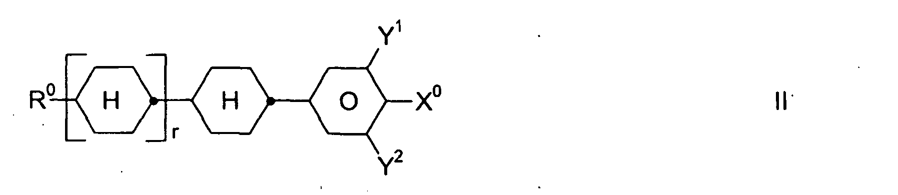 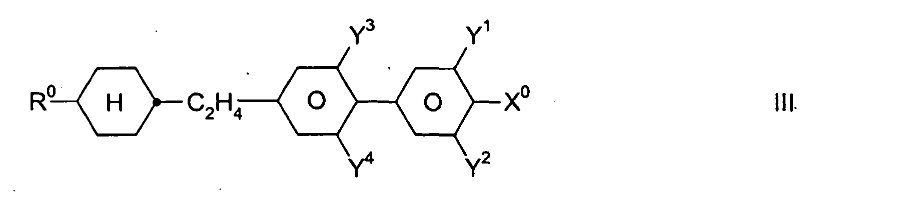 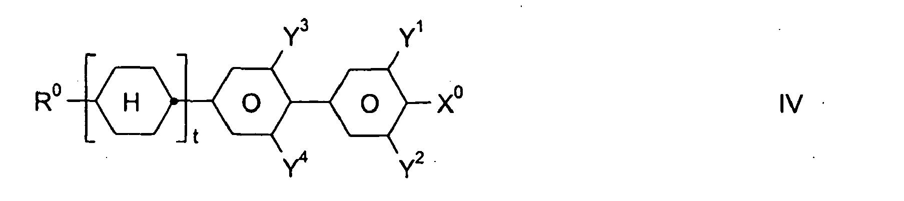 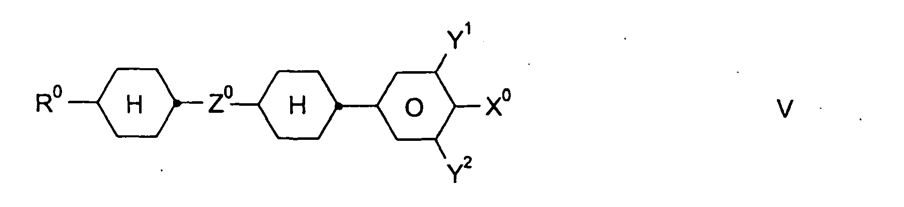 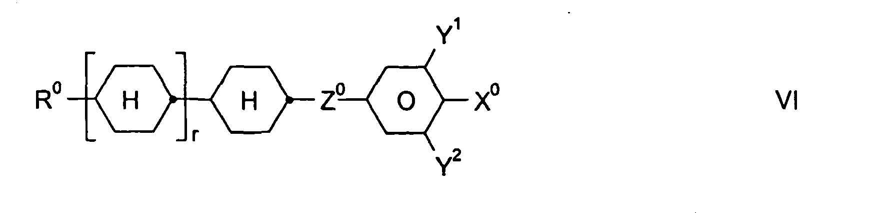 worin
R⁰ n-Alkyl, Oxaalkyl, Fluoralkyl oder Alkenyl mit jeweils bis zu 9 C-Atomen,
X⁰ F, Cl, halogeniertes Alkyl, halogeniertes Alkenyl, halogeniertes Alkenyloxy oder halogeniertes Alkoxy mit bis zu 6 C-Atomen,
Z⁰ -C₂F₄-, -CF=CF-, -C₂H₄-, -(CH₂)₄-, -OCH₂-, -CH₂O-, -CF₂O- oder -OCF₂-,
Y¹ bis Y⁴ jeweils unabhängig voneinander H oder F,
r 0 oder 1, und
t 0, 1 oder 2
bedeutet.

7. Flüssigkristallines Medium gemäß mindestens einem der Ansprüche 1 bis 6, **dadurch gekennzeichnet, dass** es zusätzlich eine oder mehrere Verbindungen ausgewählt aus der Gruppe bestehend aus den allgemeinen Formeln XIV und XV enthält: 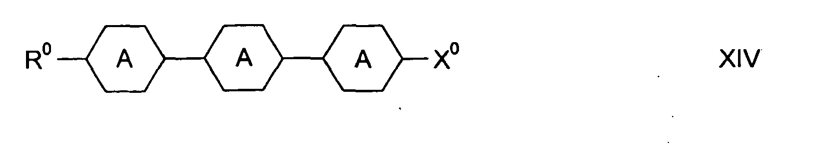 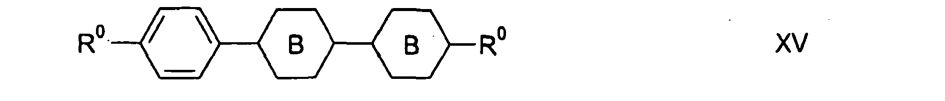 worin
R⁰ n-Alkyl, Oxaalkyl, Fluoralkyl oder Alkenyl mit jeweils bis zu 9 C-Atomen,
X⁰ F, Cl, halogeniertes Alkyl, halogeniertes Alkenyl, halogeniertes Alkenyloxy oder halogeniertes Alkoxy mit bis zu 6 C-Atomen,
Ring A und B, unabhängig voneinander, 1,4-Phenylen mit 0, 1, oder 2 Fluor substituiert, und
wobei je mindestens einer der 1,4-Phenylenringe ein- oder mehrfach durch Fluoratome substituiert ist,
bedeutet.

8. Flüssigkristallines Medium gemäß mindestens einem der Ansprüche 1 bis 7, **dadurch gekennzeichnet, dass** es zusätzlich eine oder mehrere Verbindungen der Formeln E-a bis E-d, 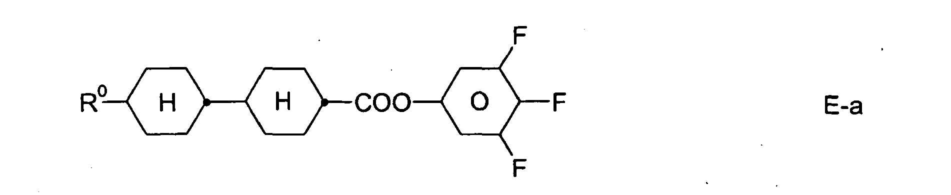 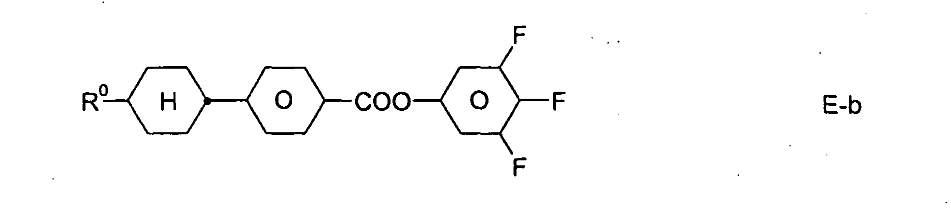 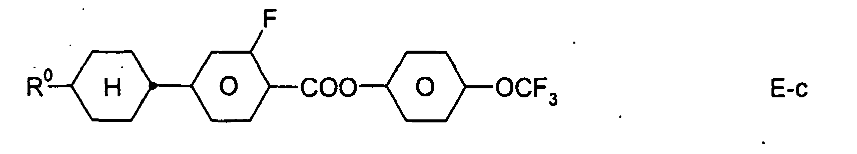 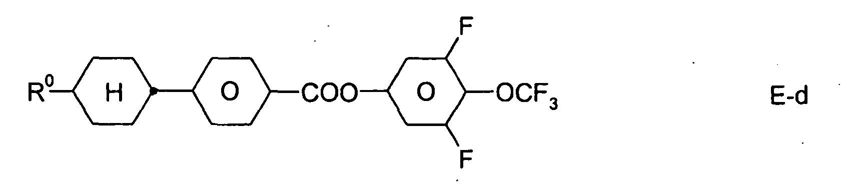 worin
R⁰ die in Anspruch 1 angegebenen Bedeutungen hat,
enthält.

9. Flüssigkristallines Medium gemäß mindestens einem der Ansprüche 1 bis 8, **dadurch gekennzeichnet, dass** es eine oder mehrere Verbindungen der Formeln IIa bis IIg, 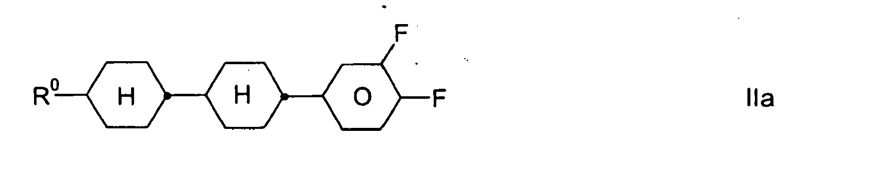 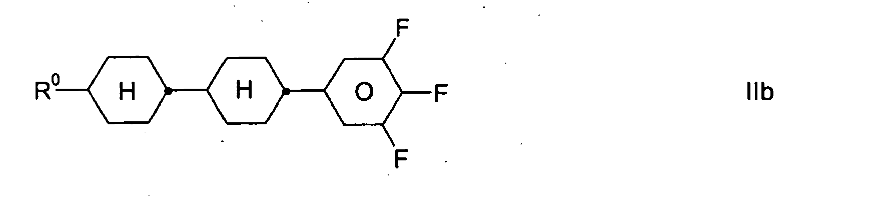 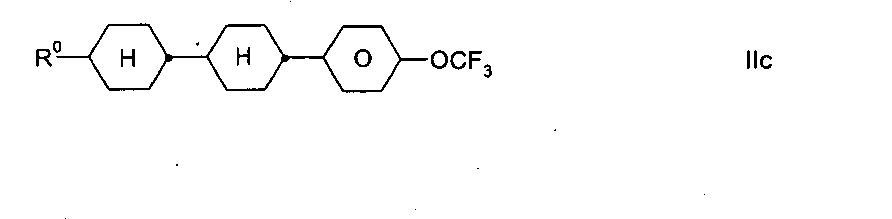 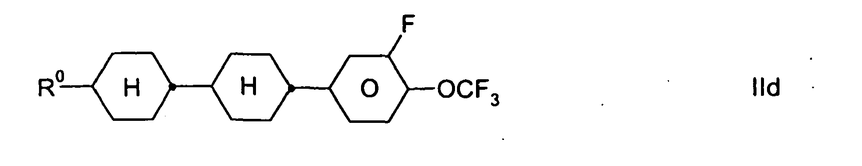 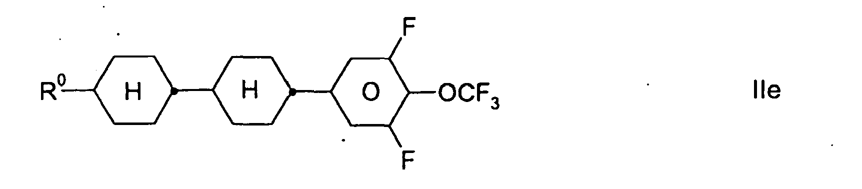 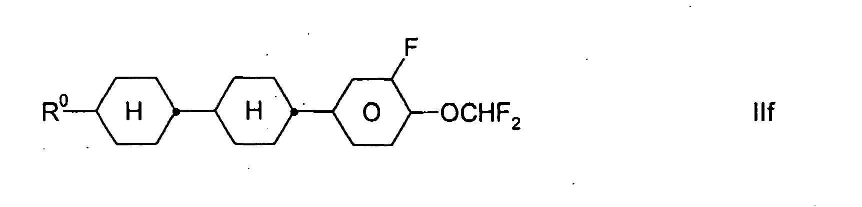 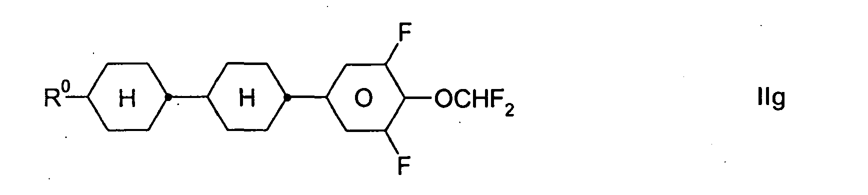 worin
R⁰ die in Anspruch 1 angegebenen Bedeutungen hat,
enthält.

10. Flüssigkristallines Medium gemäß mindestens einem der Ansprüche 1 bis 9, **dadurch gekennzeichnet, dass** es ein oder mehrere Verbindungen der Formeln O1 und O2, 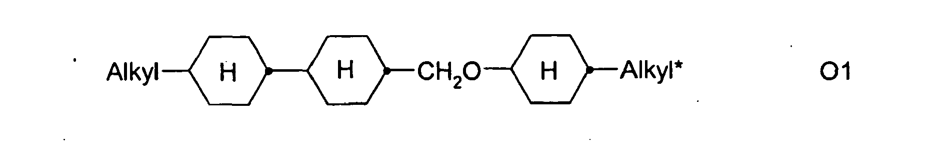 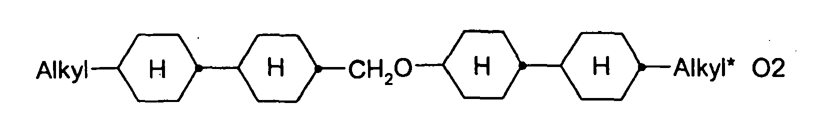 worin
Alkyl und Alkyl* jeweils unabhängig voneinander eine geradkettige oder verzweigte Alkylgruppe mit 1-7 Kohlenstoffatomen bedeuten,
enthält.

11. Flüssigkristallines Medium gemäß mindestens einem der Ansprüche 1 bis 10, **dadurch gekennzeichnet, dass** es ein oder mehrere Dioxan-Verbindungen der Formeln D1 und/oder D2, 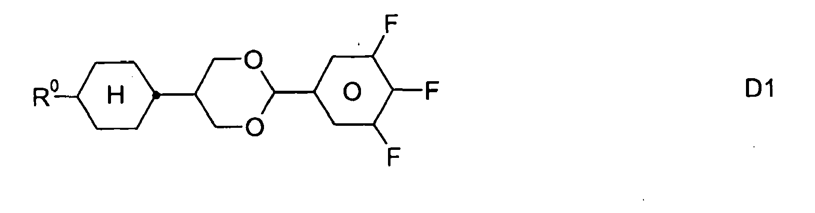 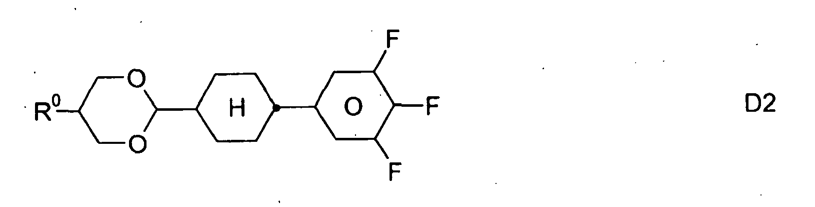 worin
R⁰ die in Anspruch 3 angegebenen Bedeutungen hat,
enthält.

12. Verwendung des flüssigkristallinen Mediums gemäß mindestens einem der Ansprüche 1 bis 11 für elektrooptische Zwecke.

13. Elektrooptische Flüssigkristallanzeige enthaltend ein flüssigkristallines Medium gemäß mindestens einem der Ansprüche 1 bis 11.

14. Verbindungen der Formel Ic 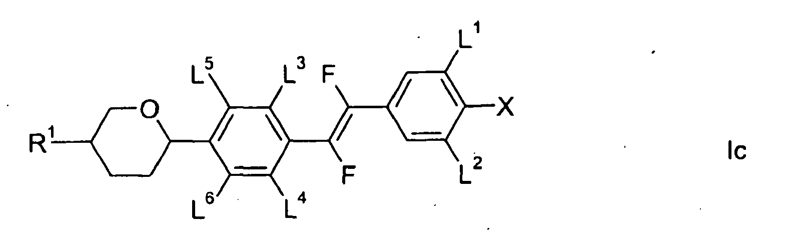 worin
R¹, L¹, L², L³, L⁴, L⁵, L⁶ und X die in Anspruch 1 angegebene Bedeutung haben.

## Claims

1. Liquid-crystalline medium of positive dielectric anisotropy based on a mixture of compounds, **characterised in that** it comprises one or more compounds of the formula I 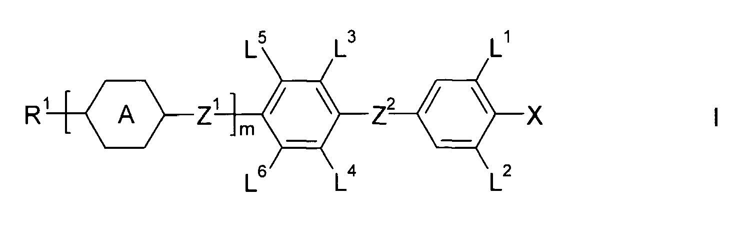 in which
R¹ denotes a halogenated or unsubstituted alkyl or alkoxy radical having 1 to 15 C atoms, where, in addition, one or more CH₂ groups in these radicals may each be replaced, independently of one another, by -C≡C-, -CH=CH-, -O-, -CO-O- or -O-CO- in such a way that O atoms are not linked directly to one another,
A denotes a ring system of the formulae 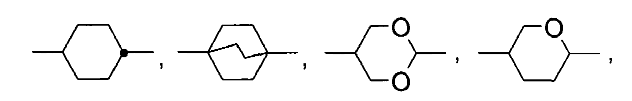 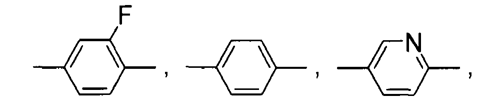 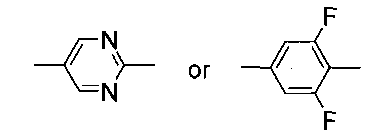 pointing to the left or right,
Z¹, Z² denote a single bond, -C≡C-, -CF=CF-, -CH=CH-, -CF₂O- or -CH₂CH₂-, where at least one group from Z¹ and Z² denotes the group -CF=CF-,
X denotes F, Cl, CN, SF₅ or a halogenated or unsubstituted alkyl or alkoxy radical having 1 to 15 C atoms, where, in addition, one or more CH₂ groups in these radicals may each be replaced, independently of one another, by -C≡C-, -CH=CH-, -O-, -CO-O- or -O-CO- in such a way that O atoms are not linked directly to one another,
L¹, L², L³, L⁴, L⁵ and L⁶ each, independently of one another, denote H or F, and
m denotes 0 or 1,
and
additionally one or more selected compounds of the general formulae K-1 to K-12: 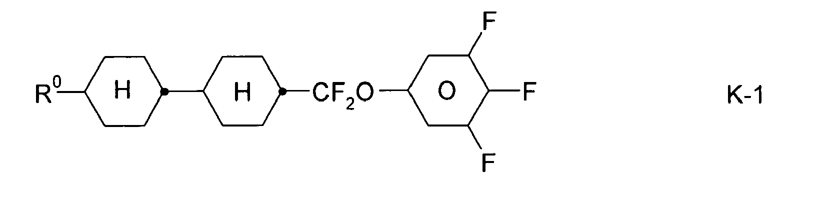 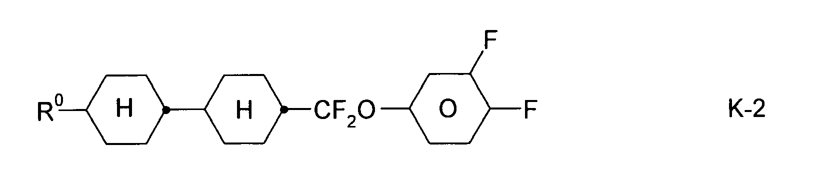 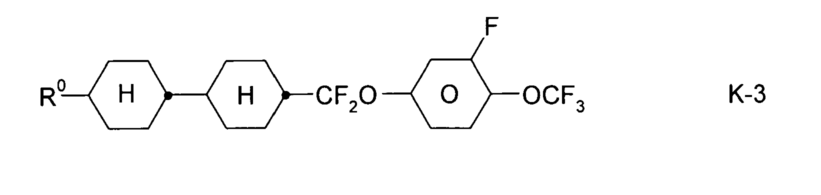 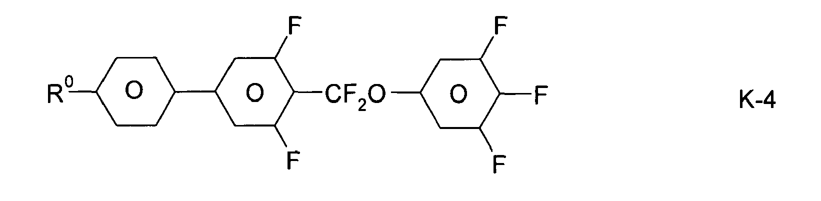 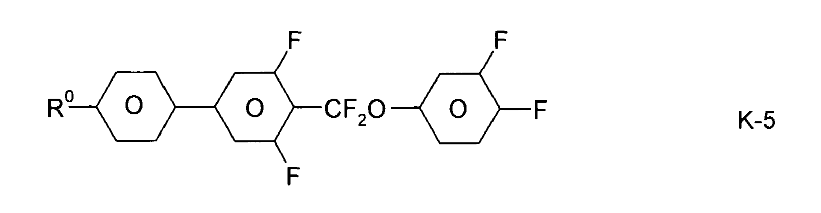 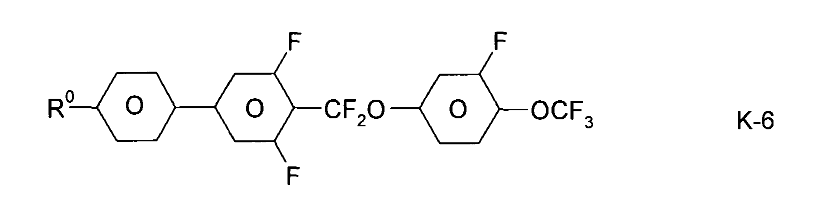 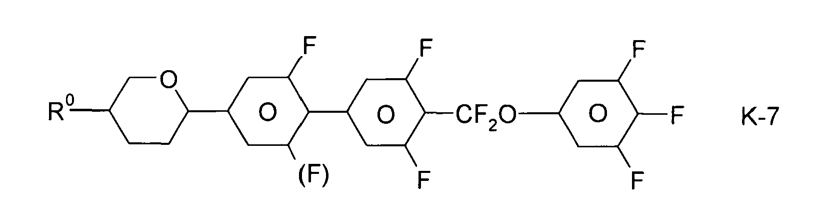 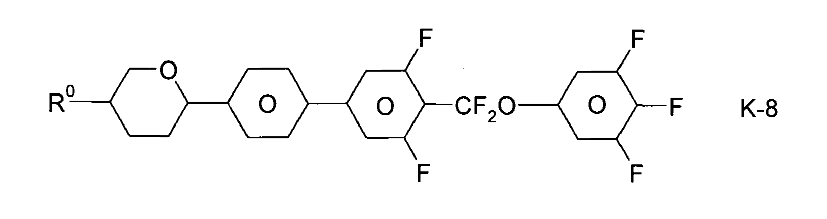 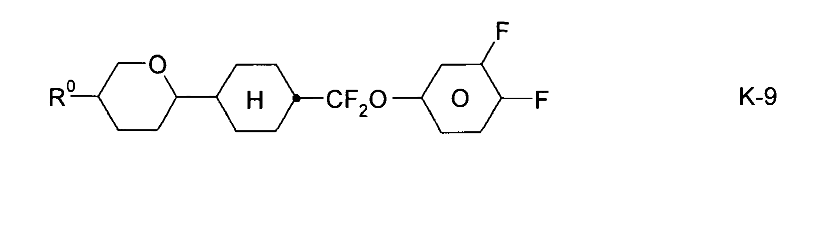 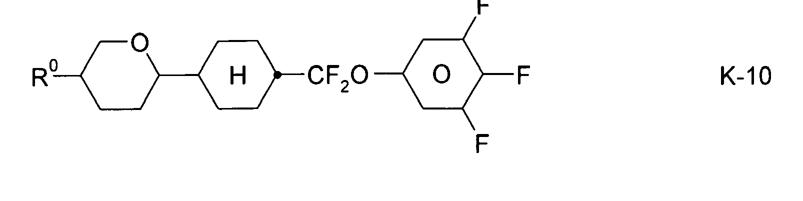 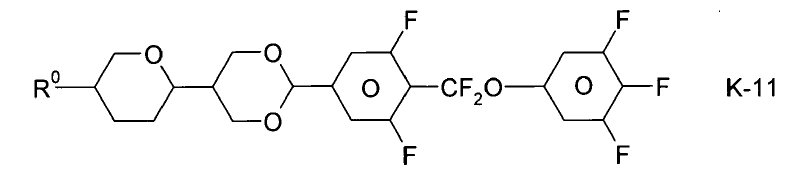 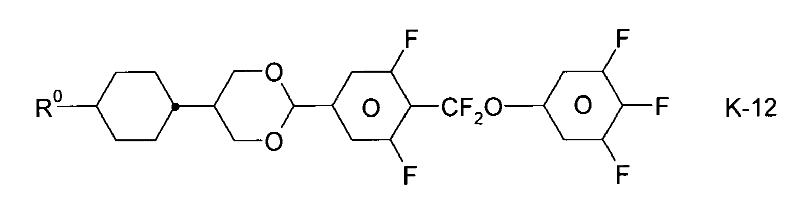 in which
R⁰ denotes n-alkyl, oxaalkyl, fluoroalkyl or alkenyl, each having up to 9 C atoms.

2. Liquid-crystalline medium according to Claim 1, **characterised in that**
X denotes F, Cl, CN, or a halogenated alkyl or alkoxy radical having 1 to 15 C atoms, where, in addition, one or more CH₂ groups in these radicals may each be replaced, independently of one another, by -C≡C-, -CH=CH-, -O-, -CO-O- or -O-CO- in such a way that O atoms are not linked directly to one another.

3. Liquid-crystalline medium according to Claim 1 or 2, **characterised in that**
m denotes 1.

4. Liquid-crystalline medium according to at least one of Claims 1 to 3, **characterised in that** it comprises one, two or more compounds of the formulae I-1 to I-30: 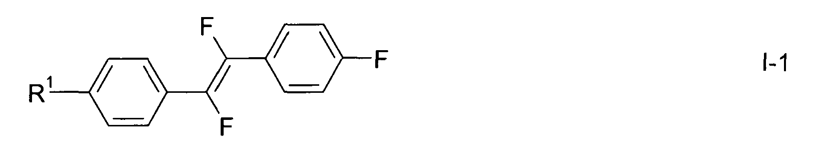 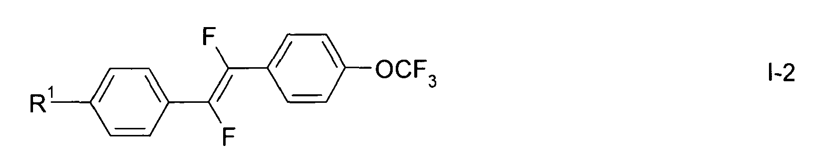 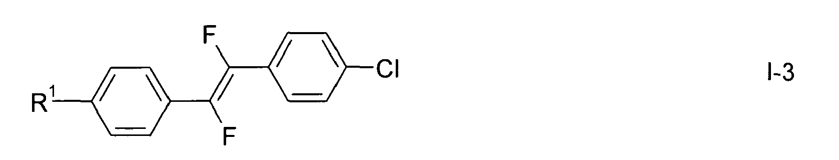 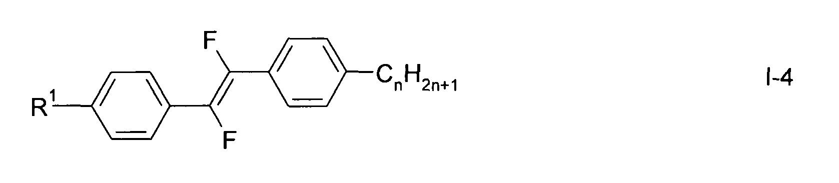 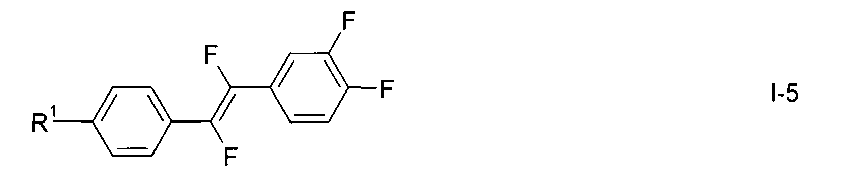 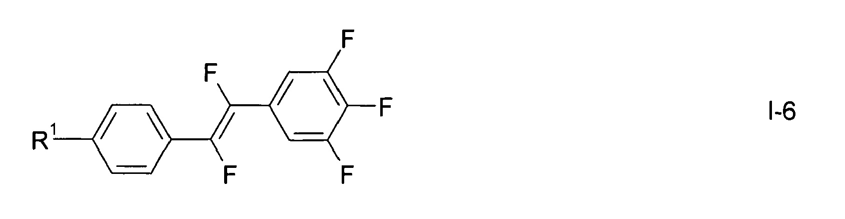 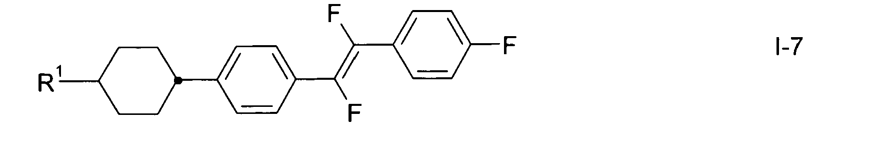 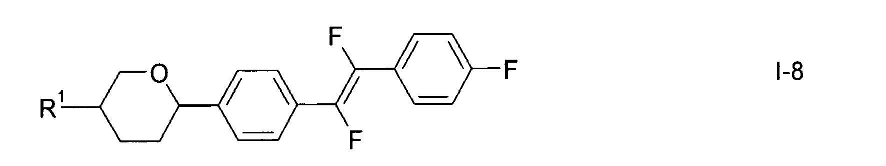 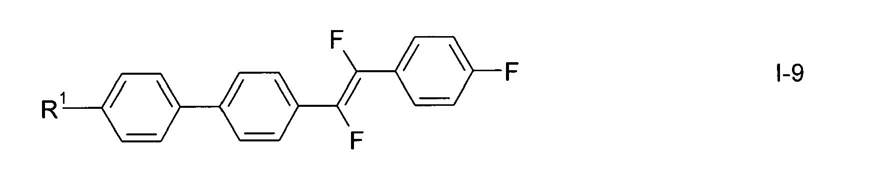 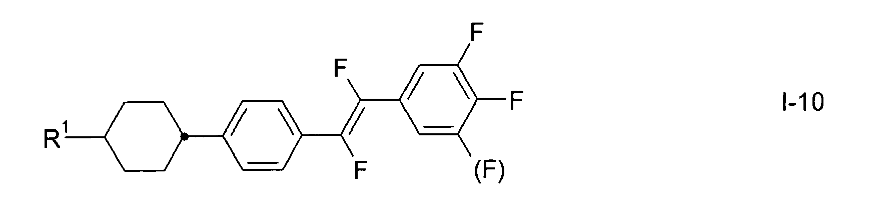 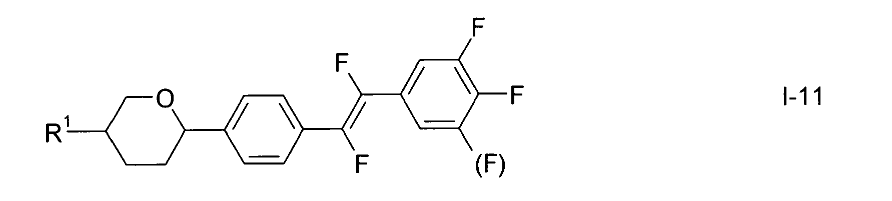 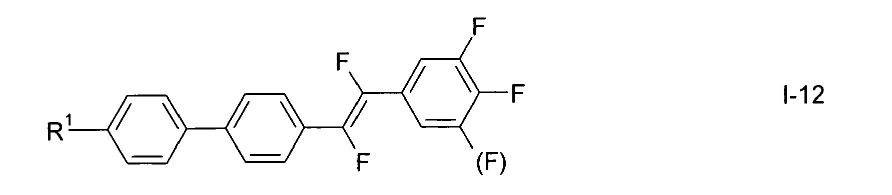 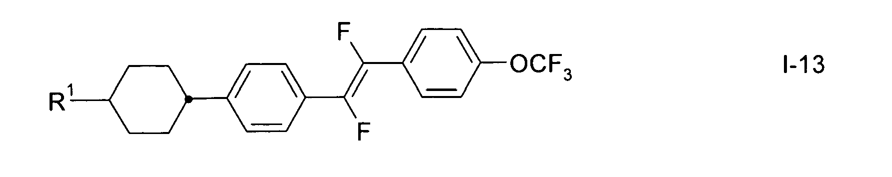 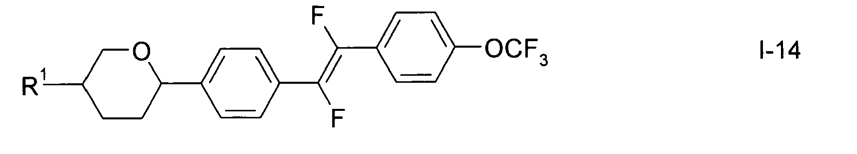 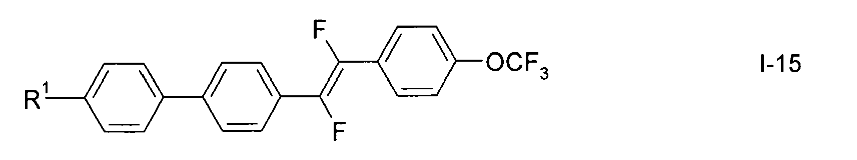 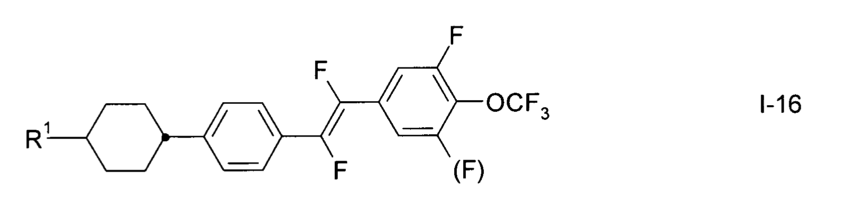 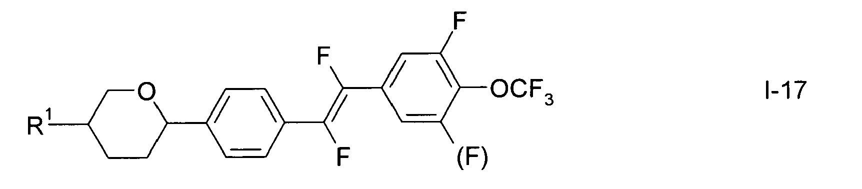 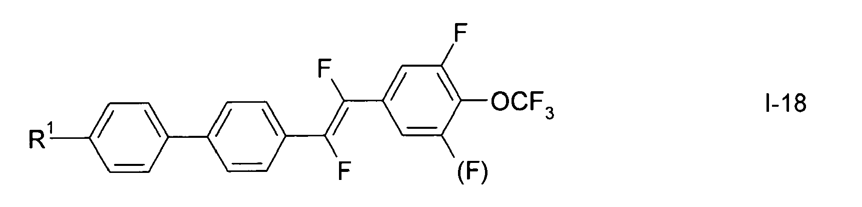 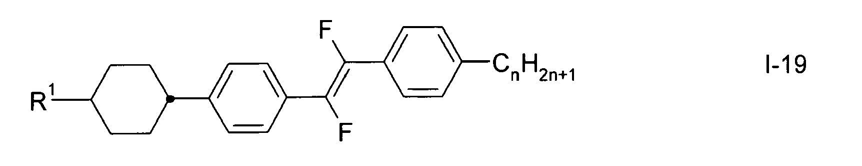 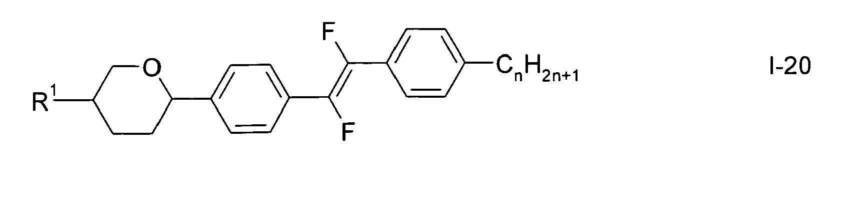 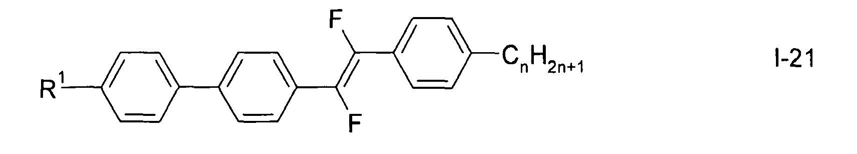 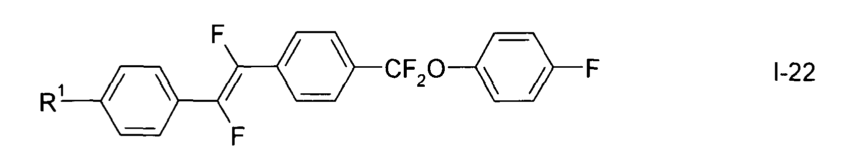 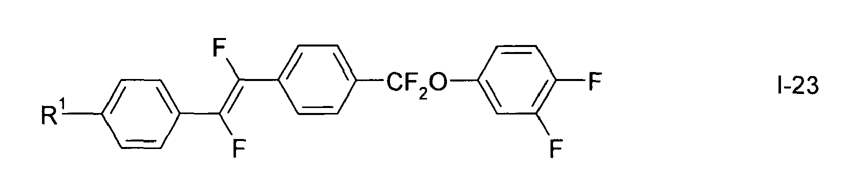 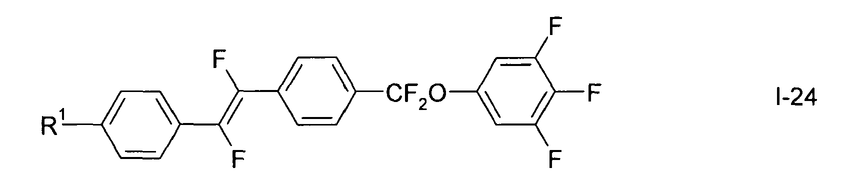 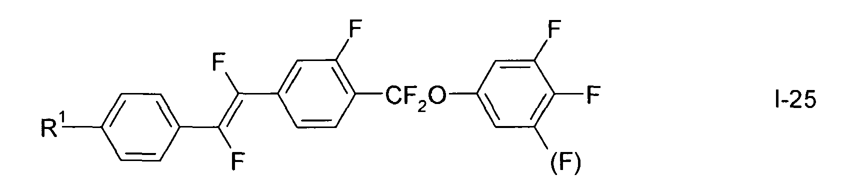 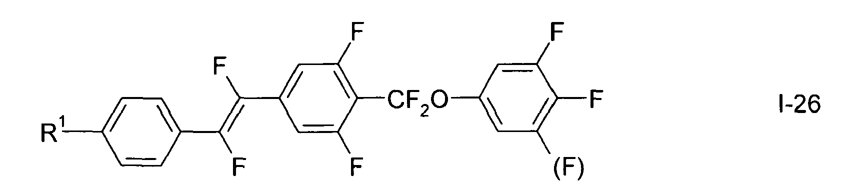 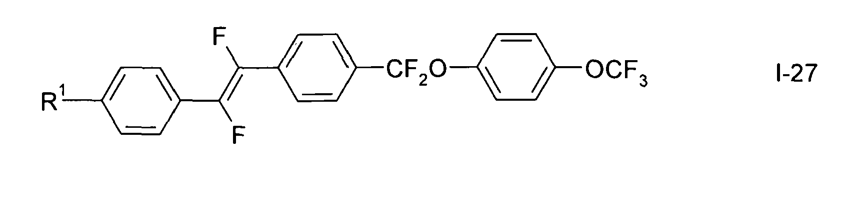 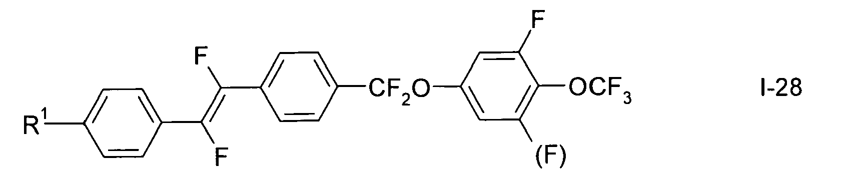 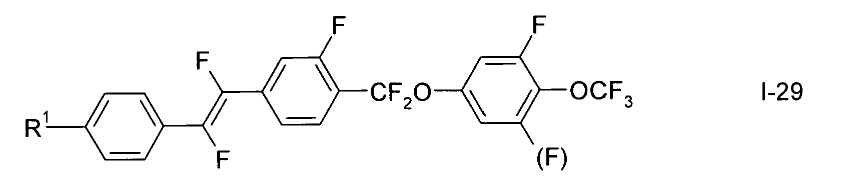 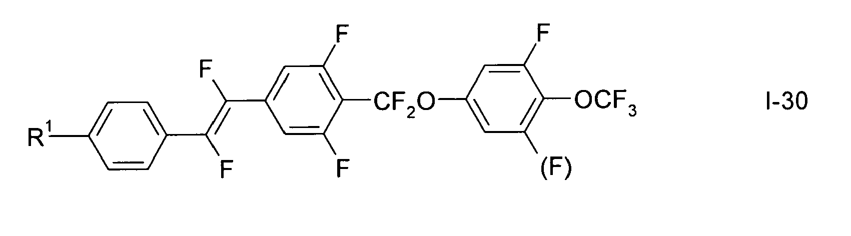 in which
R¹ has the meaning indicated in Claim 1 and n stands for 1, 2, 3, 4, 5, 6, 7 or 8.

5. Liquid-crystalline medium according to at least one of Claims 1 to 4, **characterised in that** it additionally comprises one or more selected compounds of the general formulae Z-1 to Z-9: 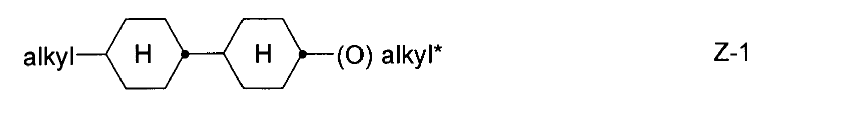 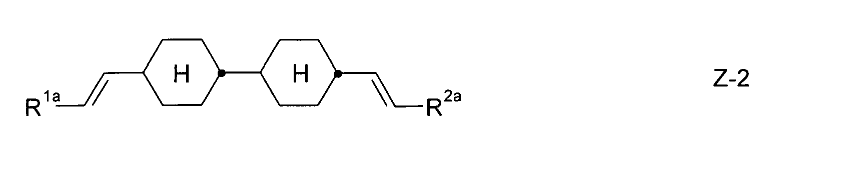 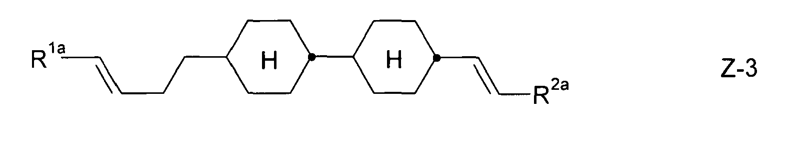 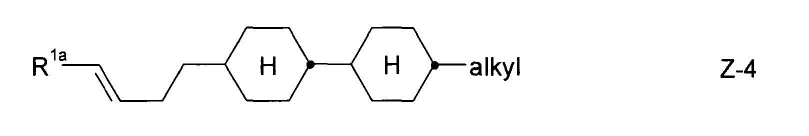 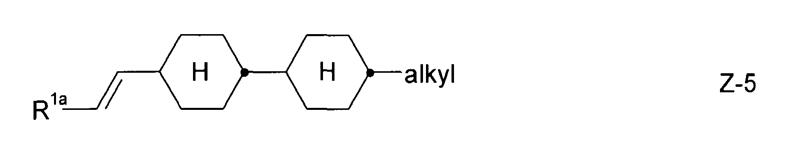 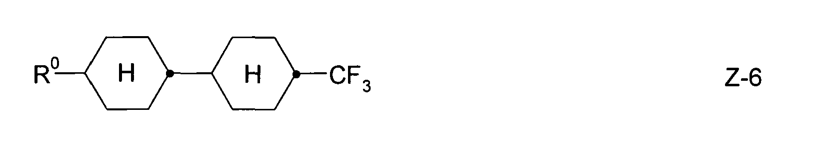 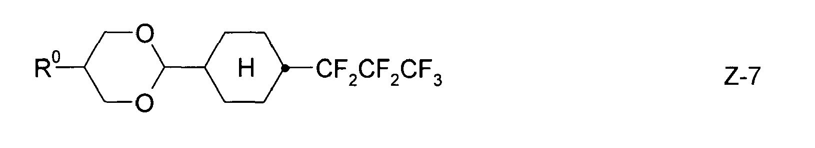 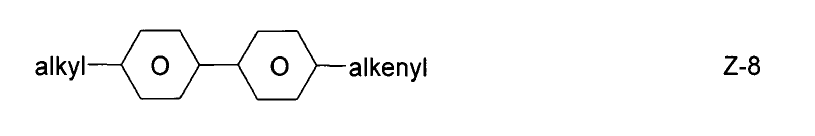 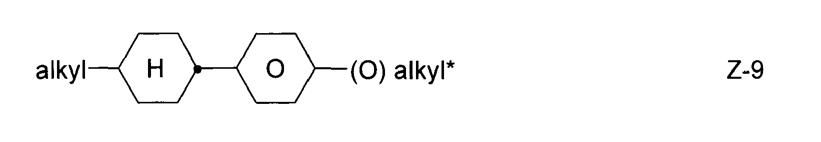 in which
R^{1a} and R^{2a} each, independently of one another, denote H, CH₃, C₂H₅ or n-C₃H₇,
R⁰ denotes n-alkyl, oxaalkyl, fluoroalkyl or alkenyl, each having up to 9 C atoms,
alkyl, alkyl* denote an unsubstituted n-alkyl radical having 1 to 7 C atoms, and
alkenyl denotes an unsubstituted alkenyl radical having 2-7 C atoms.

6. Liquid-crystalline medium according to at least one of Claims 1 to 5, **characterised in that** it additionally comprises one or more compounds selected from the group consisting of the general formulae II, III, IV, V and VI: 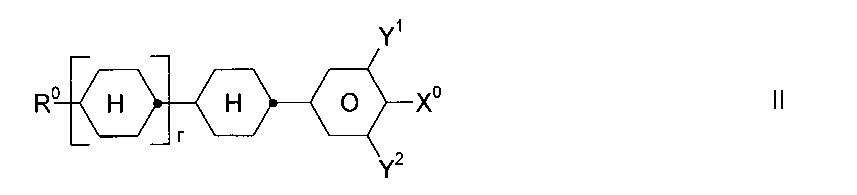 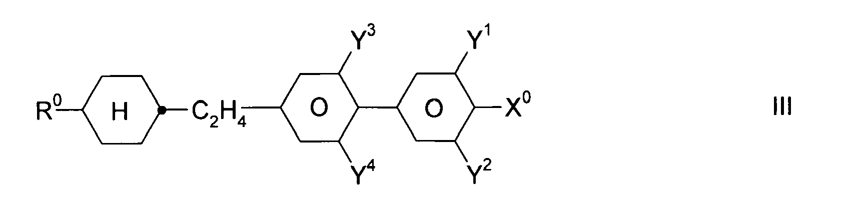 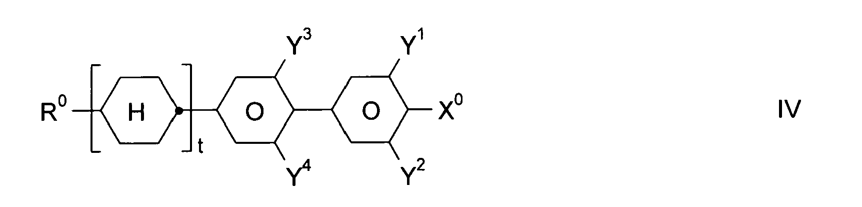 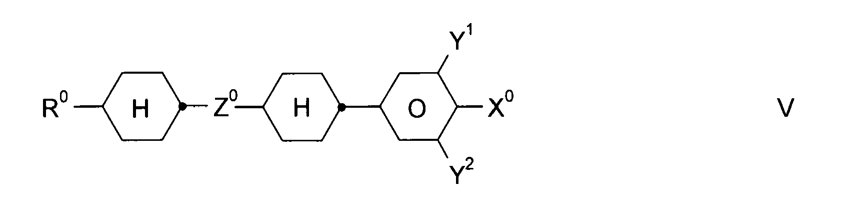 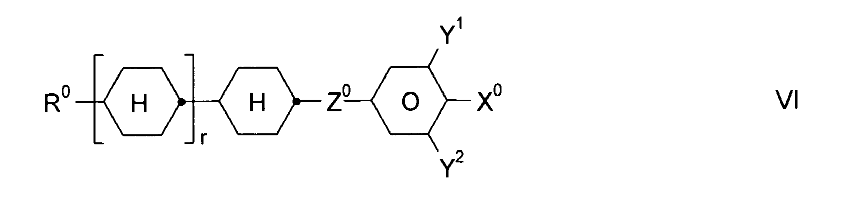 in which
R⁰ denotes n-alkyl, oxaalkyl, fluoroalkyl or alkenyl, each having up to 9 C atoms,
X⁰ denotes F, Cl, halogenated alkyl, halogenated alkenyl, halogenated alkenyloxy or halogenated alkoxy having up to 6 C atoms,
Z⁰ denotes -C₂F₄-, -CF=CF-, -C₂H₄-, -(CH₂)₄-, -OCH₂-, -CH₂O-, -CF₂O- or -OCF₂-,
Y¹ to Y⁴ each, independently of one another, denote H or F,
r denotes 0 or 1, and
t denotes 0, 1 or 2.

7. Liquid-crystalline medium according to at least one of Claims 1 to 6, **characterised in that** it additionally comprises one or more compounds selected from the group consisting of the general formulae XIV and XV: 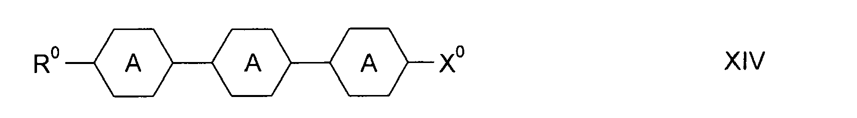 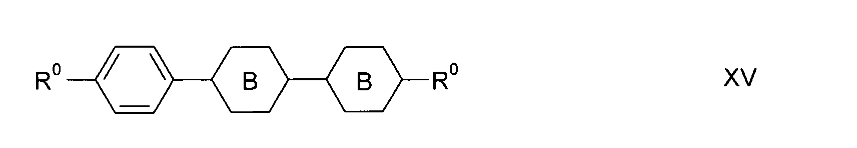 in which
R⁰ denotes n-alkyl, oxaalkyl, fluoroalkyl or alkenyl, each having up to 9 C atoms,
X⁰ denotes F, Cl, halogenated alkyl, halogenated alkenyl, halogenated alkenyloxy or halogenated alkoxy having up to 6 C atoms,
rings A and B, independently of one another, denote 1,4-phenylene which is substituted by 0, 1 or 2 fluorine, and
where in each case at least one of the 1,4-phenylene rings is mono- or polysubstituted by fluorine atoms.

8. Liquid-crystalline medium according to at least one of Claims 1 to 7, **characterised in that** it additionally comprises one or more compounds of the formulae E-a to E-d: 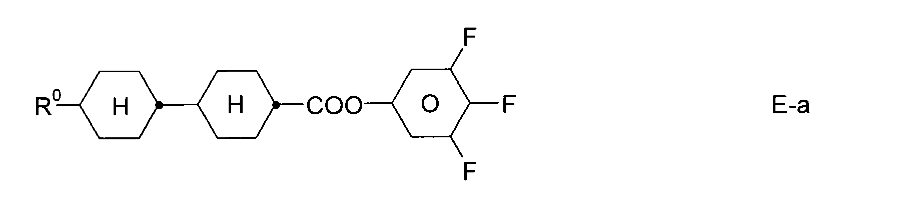 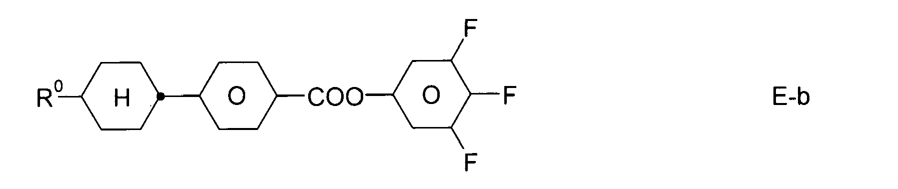 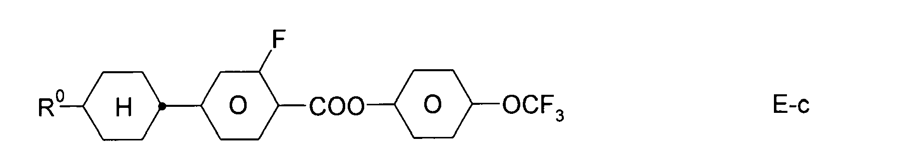 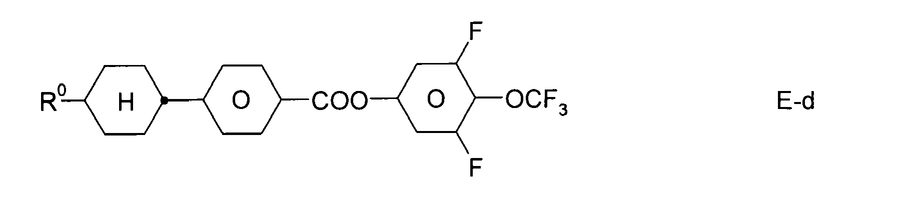 in which
R⁰ has the meanings indicated in Claim 1.

9. Liquid-crystalline medium according to at least one of Claims 1 to 8, **characterised in that** it comprises one or more compounds of the formulae IIa to IIg: 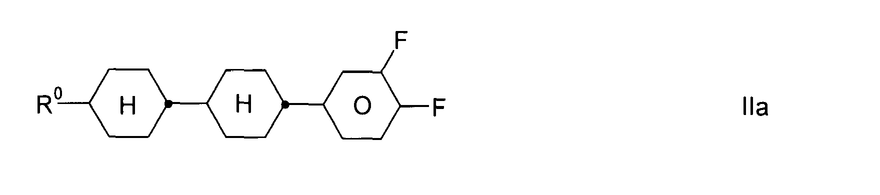 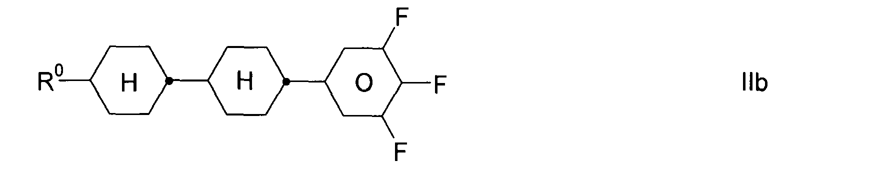 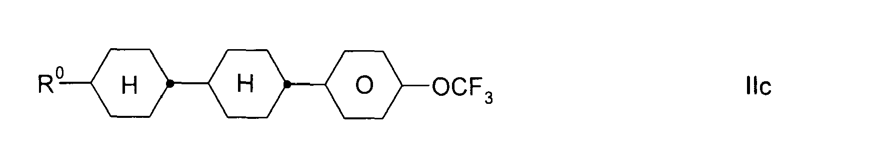 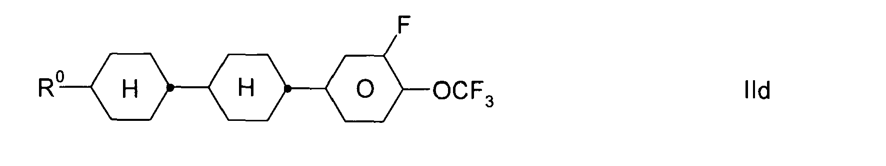 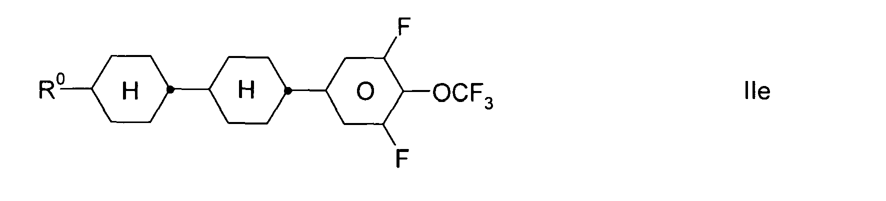 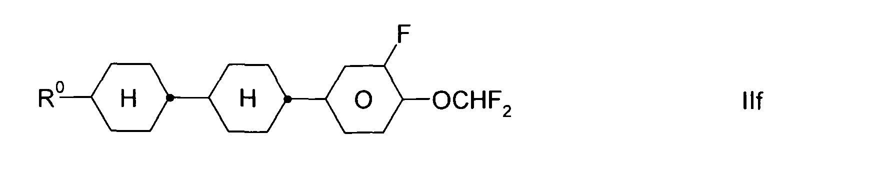 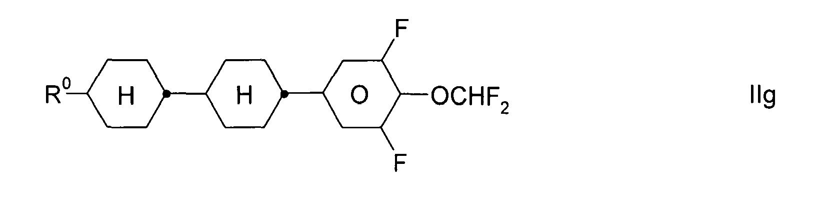 in which
R⁰ has the meanings indicated in Claim 1.

10. Liquid-crystalline medium according to at least one of Claims 1 to 9, **characterised in that** it comprises one or more compounds of the formulae O1 and O2: 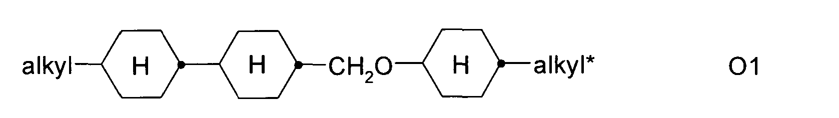 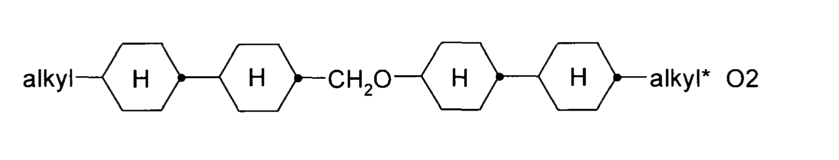 in which
alkyl and alkyl* each, independently of one another, denote a straight-chain or branched alkyl group having 1-7 carbon atoms.

11. Liquid-crystalline medium according to at least one of Claims 1 to 10, **characterised in that** it comprises one or more dioxane compounds of the formulae D1 and/or D2: 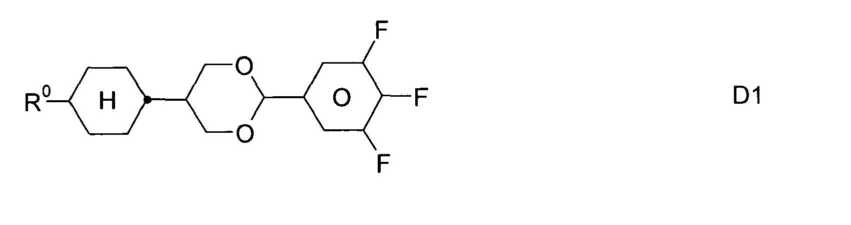 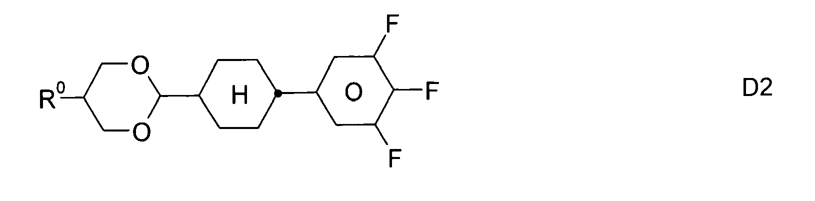 in which
R⁰ has the meanings indicated in Claim 3.

12. Use of the liquid-crystalline medium according to at least one of Claims 1 to 11 for electro-optical purposes.

13. Electro-optical liquid-crystal display containing a liquid-crystalline medium according to at least one of Claims 1 to 11.

14. Compounds of the formula Ic 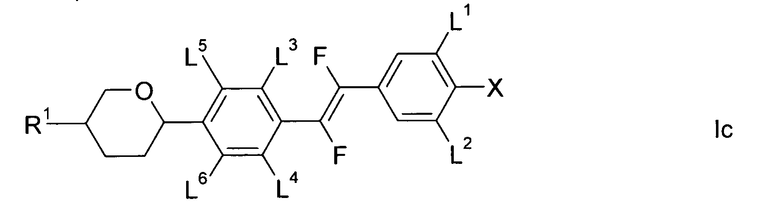 in which
R¹, L¹, L², L³, L⁴, L⁵, L⁶ and X have the meaning indicated in Claim 1.

## Revendications

1. Milieu cristallin liquide d'anisotropie diélectrique positive basé sur un mélange de composés, **caractérisé en ce qu'**il comprend un ou plusieurs composés de la formule I 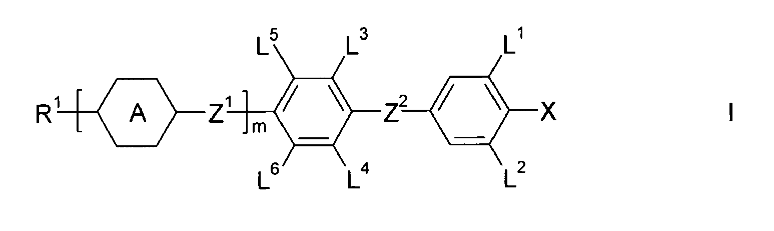 dans laquelle
R¹ représente un radical alkyle ou alcoxy halogéné ou non substitué ayant 1 à 15 atomes de C, dans lequel, en addi- tion, un ou plusieurs groupes CH₂ dans ces radicaux peu- vent chacun être remplacés, indépendamment l'un de l'autre, par -C≡C-, -CH=CH-, -O-, -CO-O- ou -O-CO- de telle sorte que des atomes de O ne soient pas liés directe- ment l'un à l'autre,
A représente un système de cycle des formules 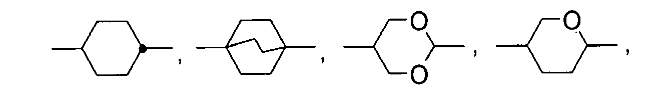 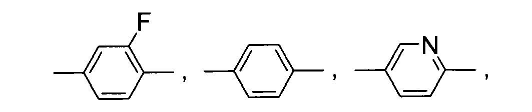 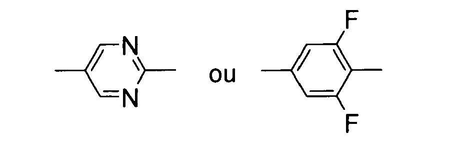 pointant vers la gauche ou la droite,
Z¹, Z² représentent une liaison simple, -C=C-, -CF=CF-, -CH=CH-, -CF₂O- ou -CH₂CH₂-, dans lequel au moins un groupe parmi Z¹ et Z² représente le groupe -CF=CF-,
X représente F, Cl, CN, SF₅ ou un radical alkyle ou alcoxy halogéné ou non substitué ayant 1 à 15 atomes de C, dans lequel, en addition, un ou plusieurs groupes CH₂ dans ces radicaux peuvent chacun être remplacés, indé- pendamment l'un de l'autre, par -C≡C-, -CH=CH-, -O-, -CO-O- ou -O-CO- de telle sorte que des atomes de O ne soient pas liés directement l'un à l'autre,
L¹, L², L³, L⁴, L⁵ et L⁶ chacun, indépendamment l'un de l'autre, représentent H ou F, et
m représente 0 ou 1,
et
additionnellement un ou plusieurs composés choisis parmi les formules générales K-1 à K-12: 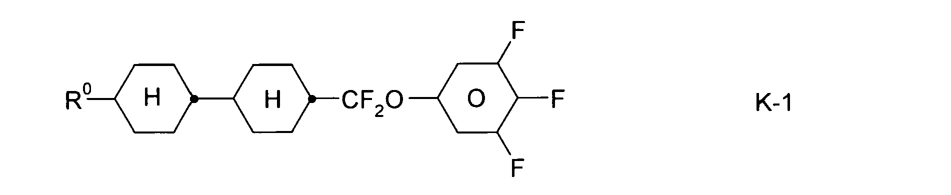 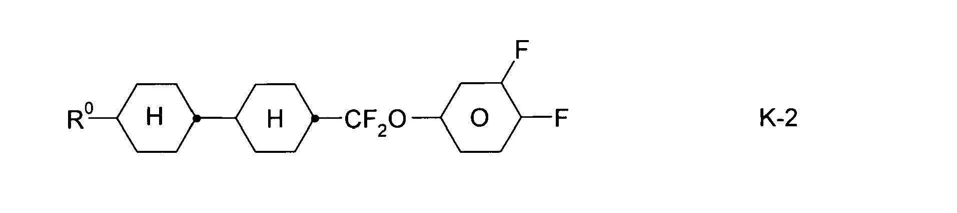 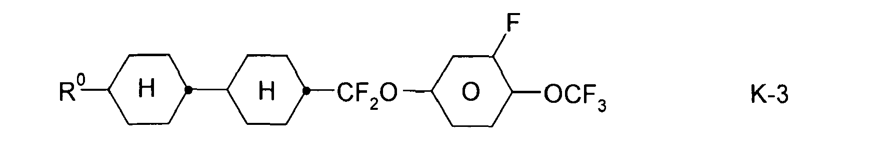 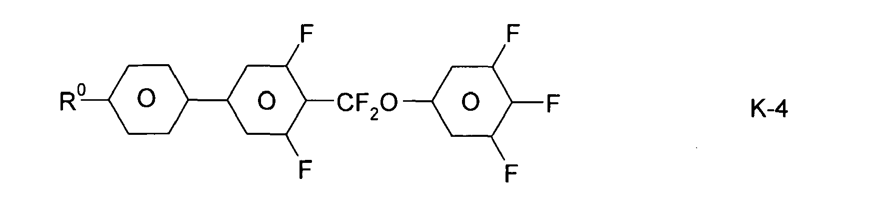 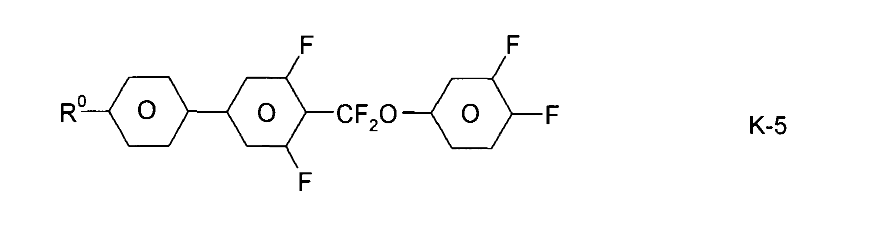 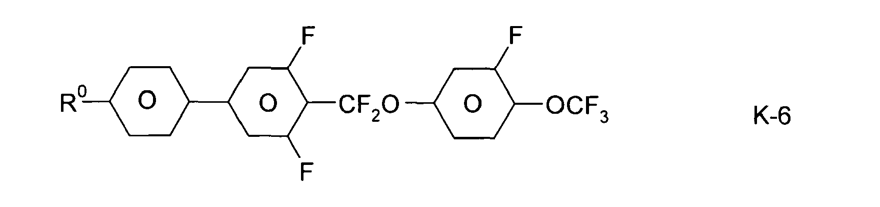 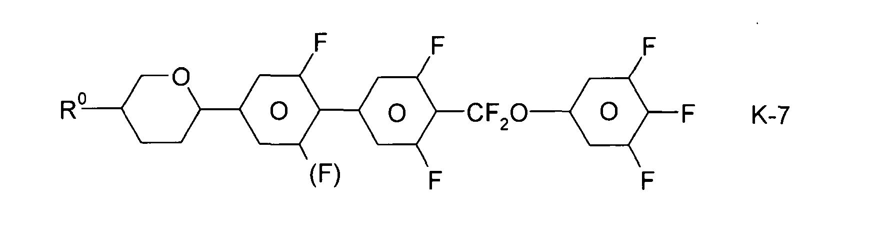 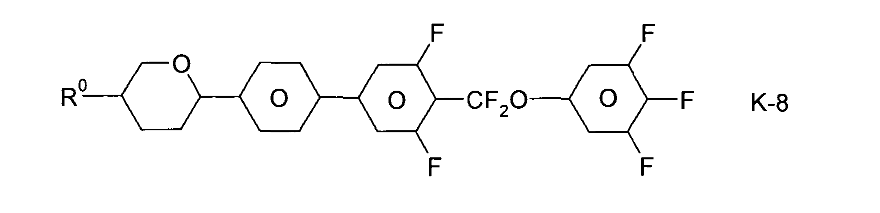 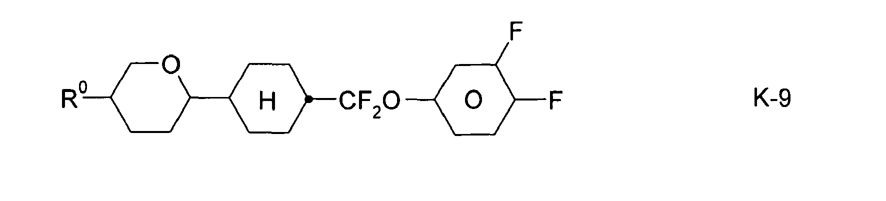 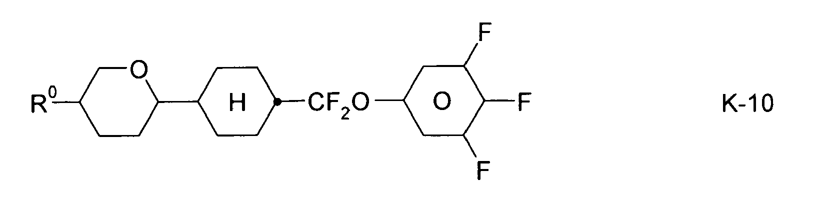 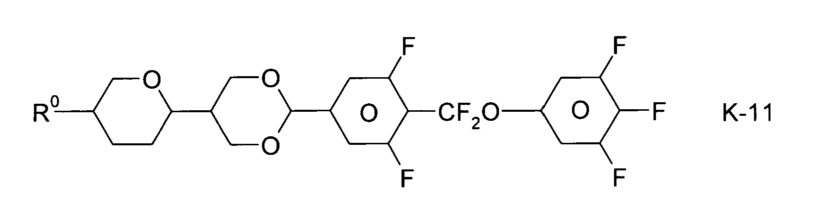 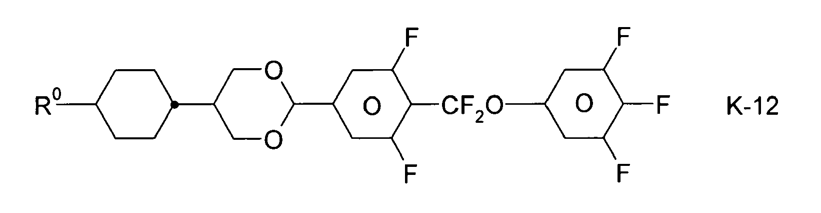 dans lesquelles
R⁰ représente n-alkyle, oxaalkyle, fluoroalkyle ou alkényle, chacun ayant jusqu'à 9 atomes de C.

2. Milieu cristallin liquide selon la revendication 1, **caractérisé en ce que**
X représente F, Cl, CN, ou un radical alkyle ou alcoxy halogéné ayant 1 à 15 atomes de C, dans lequel, en addition, un ou plusieurs groupes CH₂ dans ces radi- caux peuvent chacun être remplacés, indépendamment l'un de l'autre, par -C≡C-, -CH=CH-, -O-, -CO-O- ou -O-CO- de telle sorte que des atomes de O ne soient pas liés directement l'un à l'autre.

3. Milieu cristallin liquide selon la revendication 1 ou 2, **caractérisé en ce que**
m représente 1.

4. Milieu cristallin liquide selon au moins l'une des revendications 1 à 3, **caractérisé en ce qu'**il comprend un, deux ou plus de deux composés des formules I-1 à I-30: 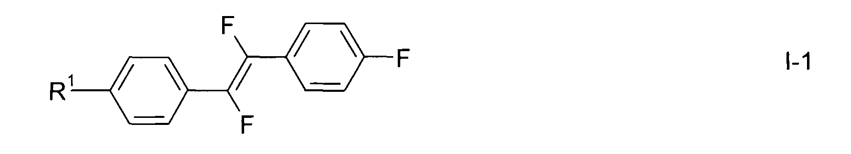 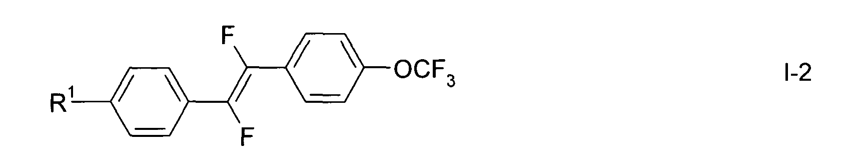 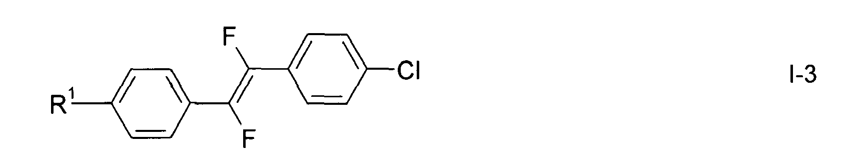 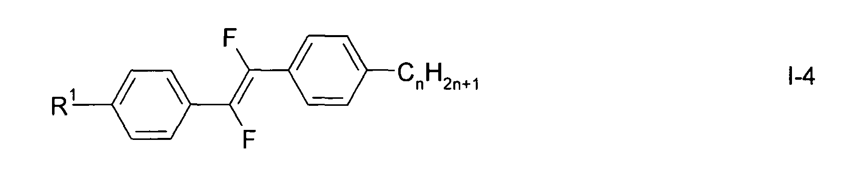 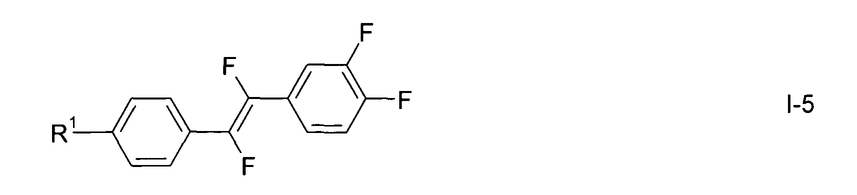 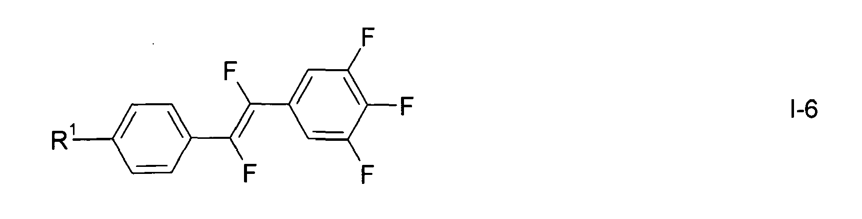 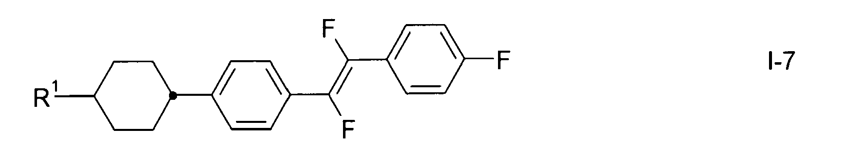 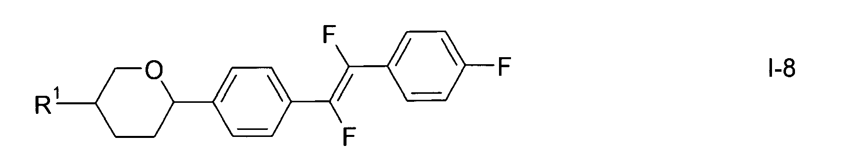 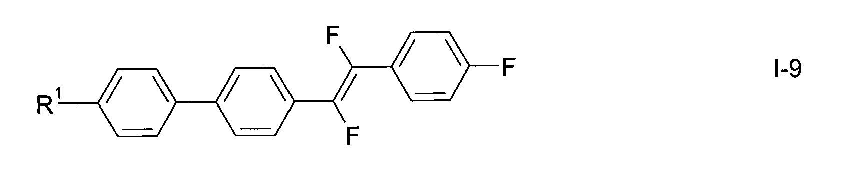 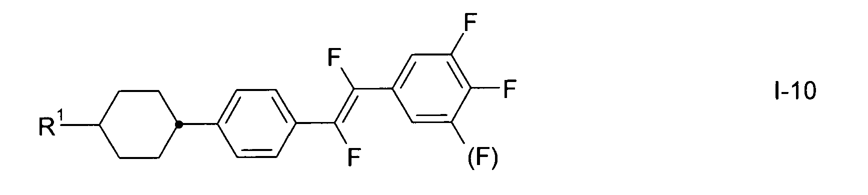 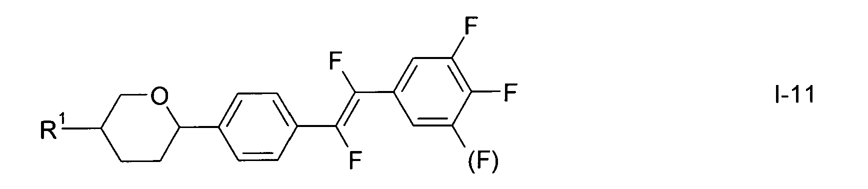 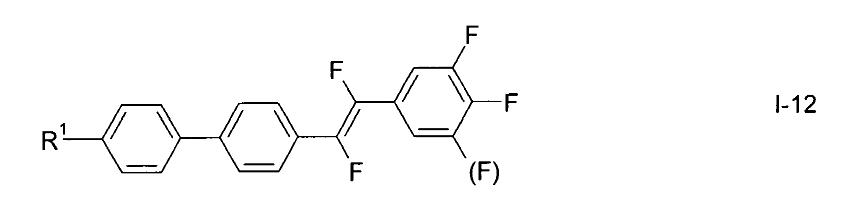 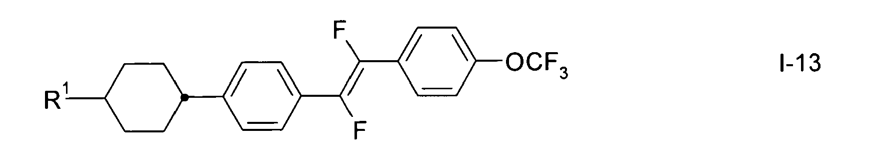 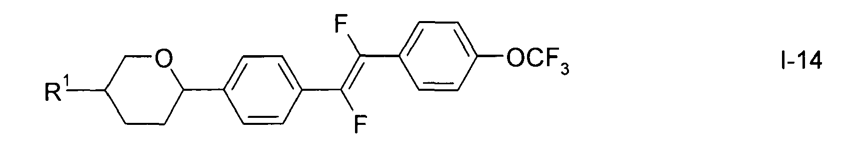 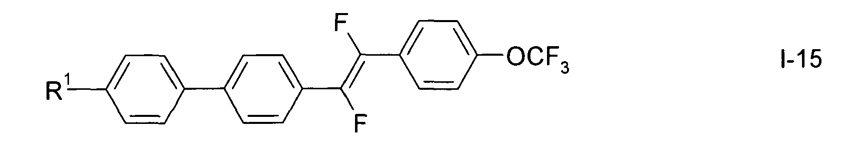 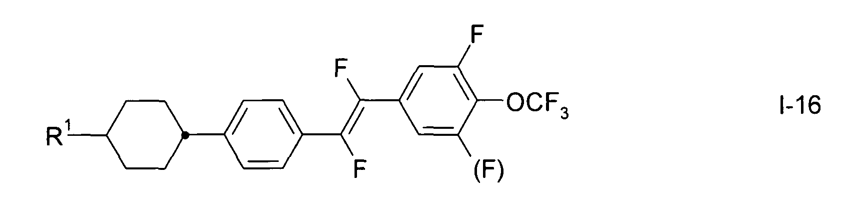 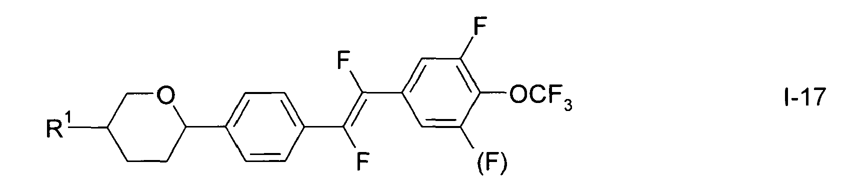 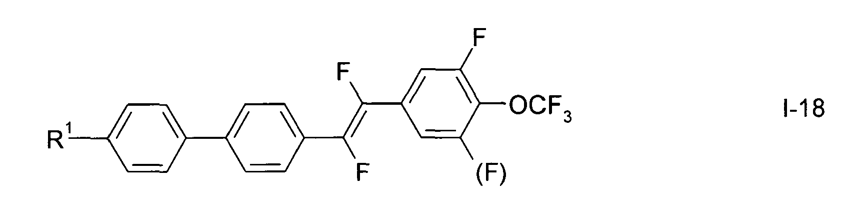 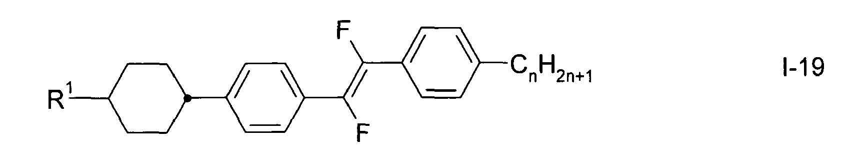 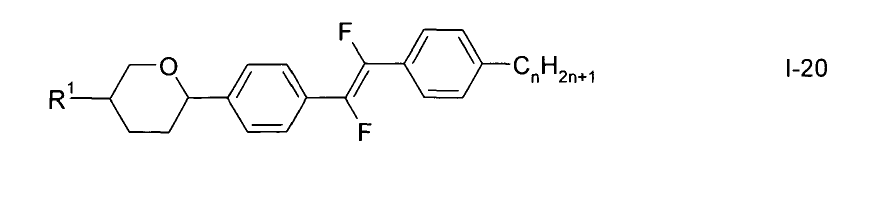 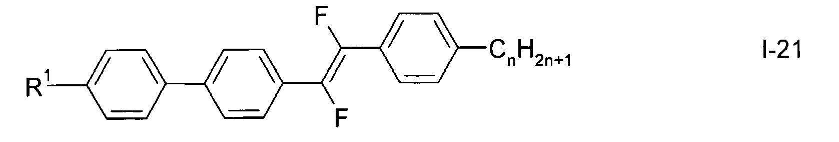 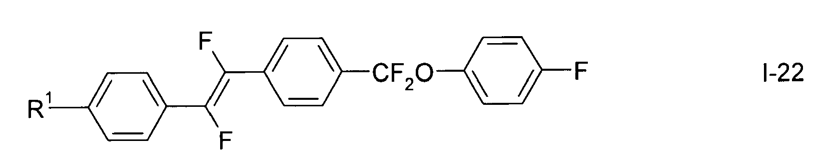 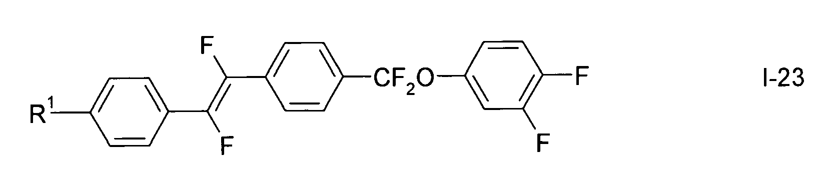 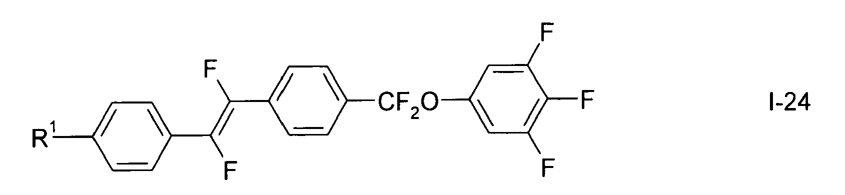 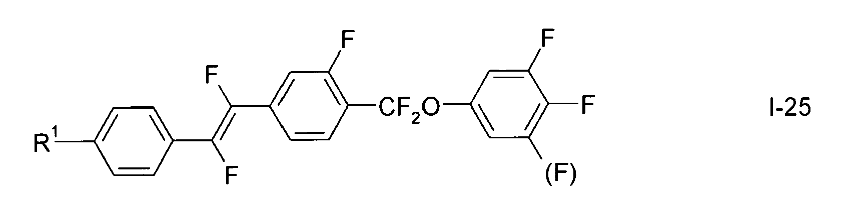 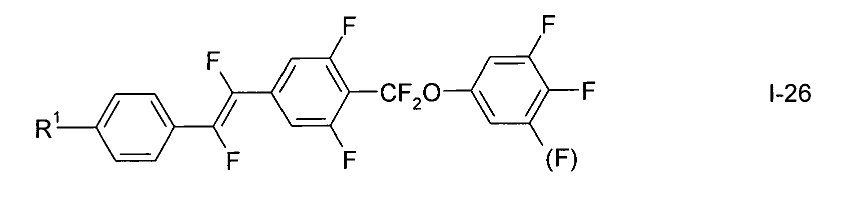 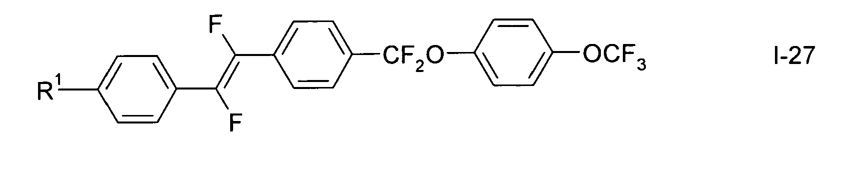 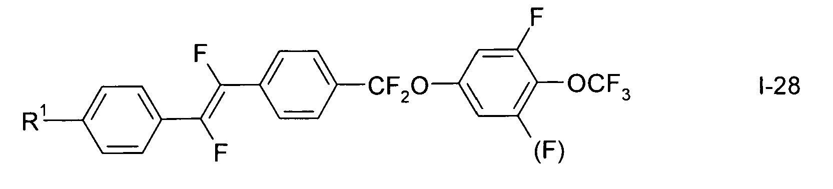 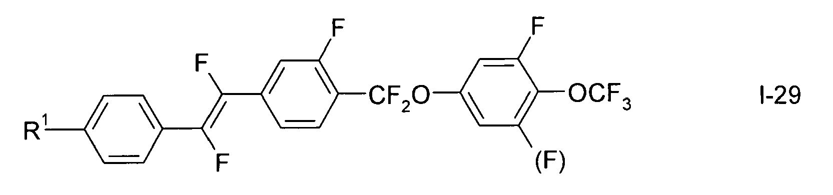 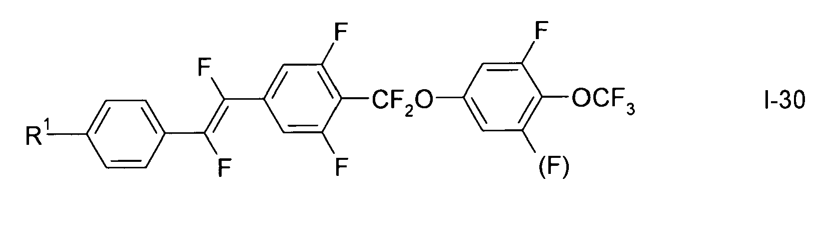 dans lesquelles
R¹ a la signification indiquée dans la revendication 1 et n représente 1, 2, 3, 4, 5, 6, 7 ou 8.

5. Milieu cristallin liquide selon au moins l'une des revendications 1 à 4, **caractérisé en ce qu'**il comprend additionnellement un ou plusieurs composés choisis parmi les formules générales Z-1 à Z-9: 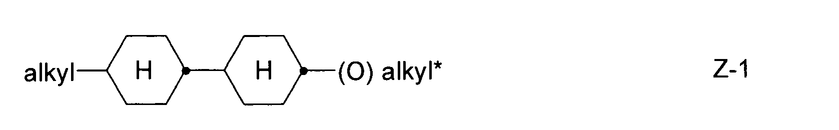 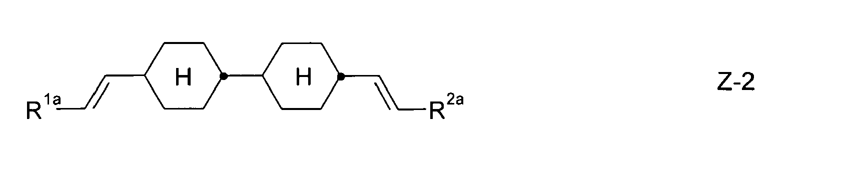 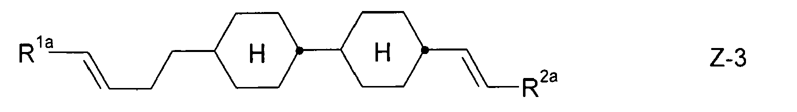 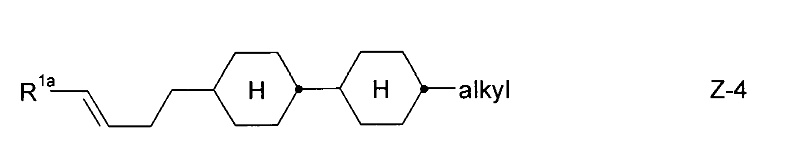 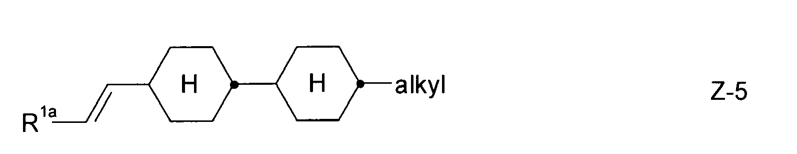 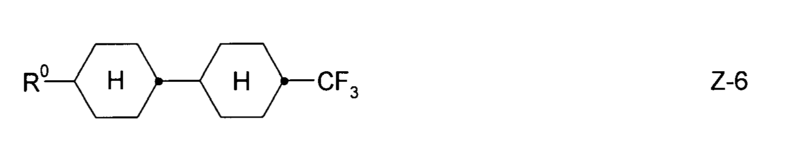 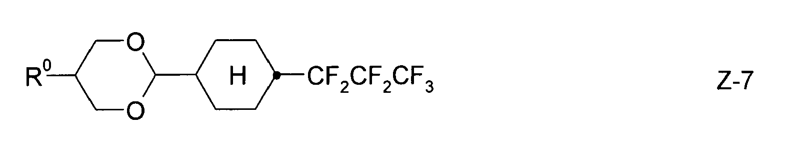 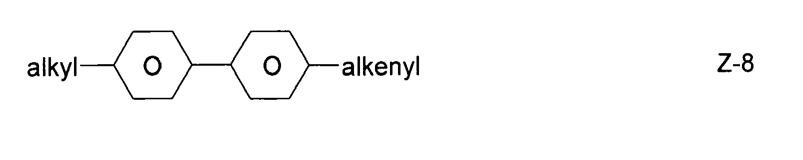 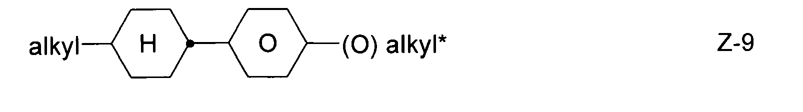 dans lesquelles
R^{1a} et R^{2a} chacun, indépendamment l'un de l'autre, représentent H, CH₃, C₂H₅ ou n-C₃H₇,
R⁰ représente n-alkyle, oxaalkyle, fluoroalkyle ou alkényle, chacun ayant jusqu'à 9 atomes de C,
alkyl, alkyl* représentent un radical n-alkyle non substitué ayant 1 à 7 atomes de C, et
alkenyl représente un radical alkényle non substitué ayant 2-7 atomes de C.

6. Milieu cristallin liquide selon au moins l'une des revendications 1 à 5, **caractérisé en ce qu'**il comprend additionnellement un ou plusieurs composés choisis parmi le groupe constitué des formules générales II, III, IV, V et VI: 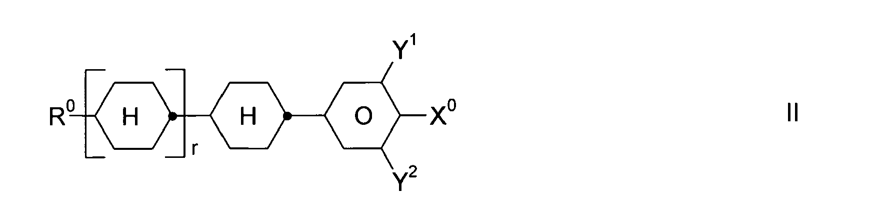 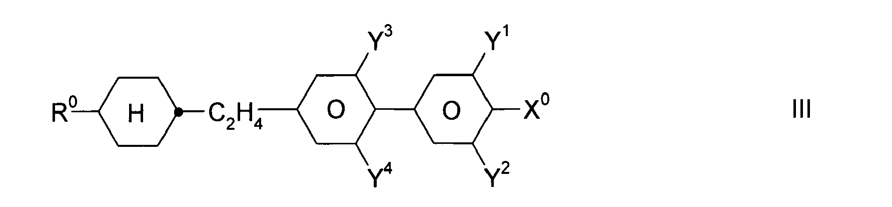 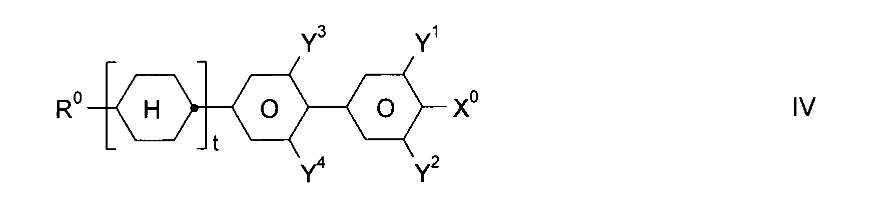 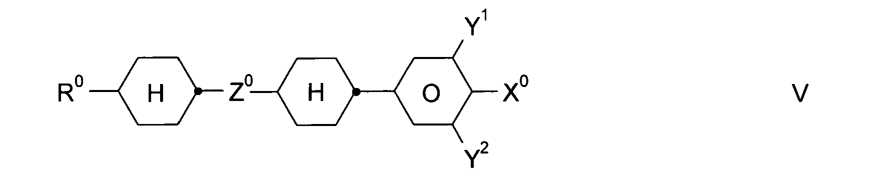 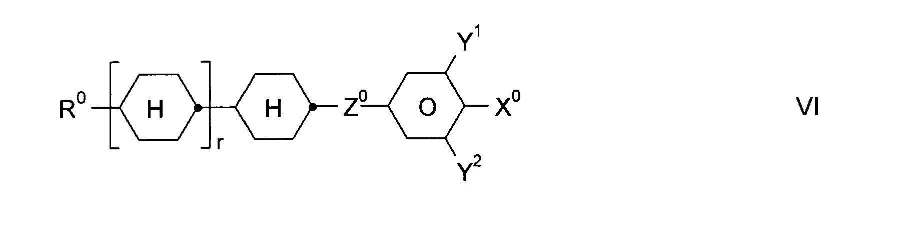 dans lesquelles
R⁰ représente n-alkyle, oxaalkyle, fluoroalkyle ou alkényle, chacun ayant jusqu'à 9 atomes de C,
X⁰ représente F, Cl, alkyle halogéné, alkényle halogéné, alkényloxy halogéné ou alcoxy halogéné ayant jusqu'à 6 atomes de C,
Z⁰ représente -C₂F₄-, -CF=CF-, -C₂H₄-, -(CH₂)₄-, -OCH₂-, -CH₂O-, -CF₂O- ou -OCF₂-,
Y¹ à Y⁴ chacun, indépendamment l'un de l'autre, représentent H ou F,
r représente 0 ou 1, et
t représente 0, 1 ou 2.

7. Milieu cristallin liquide selon au moins l'une des revendications 1 à 6, **caractérisé en ce qu'**il comprend additionnellement un ou plusieurs composés choisis parmi le groupe constitué des formules générales XIV et XV: 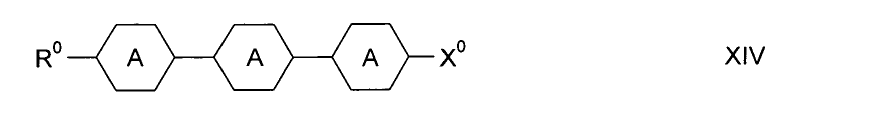 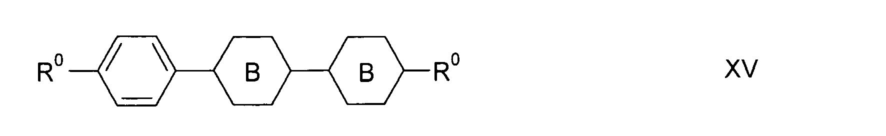 dans lesquelles
R⁰ représente n-alkyle, oxaalkyle, fluoroalkyle ou alkényle, chacun ayant jusqu'à 9 atomes de C,
X⁰ représente F, CI, alkyle halogéné, alkényle halogéné, alkényloxy halogéné ou alcoxy halogéné ayant jusqu'à 6 atomes de C,
cycles A et B, indépendamment l'un de l'autre, représentent 1,4-phénylène qui est substitué par 0, 1 ou 2 fluors, et
dans lequel, dans le cas, au moins un des cycles 1,4-phénylène est mono- ou polysubstitué par des atomes de fluor.

8. Milieu cristallin liquide selon au moins l'une des revendications 1 à 7, **caractérisé en ce qu'**il comprend additionnellement un ou plusieurs composés des formules E-a à E-d: 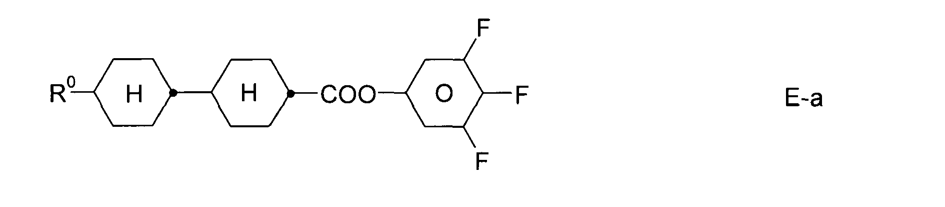 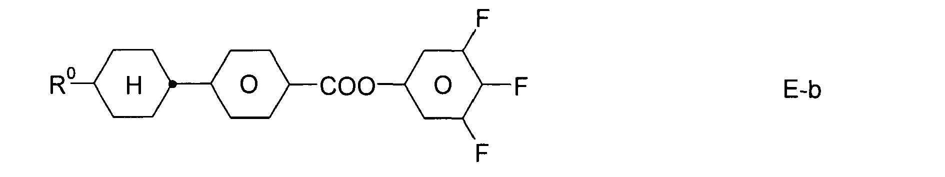 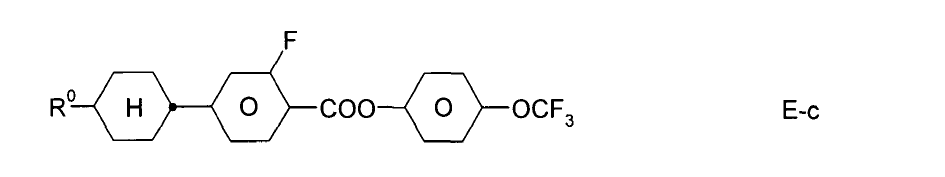 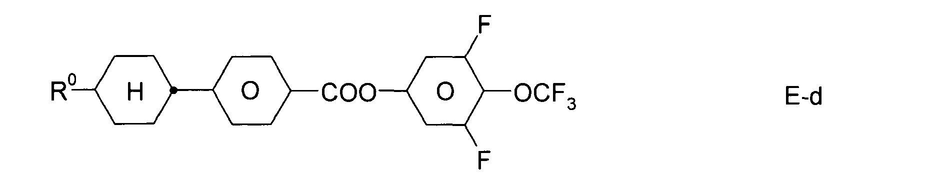 dans lesquelles
R⁰ a les significations indiquées dans la revendication 1.

9. Milieu cristallin liquide selon au moins l'une des revendications 1 à 8, **caractérisé en ce qu'**il comprend un ou plusieurs composés des formules IIa à IIg: 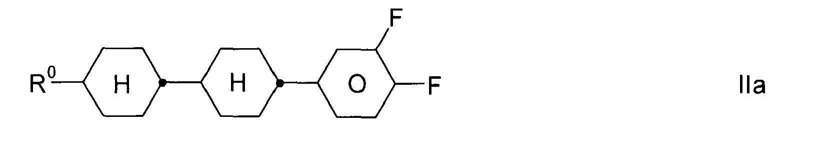 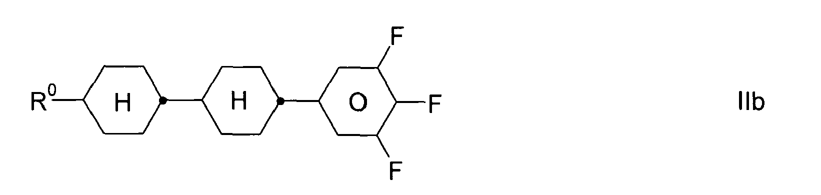 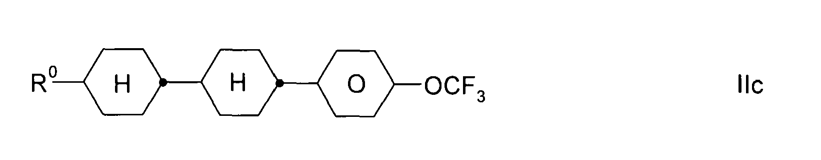 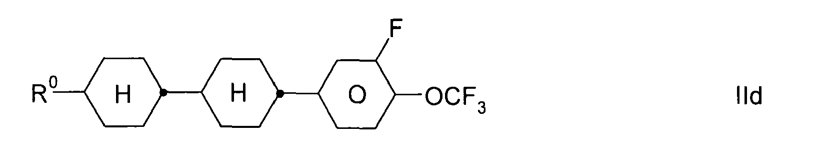 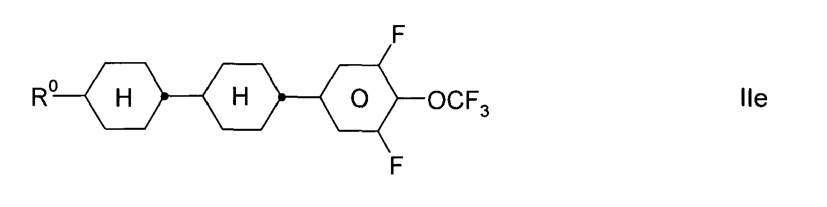 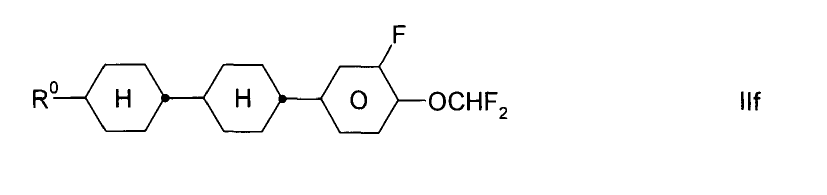 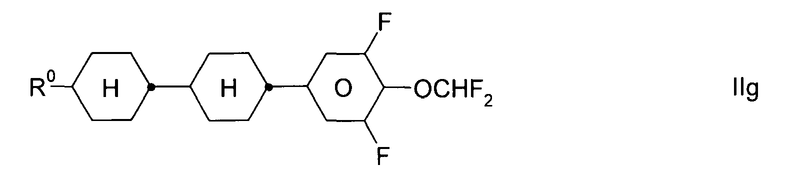 dans lesquelles
R⁰ a les significations indiquées dans la revendication 1.

10. Milieu cristallin liquide selon au moins l'une des revendications 1 à 9, **caractérisé en ce qu'**il comprend un ou plusieurs composés des formules O1 et O2: 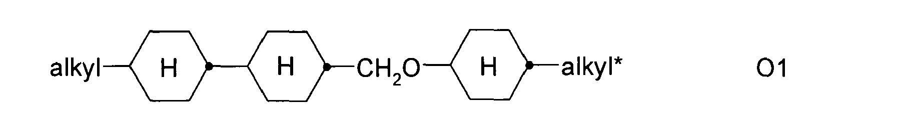 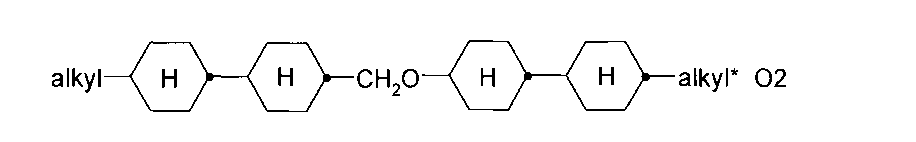 dans lesquelles
alkyl and alkyl* chacun, indépendamment l'un de l'autre, représentent un groupe alkyle en chaîne droite ou ramifié ayant 1-7 atomes de carbone.

11. Milieu cristallin liquide selon au moins l'une des revendications 1 à 10, **caractérisé en ce qu'**il comprend un ou plusieurs composés dioxane des formules D1 et/ou D2: 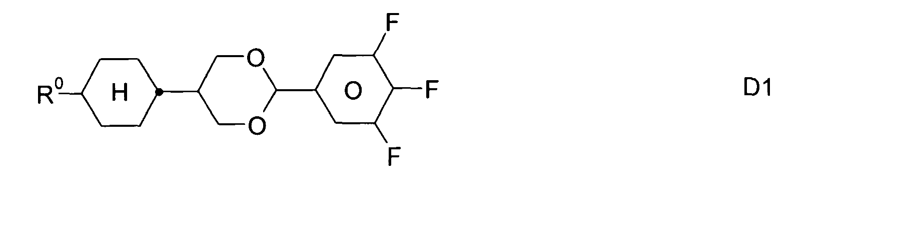 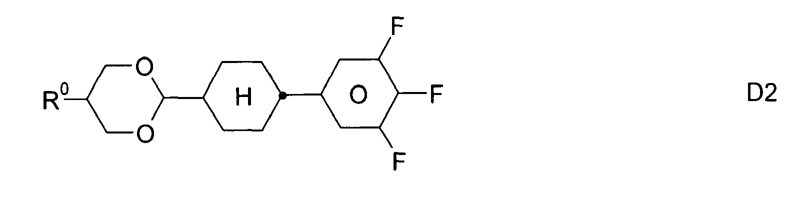 dans lesquelles
R⁰ a les significations indiquées dans la revendication 3.

12. Utilisation du milieu cristallin liquide selon au moins l'une des revendications 1 à 11 dans des buts électro-optiques.

13. Affichage à cristal liquide électro-optique contenant un milieu cristallin liquide selon au moins l'une des revendications 1 à 11.

14. Composés de la formule Ic 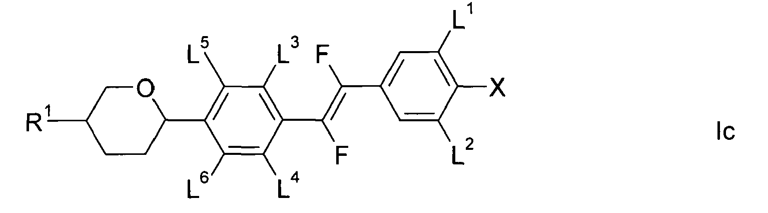 dans laquelle
R¹, L¹, L², L³, L⁴, L⁵, L⁶ et X ont les significations indiquées dans la revendication 1.
